Europäisches Patentamt

European Patent Office

Office européen des brevets

(19)

(11) **EP 1 724 684 A1**

(12) **EUROPEAN PATENT APPLICATION**

(43) Date of publication:
**22.11.2006 Bulletin 2006/47**

(51) Int Cl.:
*G06F 9/46* *(2006.01)*

(21) Application number: **05291064.3**

(22) Date of filing: **17.05.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **BUSI Incubateur d'entreprises d'AUVEFGNE**
**63360 Saint Beauzire (FR)**

(72) Inventors:
• **Coudon, Julien**
**63170 Aubière (FR)**
• **Gineste, Laurent**
**63540 Romagnat (FR)**

• **Hou, Kun Mean**
**63000 Clermont Ferrand (FR)**
• **Ponsonnaille, Jean**
**63400 Chamalières (FR)**
• **De Vaulx, Christophe**
**43110 Aurec/Loire (FR)**
• **Zhou, Haiying**
**63172 Aubière (FR)**

(74) Representative: **Thurgood, Alexander John**
**Cabinet Richebourg**
**"Le Clos du Golf"**
**69, rue Saint-Simon**
**42000 Saint-Etienne (FR)**

(54) **System and method for task scheduling, signal analysis and remote sensor**

(57)     The present invention concerns a real-time operating system, associated network transmission protocol implementation, a method of analysis of an ECG signal, and a wireless sensor network device integrating all of said, wherein the method comprises : acquiring one or more ECG signals over a period of time; filtering said one or more ECG signals with at least one filter selected from the group consisting of a low pass filter, a high pass filter, and an adaptive filter; and extracting one or more features from said filtered signal.

Fig. 1a

EP 1 724 684 A1

Screen    Sensors

14 pin J-Tag connector ⟷ MSP430F149 ⟷ Serial Flash

RFM

**Fig. 1b**

Sensors

Screen    MSP430F149

14 pin J-Tag connector ⟷ CP3000 ⟷ Serial Flash

Bluetooth/Wifi    RFM

**Fig. 1c**

**Description**

Wireless sensor networks (WSN) technology is a powerful technology that is currently shaping science and engineering in the twenty-first century. Revolutionary changes are already underway in a broad range of monitoring and control fields: transportation, manufacturing, health care, environmental surveillance, virtual reality, safety and security, to name just a few. Each of these areas has developed corresponding technologies, with their own hardware, algorithms, mathematics and specialized techniques. Advances in networking and integration have enabled small, flexible and low-cost nodes that interact with their environment through sensors, actuators and communication mediums. Single-chip systems are now merging that integrate low-power CPU and memory, radio or optical communication device and substantial Micro-ElectroMechanical-based Sensors (MEMS). Target costs for single-chip nodes are very cheap per unit, which enables networks with potentially tens of thousands of nodes. Target power consumption means that nodes can last years with low-bandwidth communication or even be battery-free when fuelled by ambient power, e.g. heat, light or vibration from the environment. The combination of sensing, computation and wireless communication into a single tiny node is termed a WSN node, also known as a "mote" or smart dust. WSN nodes are tiny wireless MEMS that can detect everything from light to vibrations. Due to recent breakthroughs in silicon and manufacturing techniques, these "motes" could eventually be the size of a grain of sand, and yet each would still contain sensors, computing circuits, bidirectional wireless communications technology and a power supply. Motes can gather a large number of data, run computations and communicate the information obtained using for example two-way band radio between motes. The emergence of WSN has created a new way of thinking about the computer and greatly extends the range of potential sensor applications. In general, traditional wireless devices, such as cell phones, personal digital assistants (PDA) or laptops equipped with IEEE 802.11 wireless network interfaces, cost hundreds of dollars, target specialized applications and reply on the pre-deployment of extensive infrastructure support. In contrast, a WSN system uses small, low-cost embedded devices for a wide range of applications and does not depend on any existent infrastructure. The fundamental feature of a WSN node is its tiny size and associated tiny cost. The core of a WSN node is a small, low-cost, low-power microcontroller that performs both application and protocol-level processing. By monitoring one or more sensors, a WSN node can obtain various exterior information including signals for temperature, light, sound, position, acceleration, vibration, stress, weight, pressure, humidity, etc. A WSN node can connect with other nodes or access points through a small and low-power radio at a distance of approximately 100 meters. The real-time monitoring capability is the fundamental feature of a WSN system. The most straightforward application of WSN technology is to monitor in real time remote environments for low frequency data trends. Typical usage scenarios for a WSN system range from real time tracking, environment monitoring, real time patient care, structural monitoring, or even battlefield monitoring, etc.

Telemedicine is defined as the delivery of healthcare and sharing of medical knowledge over a distance using telecommunications systems. The term telemedicine is usually applied to interactive televideo "store-and-forward" image and medical record transmission via personal computers, and to remote monitoring. Currently, there are over 450 telemedicine programs worldwide. There are programs in virtually every medical speciality, and the patients they most commonly serve include those who live in rural areas, the elderly and etc. With the growing ability of modern computer and communication technologies, key clinical data, such as ECG (electrocardiograph), images, etc, can be captured and immediately transmitted to remote servers. In this way, clinical data are used to improve the health care of all the patients who live in areas where medical personnel are not readily available. Telemedicine also allows remote monitoring of patients anywhere and at anytime, and provides a new approach to health care. Despite the widespread use of telemedicine in all areas of health care delivery, this area is still considered unacceptable or unavailable for most people. One of the three main reasons currently impeding the growth of telemedicine is uncertainty about its efficacy, which has resulted in a certain unwillingness to implement telemedicine programs. A number of systematic reviews noted that although telemedicine technology shows promise in certain areas, the overall methodological quality of the evaluative studies was low. There is only a small amount of evidence that interventions provided by telemedicine result in clinical outcomes that are comparable to or better than face-to-face care. The low cost-effectiveness of telemedicine is another of the main obstacles to its current development. The costs of home-based telemedicine devices, remote network connection and services for clinical review are much more expensive than face-to-face care in many telemedicine applications. There is still a lack of a plan for the most appropriate and cost-effective use of telemedicine. Finally, conventional telemedicine is a "store-and-forward" operation mode, where clinical data is collected and forwarded for review later in a completely asynchronous manner. This mode is effective for patients with chronic diseases, but does not work for those sudden diseases, such as the heart attack which often results in a sudden cardiac death if the patient does not receive a treatment in time.

The emergence of WSN technology thus brings great opportunity to the traditional health care field. The combination of WSN technology and telemedicine technology allows patients to receive 24 hours per day real time care with time and space decoupled. The sensors of a WSN node capture patients' biomedical information, e.g. temperature, blood pressure, electrocardiograph (ECG) etc, over a long period ranging from a few days to several months. The wireless delivery mode of a WSN node allows patient daily activities when receiving a health care service without motion limitation. The computation capability of a WSN node makes it possible for real-time analysis and diagnosis of clinical information. It should be noted however, that technical problems still remain to be solved in this application field including providing a suitable biomedical wireless sensor device dedicated to the applications of health care or other biological signal processing.

The object of the invention is therefore to provide such a wireless sensor device or remote sensor system, and an embedded real-time multi-tasking operating system dedicated to said WSN device. Other objects of the invention are an embedded wireless communication protocol stack and a real-time method of biological signal analysis that is particularly suited to applications in the field of health care, and most notably in the detection and prevention of cardiac arrhythmia.

Insofar as some of these objects are concerned, it is possible, and the inventors have demonstrated, that the implementation can be software or hardware driven. In particular, the present applicants have developed a super-small distributed real-time micro kernel, hereafter designated SDREAM, a minimal real-time embedded TCP/IP stack, herein identified as μRET, a real-time automated ECG diagnostic (AED) algorithm used for analysing and processing the biological signals that are captured by the sensor device or system, and a telemedicine platform system for multi-user remote real-time ECG detection and diagnosis, all of which work together with the device to perform the necessary signal processing and handling.

Consequently, one object of the present invention is a Super-small Distributed REAl-time Micro Kernel, hereinafter known as SDREAM. Realizing a WSN system presents very significant challenges, especially at the architectural, operation system and protocol, software or hardware level. Major steps forward are required in the field of operating systems, communications protocols, data processing, and application support. One of these challenges is the design of an Embedded Operating System (EOS) on a WSN device having limited resources and yet still meeting real-time requirements. Comparing to conventional embedded devices, a WSN device has far stricter resource constraints and requires the ability to communicate wirelessly, which means that most popular EOSs can not be migrated directly onto a WSN device. In addition, WSN applications often contain real-time concurrent activities, e.g. simultaneous data acquisition, data processing and data transmission, which means that a WSN OS must also be a Real-Time Operating System (RTOS) that supports real-time multitasking operations. A WSN OS is therefore effectively an embedded real-time OS that is tuned to strictly constrained resources such as limited power, processing, storage, and bandwidth, and which supports wireless communication and real-time distributed activities. The present applicants have succeeded in creating a Super-small Distributed REAl-time Micro kernel (SDREAM) that is primarily aimed for such a WSN system. The SDREAM of the present invention is a tuple-based message-driven real-time operating system dedicated to resource constrained wireless devices. It has been designed to run on general purpose architectures where a single CPU is shared between application and system processing. The SDREAM of the present invention also provides a highly efficient communication mechanism and a fine-grained concurrency mechanism to real-time applications. It enables the development and implementation of multi-tasking real-time applications by providing a "priority-based preemptive scheduling" scheduling strategy. The tuple concept of LINDA parallel programming is preferably used for inter-task communication and task synchronization. The SDREAM of the invention has a flexible hardware abstraction capability which enables it to be rapidly migrated onto different WSN platforms and tiny embedded devices. Preferred microcontrollers for the implementation of the SDREAM and that may be used alone or combined in accordance with the invention are any 8 bit to 32 or even 64 bit multichannel microcontrollers, such as the 16 bit TMS320C541 0 microcontroller available from Texas Instruments, the 16 bit MSP430F149 microcontroller available from Texas Instruments, and the 16 bit CP3000 microcontroller available from Texas Instruments. In a most preferred embodiment, the MSP430F149 microcontroller is used for signal acquisition and the CP3000 microcontroller is used for system management.

All WSN nodes comprise four basic subsystems :

- a power subsystem: in order to monitor an environment for any length of time, the WSN node ought to have enough energy to survive anywhere from a few hours to months at a time. The energy can be provided by any well known means, for example, direct electrical current, batteries or accumulators, magnetic field induction, radio frequency induction, solar energy and the like, but obviously small and compact energy provider solutions are preferred in view of the intended application of the WSN node;

- a computation subsystem: since the WSN node is expected to communicate and process sensor data, it will generally have computational requirements that range anywhere from an 8 bit microcontroller to a 16, 32 or 64 bit microprocessor;

- a sensor subsystem: the interface between the environment and the WSN node is the sensor. The WSN nodes usually have at least one sensor, which may include temperature, pressure, humidity, light, sound, acceleration, magnetic fields, etc;

- communication: communication must be possible in standard outside environments. WSN nodes usually have the ability to communicate with one another at ranges from a few meters to several kilometers.

"Commercial Off-The-Shelf" (COTS) motes are well known WSN platforms per se and serve as the "de facto" WSN platform standard due to their flexibility and availability of components for a great many applications. The COTS motes provide a practical method to test basic WSN functionality. A survey of the three generations of quickly developed WSN prototypes that have been built to date using COTS components is presented hereafter.

The COTS mote is also named COTS Dust, the platform architecture of which originated from the research of Seth Hollar. All COTS motes have a similar architecture, but differ in sensor medley and communication type. The COTS mote architecture was defined in detail in the master's thesis of Seth Hollar. An Atmel AVR microcontroller served as the brain, and depending on the flavour of the device, either optical or RF communication was used. Most COTS devices were powered by a 3V lithium battery. Finally, each device had its own sensor suite chosen according to the demonstration it was to present.

A representative implementation of the first generation COTS mote is the Radio Frequency (RF) mote. The RF Mote consists of the Atmel AT90LS8535, an RF Monolithics 916MHz transceiver set, and 7 sensors (temperature, light, barometric pressure, 2-axis acceleration, and 2-axis magnetometers). The RF Mote system clock of the Atmel MicroController Unit (MCU) runs at 149.475 KHz with a single instruction performed per clock cycle. Nineteen instructions are required to send and receive raw data bits through the RF system. The raw data rate of the RF system is 149.475 KHz / 19 Cycles / bit = 7.867 Kbps, but with the implementation of Manchester encoding, the data rate is cut in half to 3.934 Kbps. The weC mote is basically a smaller version of its predecessor, the RF mote. The weC Mote consists of an RFM TR1000 RF transceiver, an integrated Printed Circuit Board (PCB) antenna, temperature and light sensors, and an additional MCU, an Atmel AVR AT90S2313, which is a smaller version of the AT90S8535. The original intent of the weC mote was to test out amorphous computing style algorithms specifically for distributed sensor networks based on pheromone messaging.

The René mote is the mote of Crossbow resulting from collaboration with the University of California Berkeley. The René mote is based on a modular design concept. The main processor-radio board contains the same components as the weC Mote. The modular design introduces two 51 pin connectors (male and female) for interfacing to sensor and programming boards. Lastly, the sensor board emphasizes the modularity of the René mote. A sensor board has the same dimensions as the main processor-radio board and also has two connectors. The first sensor board, known as the "Basic Sensor Board", contains a light and a temperature sensor and a prototyping area. Thus, a sensor can be connected to the sensor board. Moreover, any number of sensor boards can be attached to the main board by stacking them. The René2 mote replaces the main MCU of the Rene Mote, the AT90S8535, with the ATMEGA163. This MCU has 1 KBytes RAM and l6Kbytes flash memory. The Dot Mote is a circular platform (diameter = 1 inch or 2.54 cm). The Dot Mote consists of the same components as the René2 Mote. Without a 51-pin sensor board interface, the Dot Mote has a temperature and light sensor. The Dot Mote was used at the August 2001 Intel Developers Forum (IDF). The demonstration consisted of wirelessly reprogramming the 800 Dot Motes with a Simple Voting Demo. The entire demo demonstrated Network Discovery, Routing, and Aggregation.

The Mica Mote consists of a RFM TR1000 RF transceiver, 4 Mbit external flash memory (AT45DB041 B), 6 ADC channels available for different sensors, and an 8-bit MCU with a CPU clock of 4MHz consisting of an Atmel ATMEGA103 microcontroller (later versions are an ATMEGA128 in 103 Mode). The original intent of Mica Mote is to overcome the shortcomings of René2 mote. Compared to the René2 mote, the Mica Mote has more storage, more communication bandwidth, stabilized board voltage and more board capability. The Mica Mote has more flexibility for communication protocols, time synchronization, and other algorithms. The Mica2 Mote is the second generation of the Mica Mote. The main improvements of the Mica2 over the Mica are: 1) main MCU with a CPU clock of 7.3827 MHz: ATMEGA128 in 128 Mode, 2) New Chipcon CC1000 RF transceiver with 916/433 MHz bands, 3) Coax antenna connector for external antenna, 4) Streaming ADC data to ATMEGA128 at max rate: 20KHz at 10-bits, 50KHz at 8-bits, etc. Like the Dot mote of the René series, Mica2 also has a circular simplified platform (diameter=1 inch or 2.54 cm). The Mica2Dot consists of the same components as the Mica2 Mote with only the difference in CPU crystal: 4MHz in Mica2Dot vs 7.3827 MHz in Mica2.

Despite the fact that COTS motes have been used predominantly in previous WSN systems research and development, other WSN platforms have been developed and are in use. The EYES project, for example, of the European Research Consortium (ERC) which initiated in 2002 is one such other non COTS mote system. The EYES project represents another approach to WSN development by presenting a different platform from the COTS motes. The EYES project focuses on the architecture, protocol and software issues of their WSN system, and on self-organizing and collaborative energy-efficient sensor networks. It addresses the convergence of distributed information processing, wireless communications, and mobile computing. In the EYES architecture, applications rely on a two-level structure. The lower layer deals with sensors and networking, whereas the upper layer provides distributed services to the application by using the networking and transport capabilities offered by the lower layer. The EYES mote consists of a RFM TR1001 RF transceiver with OOK/ASKS modulation, 1Mbits external flash memory, and a 16-bit MCU: TI MSP430F149 with 2KBytes RAM and 60KBytes program memory.

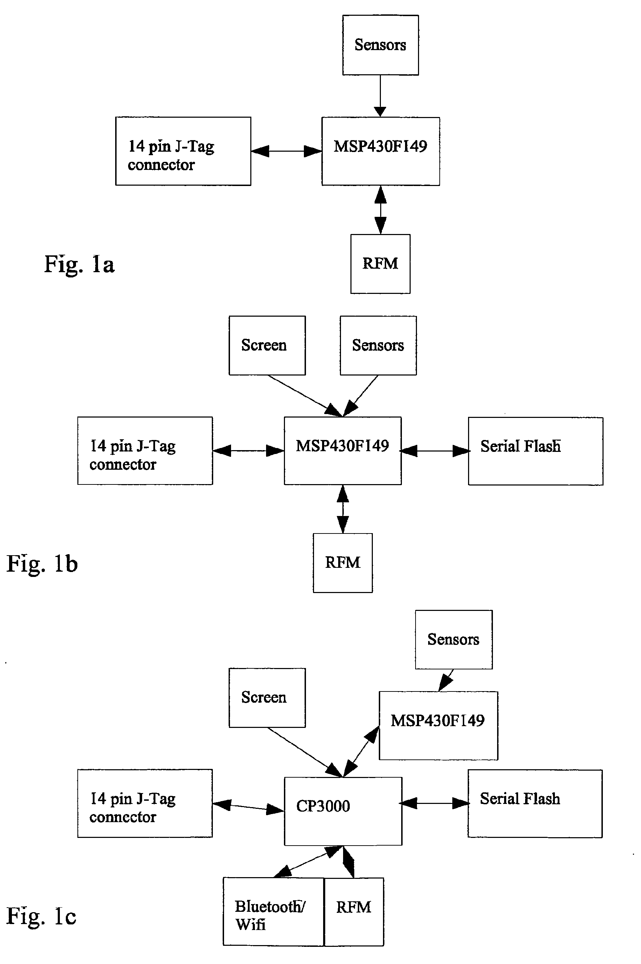

The WSN project that is one of the objects of the present invention is also different to the known COTS mote systems, and is particularly suited to real-time cardiac arrhythmia detection and diagnosis. The present invention is exemplified with regard to applications of the WSN in real-time ECG detection and diagnostics. Preferably, a Texas Instruments MSP430F149 microcontroller serves as the brain which has a CPU clock of 8MHz and a maximum of 8 channels available for sensors, for example, ECG electrodes, while different wireless communication models can be used, for example and preferably, IEEE802.11 b, or even the Bluetooth wireless transmission protocol. In a still yet even more preferred embodiment, the microcontroller used is a CP3000, to manage the system, while a MCU MSP430F149 is preferably dedicated to signal acquisition. System block diagrams of three types of SDREAM architectures as described above are illustrated in Figure 1.

The operating system is viewed as a layer of software that multiplexes, as well as abstracts, hardware resources, such as memory, processor cycles, I/O devices, etc, for applications, and acts as an interface between applications and the hardware platform. The application programs rely on facilities provided by the operating system to gain access to hardware resources. There are a number of existing RTOSs that are potential candidates for use on a WSN node. A number of RTOSs adopt a form of "Modularity" through the use of microkernel architecture, for example, VxWorks, QNX, WinCE, PalmOS, RTLinux. The TinyOS is a well-known system software platform used by a WSN node. TinyOS is dedicated to providing a software solution based upon severe resource constraints.

An "embedded system" for the purposes of the present specification and claims, is generally any computer system or computing device that performs a dedicated function or is designed for use with a specific embedded software application. An embedded system usually involves a microcontroller with limited computational power and a simplified architecture. A good embedded OS should desirably be configurable and scalable with a modular architecture. It generally has a small footprint and low CPU requirement in terms of low system resource constraints compared to those of a general purpose processor.

A "real-time system", for the purposes of the present application, is one in which the correctness of the computation not only depends upon the logical correctness of the computation but also upon the time at which the result is produced. If the timing constraints of the system are not met, system failure is said to have occurred. The ability of a real-time operating system is to provide a required level of service in a bounded response time. A real-time operating system provides an infrastructure for running software on an embedded hardware platform. It abstracts the hardware wherever possible and provides access to the hardware wherever needed. The key difference between general-computing operating systems and RTOS is the need for "deterministic" timing behaviour in the real-time operating systems. The RTOS must guarantee Interrupt Server Routine, or ISR, latency and context switch time in order to achieve deterministic performance. A Real-time system is generally classified into two categories: a hard real-time system and a soft real-time system. The former must keep to very strict timing constraints. Missing timing constraints in a hard real-time system will cause the result to be totally useless, and thus regarded as a system failure even it is correctly computed. In contrast, in a soft real-time system, missing timing constraints occasionally is not fatal, although it might indicate a flaw in the design.

A kernel is the smallest portion of the operating system that provides an abstraction layer upon which the application software will run. The kernel hides the hardware details of the processor, or set of processors, for application software. A RTOS kernel normally consists of four main categories of basic services: task management, intertask communication & synchronization, timer & I/O interrupts, and memory management. The task management is the core of the RTOS kernel, which contains a task scheduling scheme and context switches. A scheduling scheme is mandatory in all microkernel instances, which is a set of rules, procedures, or criteria used in making processor scheduling decisions. The typical scheduling scheme for RTOS has two aspects: priority-based scheduling and pre-emptive scheduling. Cooperating processes need to exchange information, as well as to synchronize with each other, to perform their collective task(s). Methods for effective sharing of information among cooperating processes are collectively known as intertask communication & synchronization. Two basic models are used: message passing and shared memory. The main mechanisms of intertask communication & synchronization include mutex, pipe, semaphore, message queue, and monitor, etc. In an embedded system, a RTOS normally adopts a shared memory model for huge data exchange to decrease the overhead of data-copying and to reduce the storage consumption. The timer is an essential unit of any microkernel. It is normally configurable to show the time consumption by the changes of time-tick variables. With regard to the differences in applications and hardware platforms, there are different I/O interrupts in a RTOS. They provide interactive interfaces between embedded devices and the external environments. The RTOS must be carefully designed to meet timing constraints of real-time applications. Several key time factors in a RTOS include interrupt action latency, interrupt dispatch latency, and task scheduling time, or context switch time. The memory management of a RTOS is normally simple due to the bounded memory resources. There are generally two different management models: dynamic and static memory allocation.

There are lots of commercial and academic real-time operating systems, ranging from small real-time kernels with the size of several kilo-bytes to huge packages with full support for a file system, networking systems, graphics, etc. A brief overview on the designs of some well-known real-time kernels is provided hereafter.

μC/OS-II is a fully pre-emptive kernel written in ANSI C by Jean J. Labrosse. It can manage up to 64 tasks, eight are reserved for system use and the remaining 56 tasks are available for user applications. As each task has a unique priority assigned to it, thus there are 64 priority levels in µC/OS-II. The priorities are dynamic and alterable during execution. µC/OS-II is a configurable kernel so only the required kernel features are selected to reduce memory consumption. µC/OS-II can behave as a non-pre-emptive system by disabling the scheduler. µC/OS-II is a multi-tasking operating system and provides a protection mechanism for shared resources. It provides multiple IPC and synchronization services like mailboxes, message queues, semaphores, fixed-sized memory partitions, time-related functions, etc.

VxWorks5.x from Wind River Systems, Inc. is a priority real-time operating system and supports a wide range of industry standards including POSIX 1003.1 b Real-Time Extensions, ANSI C and TCP/IP networking. Both a pre-emptive priority and a round-robin scheduling mechanism are available in VxWorks5.x. There are 256 priority levels and the maximum number of tasks is only limited by the amount of the memory available. All tasks share the same address space, but an optional component (VxVMI) can allow each task to have its private virtual memory and provide memory protection. Interrupt Service Routines (ISRs) in VxWorks5.x run in a special context outside of any task's context, so that there are no additional context switches involved. The intertask communication mechanisms include shared memory, semaphores, message queues, pipes and signals, etc. VxWorks5.x adopts a modular framework and is thus scalable. By selecting various modules, VxWorks5.x can be configured for the use in a small embedded system having tough memory constraints up to complex systems. Furthermore, individual modules themselves are scalable. Individual functions may be removed from the library, or specific kernel synchronization objects may be omitted if they are not required by the application.

QNX Neutrino from QNX Software Systems Ltd. has a client-server architecture consisting of a lean microkernel that implements only core services and optional cooperating processes, such as signals, timers, and scheduling. All other components: file systems, drivers, protocol stacks and applications, run in the safety of memory-protected user space. QNX Neutrino is a message-based multi-process operating system. Message passing is the fundamental means of IPC. This message passing forms a virtual "software bus" and messages can flow transparently across processor boundaries, providing seamless access to any resource, anywhere on the network. QNX Neutrino is a self-healing system and has powerful fault resilience ability. In QNX Neutrino, every process has its own virtual memory, code and data segment. QNX Neutrino provides full MMU (memory management unit) support for memory protection. An interrupt handler execution can be pre-empted by interrupt handlers from a higher priority interrupt source. QNX Neutrino complies with POSIX 1003.1-2001 and features an extensive POSIX feature set, including real-time extensions and threads. As a result, it's very easy to port Linux, Unix, and other POSIX-based open source programs. Moreover, the QNX Neutrino is the only commercial RTOS to support true symmetric multiprocessing (SMP): it allows any thread in any process to be scheduled to run on any processor of an SMP system.

pSOSystem3 from Wind River Systems, Inc. is a modular, high-performance, memory protected, highly reliable real-time operating system, designed specifically for embedded microprocessors. It includes a kernel pSOS+ and the integrated development environment pRISM+. The configurability and extensibility is achieved by highly modularized design; there are separate modules that support single and multiple processors, file management and TCP/IP, streams communication, graphics, and JAVA. pSOS+ is a variant of the client-server architecture and uses a software bus to communicate between the different modules. pSOS+m is a multiprocessor version of the pSOS+ kernel. It requires one node to be a master and the rest are slaves. The nodes can be connected in different ways: shared memory, message-based buses or custom links, etc. Priority FIFO scheduling and round-robin scheduling schemes are adopted in pSOSystem3. pSoSystem3 has a flat memory space and the memory protection function is optional. It offers two sets of memory handling functions: Regions, i.e. non-fixed block size pools and Partitions, i.e. fixed-size block partitions.

LynxOS4.0 from LynuxWorks is a hard real-time operating system. Each OS component of LynxOS 4.0 is designed for absolute determinism. It performs a complex series of tasks respecting the time constraints (predictable response). LynxOS4.0 is a multi-tasking RTOS and has four scheduling schemes (FIFO, Priority Quantum, Round-Robin and Non-pre-emptive). It offers comprehensive intertask communication facilities: message queues, semaphores, shared memory, sockets, signals, pipes, mutexes and condition variables. LynxOS4.0 provides address spaces protection for tasks through MMU, and is linearly scalable to an unlimited number of tasks. Lynx4.0 conforms to open system standards: Linux, UNIX and POSIX. It can offer true Linux binary-compatibility which means that applications written for Linux run on LynxOS 4.0 with no loss of functionality. Moreover, LynxOS gives access to a full state of the art TCP/IP stack derived from FreeBSD4.2.

RTLinux from New Mexico Tech. is a hard real-time operating system that handles timecritical tasks. It runs Linux as its lowest priority execution thread. RTLinux uses a virtual machine layer to make the standard non-real-time kernel be fully pre-emptive, so that real-time threads and interrupt handlers are never delayed by non-real-time operations. Interrupt off/on operations are emulated by a lower level kernel. It allows the system to run accurate time constraint applications to perform data acquisition, system control, etc, while serving as a standard Linux workstation. Real-time threads in RTLinux can communicate with Linux processes via shared memory or a file-like interface, so real-time applications can make use of all the powerful, non-real-time services of Linux. The results of interrupt latency measurement indicate that a great improvement over the Linux operating system is achieved; the interrupt latency of Real-Time Linux is about one third that of the Linux operating system on 33MHz Intel 80486 machines, while one eighth on 120MHz Intel Pentium machines.

Windows CE.NET is a hard real-time embedded operating system of Microsoft Corporation. Lots of processors have been validated to support Window CE.NET and its main application areas are: PDAs (Personal Digital Assistants), industrial embedded systems, and game consoles. Windows CE .NET is a "componentized" operating system. It includes a comprehensive set of more than 350 technology components. It provides a graphical integrated development environment (IDE) listing a comprehensive and extensible catalog of all operating system components. Through its extensible and robust componentization, Windows CE .NET offers a scalable operating system for a wide range of device types. Windows CE.NET supports true real-time behaviors. It provides 256 levels of thread priority. Nested interrupts are supported to ensure that a high priority level interrupt is serviced immediately. The per-thread quantum feature means users can adapt the scheduler according to the current need of application. Priority inversion technology enables the lower-priority thread to inherit the more critical thread's priority and to run at the higher priority until it releases the critical resource. Moreover, Windows CE .NET provides networking and communications capabilities, i.e. a full TCP/IP stack, that enable devices to connect and communicate more securely with other devices and people over both wireless and wired networks.

DREAM is a software real-time pre-emptive microkernel written in ANSI C by C. de Vaulx. DREAM is dedicated to intelligent biomedical sensors. It performs sets of tasks within predictable time. DREAM is a multi-tasking RTOS and provides three classes of tasks: periodic, aperiodic and daemon. It offers two inter-task communication facilities: semaphore and tuple, a concept of LINDA. DREAM does not manage virtual memory and does not provide address space protection for tasks. DREAM is a fault-tolerant system which gives an abstract definition for system behaviours and primitives by Meta language. It is also a distributed system that can run over a set of processors. DREAM supports dynamic memory configurations. It does not conform to any open system interface standard.

All the RTOSs support multi-tasking pre-emptive scheduling. Each task has a unique priority level which is a static or dynamic configuration according to the needs of applications. The response time for interrupt handlers and context switches are predictable so as to guarantee deterministic timing behaviours. Virtual memory and memory protection capability are provided in most RTOSs to avoid race conditions and to enhance the reliability of the system. Moreover, most RTOSs comply with a wide range of industry standards including POSIX, ANSI C, or Win32 API, etc, which makes programming on different platforms similar, and ports across these platforms easier.

Since a WSN operating system is dedicated to tiny WSN hardware platforms, it ought to be small and efficient in terms of highly constrained resources - limited power, processing, storage, and bandwidth. It must also be agile enough to allow multiple applications to simultaneously use system resources such as communication, computation and memory. Moreover, a WSN OS should be flexible for the rapid release of hardware and the variety of sensors. The extreme constraints of WSN hardware make it impractical to use traditional OSs. A well-known and widely-used WSN OS is TinyOS. TinyOS is a component-based, event-driven operating system for sensor network nodes developed as part of the Berkeley WEBS project. The TinyOS design follows two basic criteria:

extremely simple and extremely efficient. It focuses on three high-level goals in sensor network system architectures:

take account of current and likely future designs for sensor networks and sensor network nodes;
allow diverse implementations of both operating system services and applications, in varying mixes of hardware (in different mote generations) and software;
address the specific and unusual challenges of sensor networks: limited resources, concurrency-intensive operation, a need for robustness, and application-specific requirements.

In order to provide a comparison with TinyOS, some micro-operating systems (Micro-OSs) dedicated to microcontrollers are also presented.

TinyOS provides an efficient architecture of an event-driven concurrency model for modularity and resource-constraints. The modular architecture allows the OS to adapt to hardware diversity while still allowing applications to reuse common software services and abstractions. The design of the TinyOS Kernel is based on a two level scheduling structure: events and tasks. Events are divided into two classes: hardware events and software events. Software events signal completion of the requested action. Hardware events are interrupts, caused by a timer, sensor, or communication device. Hardware events are intended to do a small amount of processing (e.g. timer interrupts, ADC interrupts) and can pre-empt, i.e. interrupt, longer running tasks. When no useful work is to be performed, the TinyOS system enters an ultra-low power state, causing shutdown of the WSN node except for the clock, to reduce power consumption. Tasks are a form of deferred procedure call that allows a hardware event or task to postpone processing. They are intended to do a larger amount of processing and are not time critical e.g. computing an average on an array. Tasks can be scheduled at any time but will not execute until current pending events are completed. Tasks always run to completion with respect to other tasks, which implies that a TinyOS system application only needs a single stack. System modularity is based on a component model. A named component encapsulates a private data frame of some fixed-size state and a number of tasks. Components interact with each other strictly via function call interfaces, which are related groups of commands and events. The set of interfaces that a component uses and provides define its external namespace. Commands typically represent requests to initiate some action; events represent the completion of a request or something triggered by the environment, e.g., message reception. A specific set of events are bound to hardware interrupts. An application is assembled by specifying a set of components and "wiring" their interfaces together. The TinyOS concurrency model intentionally does not support blocking or spin loops. As a result, many operations are split-phase : the request is a command that completes immediately, and an event signals the completion of the requested action. This approach is natural for reactive processing and for interfacing with hardware, but complicates sequencing high-level operations, as a logically blocking sequence must be written in a state-machine style. TinyOS allows many concurrent activities to be serviced on a single stack, reflecting the very limited memory on modern microcontrollers. It also makes handling of error conditions explicit. TinyOS intentionally defines neither a particular system/user boundary nor a specific set of system services. Instead, it provides a framework for defining such boundaries and allows applications to select services and their implementations. Nonetheless, a common set of services has emerged and is present on most platforms and used by most applications. This includes timers, data acquisition, power management, and networking. This general decomposition is not unique to TinyOS; it can be found in other sensor network platforms. It covers the basic requirements of a sensor network: use little power, periodically collect data, perform some simple processing on it, and if needed, send it to a neighboring node.

Some Micro-OSs have been written for microcontrollers with commercial or academic intentions. The Micro-OS provides a software platform over the limited resources of microcontrollers. In general, it addresses itself to a set of particular applications or a series of particular processors.

RTOSs focus on the real-time requirements of embedded applications. The "priority-based pre-emptive multi-tasking scheduling" scheme guarantees the predictable responses and deterministic behaviours. However, despite RTOSs having reduced function "bloat" to meet large scale deeply embedded devices, they still appear too "huge" for some ultra-small WSN nodes. For example, most RTOSs provide virtual memory for each task or interrupt handler. A private memory space of each task can improve the reliability of RTOS, but may cause high system overhead due to large memory consumption and more context switch. In addition, as general embedded devices have an unlimited power source, the designs of RTOSs are normally unconcerned or not tuned particularly to the strict power constraints of WSN nodes. Hence, conventional RTOSs can not be utilized directly for strict resource constrained devices, i.e. WSN nodes. In fact, in terms of the application scenarios of WSN system, many unnecessary and overkill functionalities can be omitted from RTOS, e.g. address space isolation, complex I/O subsystem, and UI/GUI, etc.

On the other hand, TinyOS focuses on the wireless sensor application over the extremely strict resource constraints of embedded devices. TinyOS is a naturally single-tasking-model event-driven system, which provides a two-level scheduling scheme (event and task) to handle interrupts. As a result of limited system resources, TinyOS can not support large numbers of real-time interrupts tasks. Moreover, traditional network protocols, i.e. TCP/IP stack, are not supported because of their relative high memory requirements and "stop and wait" semantic model, since TinyOS does not support blocking or spin loops. Because traditional TCP/IP headers and sockets are not suited to a bounded TinyOS environment, the overhead per TCP/IP packet is rather expensive for any given WSN platform. It means a WSN node that uses TinyOS can not directly connect with the existing network infrastructures.

In order to provide real-time multi-tasks processing and traditional networks connection capabilities for WSN nodes, there was therefore a need to design a new WSN operating system. Accordingly, the present invention provides such an OS, which additionally and advantageously is small enough and efficient enough for the tiny resources and low power source of a WSN node. Secondly, the operating system according to the present invention is a true RTOS, supporting real-time multi-tasking operation and TCP/IP network connection capability. An efficient task scheduling scheme and a smart inter-task communication and synchronization mechanism have been developed by the applicants which has led to reduction in the consumption of resources and power, and reduced overhead of computation and communication.

One object of the present invention is therefore a Super-small Distributed REAl-time Micro Kernel (SDREAM). SDREAM is a microkernel and comprises four main components: task management, inter-task communication and synchronization, memory management, and interrupt handling and timer management. SDREAM has an abstract system definition and description through a Meta language, known as a Kernel Modeling Language : Meta, or KML:Meta. The four components of SDREAM and their Meta description are described hereafter.

As has been mentioned above, the motivation for designing SDREAM was to implement a distributed real-time kernel for a wireless sensor for use in intelligent biomedical applications. In SDREAM, tasks are classified into two categories: periodic and priority; system primitives and behaviours are defined using a Meta language. SDREAM adopts a real-time two-level scheduling scheme called "priority-based pre-emptive scheduling". SDREAM is a tuple-based message-driven system. It supports blocking and spin loop operation. The "tuple-based message" is the basis of task communication and synchronization. A shared data memory area, named tuple space, consists of a set of tuples each having a unique ID: KEY. In SDREAM, task communication is implemented by two system primitives: SND() and RCV (). The memory management of SDREAM adopts a static pre-allocation strategy for all system elements, including a set of tasks e.g. periodic and priority, a set of tuples, etc. There are two classes of interrupts in SDREAM: an I/O interrupt and a timer interrupt. A timer ISR (interrupt service routine) manages system time variables and triggers task scheduling; leaving I/O ISRs to be handled immediately to meet the strict time constraints. In order to tune to intelligent biomedical applications of a WSN node, SDREAM is small and efficient for tiny resource consumption and low power overhead. It also satisfies the requirements of multi-tasking real-time operation. The main advantages of SDREAM are that :

it is ideally suited to distributed applications;
it is highly reliable, in that it can support long-term no-fault operation;
it contains two classes of tasks: periodic and priority;
the periodic task being interruptible and non-pre-emptible;
the priority task being interruptible and preemptible;
each task is an automaton;
it uses the IPC primitives SND() and RCV().

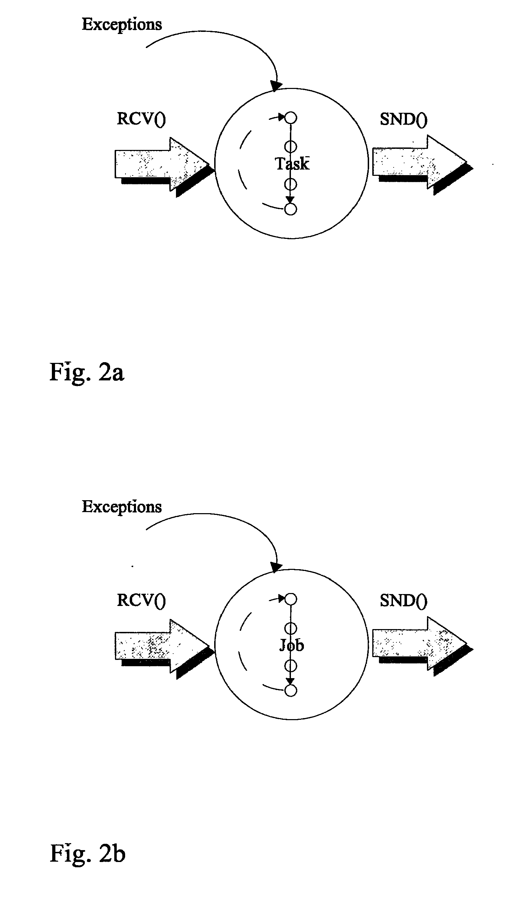

In accordance with the present invention, a task is defined as a couple, termed couple ($\amalg$, $\wp$), where $\amalg$ is a set of temporal TAGs, $\amalg$ ={tj; j =1,..., n} and $\wp$ is a set of actions, $\wp$={ai; i=1,..., n}. The $\wp$ can be an ordered sequence or a non-ordered sequence, correspondingly SDREAM supports precedence and concurrence operations. Each task is an automaton, as represented in Figures 2a and 2b. It communicates with each other through two basic system primitives, named SND() and RCV(). A task is interruptible by exceptions caused normally by interrupts.

In accordance with the present invention, an intelligent sensor has two classes of tasks: periodic and priority tasks, also called aperiodic or sporadic tasks. Periodic tasks are typically suitable to capture sensor signals, such as ECG, video image, temperature and the like, or to control actuators, which are interrupted at fixed-time intervals. Periodic tasks have a very short execution time and deterministic response time as well as the highest priority level, which is preferably 10, in SDREAM. They are interruptible but non--pre-emptible. Priority tasks are typically suitable for time constrained applications, which are interrupted at variant-time intervals. Priority tasks have predictable response times so as to conform to real-time constraints. They are interruptible and also pre-emptible, having priority levels comprised from 1 to 9, i.e. from low to high. The Daemon, the idle task of SDREAM, is a particular priority task with the lowest priority level, in this case priority 0. This task is executed only when no task is available in the task ready queue. It means that the system is in idle status. An application or job is composed of a set of tasks, including periodic and priority tasks. An application is also an automaton in SDREAM. Each application is interruptible by the timer and external peripheral interrupts.

Definitions of symbols:

P: Periodic task
$A^x$ : Priority task with a priority level x
$A^y$ : Priority task with a priority level y
<> : A set of tasks or actions
Description of an application (3P, 2$A^x$ and 3$A^y$):
Job = << $P_1$, $P_2$, $P_3$>, <$A^x_1$, $A^x_2$>, <$A^y_1$, $A^y_2$, $A^y_3$>>          (x >y)

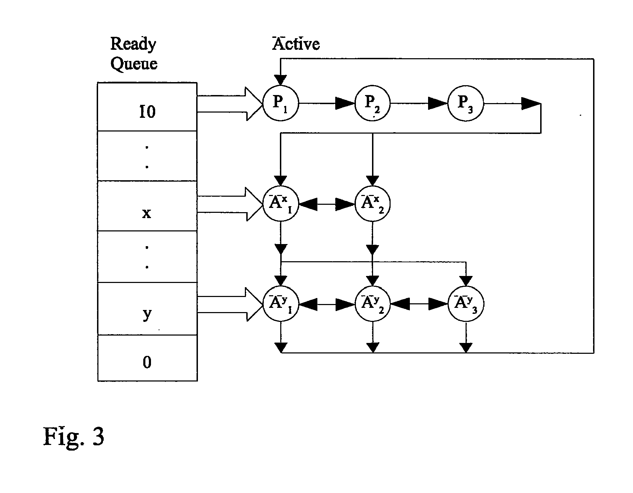

In SDREAM, each task is assigned a priority, with higher priority values representing the need for quicker responsiveness. SDREAM provides a two-level scheduling scheme according to different task types. For periodic tasks, SDREAM adopts a "FIFO (First-In, First-Out)" scheduling scheme and tasks are selected according to their arrival order in the ready queue. Two tasks that run at the same priority, e.g. 10, can use FIFO scheduling to ensure mutual exclusion to a shared resource. Neither task will be pre-empted by the other while it is executing. For example, if they shared a memory segment, each of the two tasks could update the segment without resorting to some forms of semaphoring. A task selected to run continues executing until it voluntarily relinquishes control, i.e. it is completed. The response time and execution time of periodic task are very short and deterministic in SDREAM, so they are used to respond to I/O interrupts or to monitor actuators. Moreover, all periodic tasks share the system stack, thus there is no overhead of context switch when running periodic tasks. For priority tasks, SDREAM adopts a "priority-based pre-emptive" scheduling scheme and tasks are selected according to their priority level. The scheduler always allows the higher-priority tasks to run first. The low-priority tasks will run when the higher-priority tasks have finished. When the scheduler determines a task needs to run immediately, the "pre-emptive" property enables it to stop any priority task at any execution point out of the critical section. Priority tasks are therefore suitable for strict time constrained applications. According to the same priority level tasks, the "round-robin" scheduling scheme is adopted. In round-robin scheduling, a time-slice is associated to a task, which means the lifetime of a task. Once it consumes its time-slice, a task is pre-empted and the next ready task at the same priority level will get the system control. A task selected to run continues executing until it voluntarily relinquishes control or consumes its time-slice. In fact, apart from time slicing, round-robin scheduling is identical to FIFO scheduling. Figure 3 illustrates the task scheduling scheme adopted in SDREAM corresponding to the application as defined in the expression above. The three periodic tasks have the highest priority values 10. They are run in sequence by "FIFO" scheduling. After the periodic tasks are completed, a higher-priority task ($A^x_1$) is selected to run. It has a time-slice. If it is not completed but consumes its time-slice, a next ready task ($A^x_2$) at same-priority level will pre-empt the CPU control. Until the tasks with higher-priority (x) are completed or voluntarily relinquish control, the low-priority tasks (y) are able to obtain the CPU control. Each time the scheduler is triggered by a timer interrupt, the following 5 steps are carried out. These 5 steps together are called "task switching" or "context switching":

determine whether a currently running task should continue to run. If not, suspend said current task and goto next step;
determine which task should run next;
save an environment of the task that was suspended, so it can continue later
set up a running environment of an other task that will run next;
allow this other task to run.

Meta Definitions and Descriptions
Definitions of symbols:

// : Concurrent or Parallelism operation

$\rightarrow$ : Precedence operation
Description of task scheduling of an application:

$$P1 \rightarrow P2 \rightarrow P3 \rightarrow (A^x_1 \text{ // } A^x_2) \rightarrow (A^y_1 \text{ // } A^y_2 \text{ // } A^y_3)$$

Rules:

$$(A^x_1 \text{ // } A^x_2) = (A^x_2 \text{ // } A^x_1)$$

$$(A^y_1 // A^y_2 // A^y_3) = (A^y_2 // A^y_3 // A^y_1) = (A^y_3 // A^y_2 // A^y_1)$$

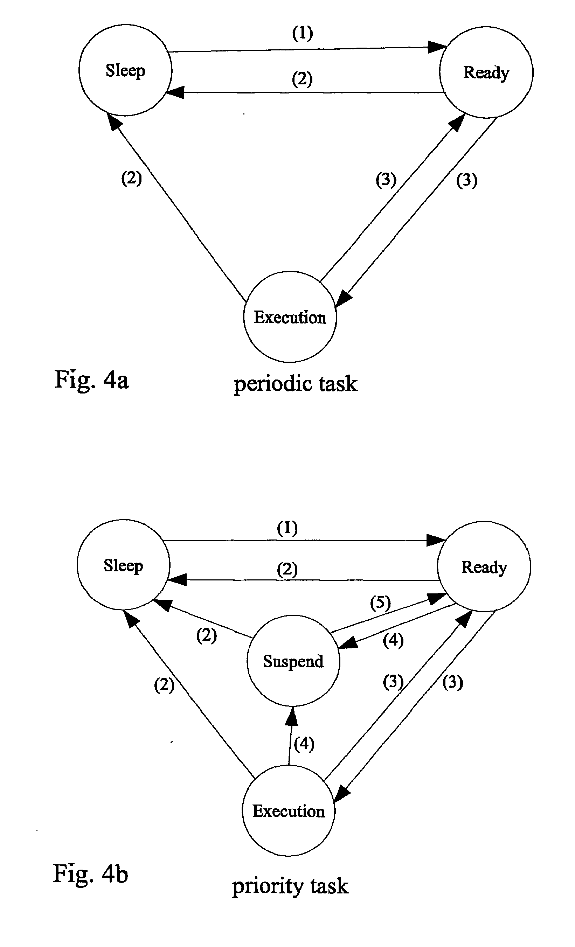

Like task scheduling, SDREAM has different task states definitions according to the task types. Three states are defined for the periodic task to describe the task operations: Sleep, Ready and Execution. The "Execution" state is a transient state which is held only when the task is running. For an activated periodic task, it is held on the "Ready" state at most of time. The "Sleep" state shows that a task has been created but not been activated in present. The state transition diagram of a periodic task is shown in Figure 4a. When compared to the periodic task, the priority task has one more state named "Suspend". If a running condition is not available, the current priority task will be blocked and the task state will be changed to "Suspend". If a running condition is available, the suspended priority task will be woken up to resume running and the task state will be changed to "Ready". The state transition diagram of priority task is shown in Figure 4b.

The transition functions table below (Table 1) shows the operations of task state transitions.

Table 1

| Transition Functions | |
| --- | --- |
| (1) : | Activate Task |
| (2) : | Deactivate Task |
| (3) : | Scheduler |
| (4) : | Suspend Task |

(continued)

| Transition Functions | |
| --- | --- |
| (5) : | Resume Task |

SDREAM does not support the dynamic creating or releasing a task. The number of tasks is pre-configured by the user at compile time. The "Sleep" state is the original and terminal state of a task. Each task is initialized to "Sleep" state when it is created. Four system functions and the scheduler together enable a task to transit over different states. The four transition functions are described as follows:

DeactivateTask is called to terminate a task when this task is completed (priority task) or an exception happens. The task's state is changed to "Sleep".

ActivateTask is called to activate a "Sleep" task when this task is assigned to an application. The task's state is changed to "Ready".

SuspendTask is called to block a task when the running condition of this task cannot be satisfied in present. The task's state is changed to "Suspend".

ResumeTask is called to resume a task when the running condition of this task is satisfied. The task's state is changed to "Ready".

Meta definitions and descriptions
Definitions of symbols:

c : Operation of inserting a task into a task queue
⊃ : Operation of extracting a task from a task queue
M (A, Ss, Sd) : Change the state of task A from Ss to Sd

Ss : Task source state

Sd : Task destination state

rea : Task READY state

sus : Task SUSPEND state

slp : Task SLEEP state

‖...‖ : Critical section or Atomic Operation, disable interrupts
*SC*(A) : Save the context of task A
RC(A) : Restore the context of task A
SCHED : Call scheduler
I : 'Or' Operation

Descriptions of transition functions:

DeactivateTask: || $M(A^x$ , rea|sus, slp) ||;

ActivateTask: || $M(A^x$ , slp, rea) ||;

SuspendTask: || $M(A^x$ , rea, sus);

$$IF (A^x == runTask) THEN$$

$$SC(A^x) \rightarrow RC(scheduler) \rightarrow SCHED;$$

END IF||;

ResumeTask: || M(Ax , sus, rea);

$$SC(runTask) \rightarrow RC(scheduler) \rightarrow SCHED ||;$$

The inter-task communication and synchronization models of the conventional embedded operating systems are space couple or time couple. They use classical concepts such as: message passing, mailbox, port (Inter-task communication) and semaphore, mutex (task synchronization). These models were originally developed for general purpose multi-user applications, but are not adapted to the tiny resource embedded real-time applications. The key idea of the inter-task communication and synchronization service provided in SDREAM is named "Tuple". The "tuple" model originates from the LINDA concept. LINDA is a parallel programming language which supplies a number of operations or primitives that provide the ability for tasks to communicate. It uses a shared structure, called a tuple space, which is an unordered collection of tuples. A tuple is an ordered collection of elements, with each element having a value and an associated type. Tasks insert tuples into the tuple spaces, and other tasks can then retrieve those tuples using a related matching process. LINDA provides asynchronous communication between tasks via tuple. The only way that two tasks can communicate with each other is to do so through tuple spaces using tuples and the LINDA primitives. The basic LINDA primitives of posting and reading a tuple are: OUT() and IN(). When a task wants to send a message, it calls the IN() primitive by posting a tuple in the tuple space. When a task wants to read a message from a related tuple, it calls the OUT() primitive: if this tuple template matches (message is available), a message will be extracted from the tuple space; Otherwise, this task is suspended. The message is extracted based upon matching of contents, thus the inter-task is not space couple. The inter-task communication in LINDA is asynchronous, thus it is not time couple. The tuple model used in the present invention therefore enables asynchronous communication between tasks, and is extensible to support a multi-process parallel programming model and suitable for distributed multiple application processing. However, the original LINDA concept is not adequate for distributed hard real-time parallel processing, because the time of inter-process communication (IPC) is not deterministic and increases dramatically when the numbers of processes and processors are important, for example, more than 100. To overcome these problems, SDREAM provides two very light IPC primitives: Snd() and Rcv(), which are simplified implementations of IN() and OUT () of LINDA. Instead of using all the message content to match and to extract a tuple, only one numeric value (KEY) is used to identify a tuple. Thus, the numeric identifier and the type of tuples may be assigned to local, shared or distributed memory, which are statically configured by the user in SDREAM. All the message buffers are mapped into an array of bytes associated to a table of tuple which realizes the tuple space (input/output buffer table). Thus, the content of a message may be accessed immediately when a tuple is not empty, and the operation time of tuple template matching is very short and deterministic in spite of the number of tasks or processors. The SDREAM is a tuple-based message-driven system. Each message, no matter network data, sensor acquisition or task data, must associate with a unique tuple. When a message received from the network, sensors, or other tasks arrives at its associated tuple, it will drive a task scheduling and subsequently wakeup the tasks suspended on the tuple.

Meta definitions and descriptions

Definitions of symbols:

T(key) : A tuple with an ID: key

B(key) : A used data buffer associated to the tuple(key)
M : A message
N : A waiting time
E(err) : Operation of error report, err is error code
+ : Operation of inserting a message into a tuple
- : Operation of extracting a message from a tuple
-- : Operation of decrement instruction
# : Length of an object :

#B(key) : length of a used data buffer B in the tuple(key)
#T(key) : Maximum length of the tuple(key)
#M : Length of a message
#Q(key) : Number of suspended tasks in the tuple (key)

Descriptions of system primitives: SND() and RCV()

SND:

|| WHILE (#B(key) > (#T(key) - #M) && N >0 ) DO ||

N--;

ENDWHILE

|| IF (#B(key) ≤ (#T(key) - #M)) THEN

B(key)+m;

IF (#Q(key) >0) THEN

ResumeTask;

ENDIF

ELSE E(err);

ENDIF ||

RCV:

|| IF (#B(key) ≥ #M) THEN

B(key)-m;

ELSE SuspendTask;

END IF ||

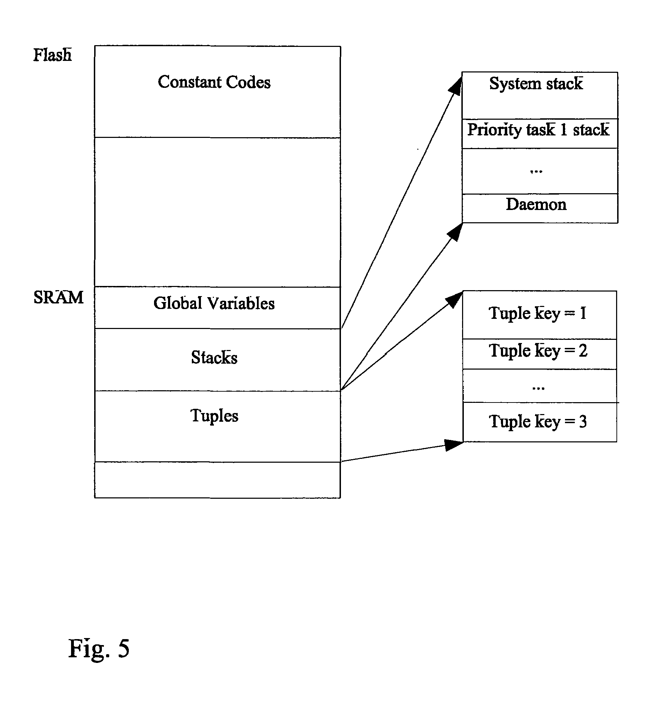

SDREAM according to the present invention has tiny memory requirements and can reside in the internal memory of a microcontroller, for example, residing in a static 60KBytes program memory and a 2KBytes dynamic RAM of a Texas Instruments MSP430F149 microcontroller. Most microprocessors and microcontrollers have only one execution mode, i.e. there is no supervisor mode, and do not have a MMU to allow memory protection or virtual memory management. SDREAM also has a unique physical memory address space (user and kernel). The same address space enables memory sharing between system kernel and tasks. Therefore, SDREAM reduces greatly the overhead of memory usage and avoids buffer copying. There are three major memory usage areas in SDREAM: stack space, tuple space, and code and data space. The memory map of SDREAM is shown in Figure 5 :

Code and Data space: the executable codes and program constants are invariable, which are normally stored in the program memory of processor. The global variables are stored in the public area of RAM and the private variables (priority task) are stored in the private stack of each task;

Stack space: each priority task has a private stack on which the accesses of other tasks are refused. The stack space contains task context, function pointers and parameters as well as functions' private variables. Since there is no MMU for memory protection, the function calls in SDREAM are not recursive, thereby minimizing system stack size and avoiding stack overflow. Each stack size is predetermined by the application developer according to the application;

Tuple space: All tasks and interrupt service routines in SDREAM share a tuple space which consists of a set of non-ordered tuples. A task has a right to read and write data in a tuple if it gets a tuple access key. Hence, tuple space is a data exchange area for kernel and tasks while the related KEY provides the memory protection. Each tuple allows simultaneously unique task writing and multi-task reading operations at the same time. Each tuple memory is a circular buffer, thus the reading operation will generally be faster than the writing operation.

The SDREAM of the present invention only supports static memory allocation. The allocation of memory with fixed position and fixed size is controlled by the compiler in a predetermined fashion. Neither dynamic memory i.e. no malloc, nor heap memory is provided in the SDREAM of the present invention. The static memory allocation mechanism avoids memory fragmentation caused by continuous dynamic allocation and de-allocation and also reduces the overhead of memory management. The main memory allocations are :

Task table i.e. fixed numbers of periodic and priority tasks;
Stacks for system and priority tasks, i.e. fixed sizes;
Tuple table, i.e. fixed sizes.

The interrupt handling in a kernel is normally closely tied to the hardware on which it is intended to run. Interrupts can be caused by a variety of conditions, both internal and external to the processor. An interrupt forces an interrupt service routine (ISR) to a predefined address in the interrupt vector. Many portable RTOSs rely on a compiler-provided mechanism for interrupt handling where C functions can be used as interrupt service routines. In this general approach, all registers are saved and restored while handling interrupts, which thus has an overhead of interrupt-oriented context switching. The compilers of the two preferred SDREAM hardware platforms, i.e. TMS320C5410 and MSP430F149 microcontrollers, adopt this approach. The external interrupts in SDREAM are normally the hardware events from the sensors and the network devices. They can be periodic events, e.g. data sampling from sensors, or aperiodic events, e.g. uncertain network connection requirements. SDREAM uses a small footprint method for handling external interrupts. Instead of function calling or task scheduling to handle interrupts, SDREAM places directly the related actions into interrupt service routines. The most important action of external interrupts in SDREAM is to copy data, preferably one or two bytes at a time, between the interrupt registers and associated tuples. The actions of ISRs are straightforward and can be performed in a very short and deterministic time. Moreover, since there are no task scheduling and no complex internal actions in external ISRs, SDREAM only needs to save and restore only parts of the general registers. The "part of registers" feature enables SDREAM to speed interrupt handling and to reduce the overhead of interrupt-oriented context switching. SDREAM internal interrupt is normally a periodic timer interrupt caused by system clock. In general, a processor contains one or multiple programmable interval timers that generate periodic timer interrupts. The time interval of the timer interrupt, i.e. the clock tick, is configurable and may vary from platform to platform. All program timer-variables used in system kernel and applications are managed by the ISR of timer interrupt. Each time a timer interrupt happens, the timer-variables will be changed. When the value of the scheduling timer-variable reaches the threshold, a task scheduling occurs. In this condition, all general registers will be saved into the stack of current running task, and then the scheduler is called in ISR to select a next task.

In one particularly preferred embodiment of the present invention, SDREAM is substantially implemented in the C programming language, except for a few hardware-related elements which are preferably implemented in assembly language. Since SDREAM is intended for wireless sensor network applications, the preferred microcontrollers of choice are the TI MSP430F149 16-bit microcontrollers, available from Texas Instruments, USA. In addition, in another preferred embodiment, it is also possible to implemented SDREAM on the TMS320C5410 16-bit digital signal processor. The implementation of the SDREAM architecture will now be described in detail. The main data structures of SDREAM are presented as is an overview of kernel initialization during system startup, and a description is made of the SDREAM scheduler, which comprises a two-level scheduling scheme for periodic and priority tasks. The task state transition functions: InitializeTask, ActivateTask, DeactivateTask, SuspendTask and ResumeTask, are also described in detail. In addition, the preferred implementations of two task communication primitives: RCV and SND are also given.

The kernel initializes itself before the applications run. The task and tuple are preconfigured by the user and initialized by the system startup program, i.e. normally by the main() function, in which hardware initialization operations are also performed.

Data structures

To simplify system implementation, a simple buffer structure such as a table or a queue is used in SDREAM. SDREAM has one periodic task descriptor table (PrdtskTable), one priority task descriptor table (PritskTable) and one tuple table (TupleTable). The members of these tables are Prdtsk, PriTsk, and Tpl respectively. Table 2 illustrates the structure of the periodic task descriptor. The task address pointer is stored in the TskAdr field. The Tid is a task identifier and its value indicates the index of a task element in task descriptor table. The value of Prd field indicates the time period of a periodic task, and the value of CurClk filed indicates the residual time interval for next task execution. The CurClk value is initialized to the task period (Prd) at the beginning of task execution, and is decremented by one at each task scheduling. A period task will keep to perform until the value of CurClk is equal to zero or the completion of task. The value Pri field indicates the task's priority. The periodic task has the highest priority value, i.e. 10 in SDREAM. This Pri value is unchangeable while the system is running. The Sta describes the task state. The periodic task has three task states: Sleep, Ready and Execution.

Table 2 : Structure of Periodic Task Descriptor

| PrdTsk | |
|---|---|
| Struct PrdTsk { | |
| short TskAdr; | /* Task Address pointer*/ |
| char Tid; | /* Task Identifier */ |
| char Prd; | /* Task Period */ |
| char CurClk; | /* Task Running Time */ |
| char Pri; | /* Task Priority */ |
| char Sta; | /* Task State */ |
| }; | |

The structure of the priority task descriptor is shown below in Table 3:

Table 3 : Structure of Priority Task Descriptor

| PriTsk | |
|---|---|
| Struct PriTsk { | |
| PriTsk | |
| short TskAdr; | /* Task Address Pointer*/ |
| char Tid; | /* Task Identifier */ |
| char Prd; | /* Task Period */ |
| char CurClk; | /* Task Running Time */ |
| char Pri; | /* Task Priority */ |
| char Sta; | /* Task State */ |
| short IniSp; | /* Start Address Pointer of Task Stack */ |
| short CurSp; | /* Current Address Pointer of Task Stack*/ |
| }; | |

PriTsk has two additional elements compared to PrdTsk: IniSP and CurSP. The identically named elements of the priority task have the same significance as for those of the periodic task. The Prd and CurClk indicate the maximal and residual lifetimes of a priority task. These two variables are useful for time sharing between same-level priority tasks. There are four states for priority tasks: Sleep, Ready, Suspend, and Execution. The priority tasks are arranged in the task descriptor table according to the sequence of task priority, i.e. from high to low priority. Each priority task has its private stack space. The value of IniSP indicates the start address of a task stack and the value of CurSP points to the address position of the current execution point in a task stack.

The Tpl structure defines a tuple, shown in Table 4. The Sta filed shows the tuple state. If a message is unavailable in a tuple, the wait tasks in this tuple will be blocked and the Sta value is set to NOREADY; whereas, if a message is available in this tuple, the Sta value is set to READY and the suspended task will thus be woken up. The Key is the access identifier of a tuple which also implies the type of a tuple. The MsgNb indicates message numbers in a tuple buffer. The WrMsgPtr and RdMsgPtr are the reading and writing pointers of the tuple buffer which point to the current reading and writing buffer addresses. The StaBfrAdr and EndBfrAdr indicate the start and end address of this tuple buffer respectively. The tuple buffer is a circular buffer space. If the WrMsgPtr or RdMsgPtr is equal to the EndBfrAdr, it will automatically jump to the StaBfrAdr. Hence, in order to ensure reliability of SDREAM, reading operations must be faster than writing operations.

Table 4 : Structure of Tuple Descriptor

| Struct Tpl { | |
|---|---|
| char Sta; | /* Tuple State */ |
| char Key; | /* Tuple Key */ |
| unsigned char *WrMsgPtr; | /* Current Write pointer */ |
| unsigned char *RdMsgPtr; | /* Current Read pointer */ |
| short StaBfrAdr; | /* Start address of Tuple Buffer*/ |
| short EndBfrAdr; | /* End address of Tuple Buffer */ |
| short MsgNb; | /* Message Number */ |
| }; | |

The SDREAM system startup program contains two main steps: system initialization and task scheduling. When a processor is reset, it obtains the address of the system startup program, the main() function is called and the kernel is running, as shown below.

## System startup routine :

## Main

```
main ()
{
        /*Initialisation of software: variables, tasks, tuples, etc... */
        Init_Software()

        /*Initialisation of hardware platform: IO interrupts, timer, clock, etc... */
        Init_Hardware()

        /*Start Scheduler*/
        While(1) {
                /*Goto the system stack IniSysSP*/
                SysSPPtr = IniSysSP;
                Set_SP();
```

```
                              /*Call function Schedule()*/
                              SysTskPtr = (unsigned short)schedule;
                              Set_Tsk();

                              /*Run function schedule()*/
                              Run_Tsk();
                    }

}
```

The definitions and the configurations of tasks and tuples are carried out by the developer in the system header file. The numbers of periodic tasks, priority tasks, and tuples are pre-configured, and the buffer size of stacks and tuples are pre-allocated. The *Init_software()* function initializes system variables, two classes of tasks and the tuples. It loads the tasks by calling the system function *ActivateTask().* An associated stack will be assigned to a priority task in this system function. The *Init_hardware()* function initializes all hardware-related information, such as system clock frequency, I/O interrupts, timer period, sample frequency of sensor, and system mode, etc. Subsequently, the scheduler is called, and the system enters an infinite loop. In a preferred example of SDREAM implementation in MSP430F149, there is one periodic task, four priority tasks, including the daemon, and five tuples. The four stacks are pre-allocated to priority tasks, which have different sizes according to the practical applications. In this preferred example, the first priority task is dedicated to network communication based on the point-to-point protocol (PPP), named *Net_PPP()* with a priority of 9; the second task aims to real-time transmit sensor signals, named *ECG_SER()* with a priority of 9; the third task is a minimal Web Server, named *WEB_SER()* with a priority of 8; and the fourth task is a daemon program with the lowest priority of 0. The stack sizes for the four priority tasks identified in this example are, in bytes : 100, 120, 120, and 30 respectively. This instance pre-allocates five tuples in the tuple space, including: USART (Universal Synchronous and Asynchronous Receiver and Transmitter) serial output tuple (OutUartBfr), USART serial input tuple (InUartBfr), sensor input tuple (McbSpBfr), UDP input tuple (UdpBfr), and TCP input tuple (TcpBfr), the tuple buffer sizes of which are, in bytes : 256, 128, 130, 4, and 6 respectively. In order to avoid a data race condition, SDREAM supports only a single UDP connection and a single TCP connection at a time.

SDREAM according to the present invention provides a two-level scheduling scheme in view of two classes of tasks. The periodic task adopts a "FIFO" scheduling scheme. All periodic tasks have the highest priority level 10, and they are executed sequentially according to their orders in the periodic task table. The priority tasks adopts a "priority-based pre-emptive" scheduling scheme. They have priority levels ranging from 9 to 0 (from high to low). A high priority task can pre-empt the CPU control of lower-priority task. This scheduling scheme supports time sharing between tasks of same priority. The priority task descriptor table ranks from highest to lowest priority and the index of each member of the table corresponds to the task identifier. The "priority-based pre-emptive" scheduler algorithm is implemented to elect a highest priority task and to allow sharing time between tasks as shown below:

```
for(i=Next_Index;i<Maximum_Number_Of_Priority_Process; i++)
{
        if(Priority_Process[i].Status==READY) {
                The process having i identifier is elected,
                break;
        }
}
/*
        Set Index to next priority process having the same priority,
        it allows time sharing
*/
if(Priority_Process[i+1].Priority == Current_Process_Priority){
        Next_Index = i+1;
}
else {
        Next_Index = 0;
}
```

Like the priority task, periodic tasks are stored in the periodic task descriptor table, but they are non-ordered because all the periodic tasks have the same priority, i.e. 10. The index of the table corresponds to the periodic tasks identifier and all the elected periodic tasks must be executed and completed before the next task scheduling. The scheduling algorithm is shown below:

```
for(index=0;index<Maximum_Of_Periodic_Process; index++)
{
        Update Periodic_Process[index].Period;
}
for(index=0;index<Maximum_Number_Of_Periodic_Process; index++)
{
        if (Periodic_Process[index].Period <= 0){
                /*
                periodic process is READY
                */
                Push The Periodic_Process[index].Address on the system
                stack;
                Update Periodic_Process[index].Period;
        }
}
```

SDREAM according to the present invention provides five system functions to manage task state transition for a periodic or priority task : InitializeTask, ActivateTask, DeactivateTask, SuspendTask, ResumeTask. In SDREAM implementations of the present invention, a task can be "sleep", "suspended" or "ready" by only changing its state variable without moving to task state queues. This approach is simple and efficient when few tasks are involved.
The Initialize Task function is called to initialize the periodic and priority tasks in the system startup program. The configurations of tasks are set manually and the fields of two task tables are filled by the developer during the compile time. The initial state of all tasks is "Sleep". An example of the InitializeTask function is presented below. It has six parameters:

Type : The type of the task (periodic task : 0, priority task: 1);
Tid : The index of the task in associated task queue;
TskAdr : The address of the task;

19

Pri : The priority of the task (periodic task: 10, priority task: from 0 to 9);
Prd : The period of the task;
SP : The stack pointer of the priority task.

## InitializeTask :

InitializeTask (char Type, char Tid, short TskAdr, char Pri, short Prd_SP)

```
{
        if (Type == 0) {
                /* Initialization of periodic task */
                PrdtskTable[ Tid ]. TskAdr = TskAdr ;
                PrdtskTable[ Tid ]. Tid = Tid ;
                PrdtskTable[ Tid ]. Pri = Pri ;
                PrdtskTable[ Tid ]. Sta = SLEEP ;
                PrdtskTable[ Tid ]. Prd = Prd_SP ;
                PrdtskTable[ Tid ]. CurClk = Prd_SP ;
        }
        else if (Type ==1 ){
                /* Initialization of priority task */
                PritskTable[ Tid ]. TskAdr = TskAdr ;
                PritskTable[ Tid ]. Tid = Tid ;
                PritskTable[ Tid ]. Pri = Pri ;
                PritskTable[ Tid ]. Sta = SLEEP ;
                PritskTable[ Tid ]. IniSP = Prd_SP ;
                PritskTable[ Tid ]. CurSP = Prd_SP ;
        }
}
```

The ActivateTask function activates a "sleep" task into a "ready" state. It is normally called during task initialization in the system startup program or when a user manages a task in an application. The algorithm description of this function is presented below and has two parameters: Type and Tid.
ActivateTask
DISABLE_ALL_INTERRUPTS:
IF current task is SLEEP THEN Modify the state of current task to READY;
ENDIF
ENABLE_ALL_INTERRUPTS;
The DeactivateTask function is called to terminate a task when this task is completed (priority task) or some abnormal exceptions happen. The task state is changed from "ready" or "suspend" to "sleep". The algorithm description of this function is presented below. It has the same parameters as those of ActivateTask.

## DeActivateTask

DISABLE_ALL_INTERRUPTS;

```
IF current task is READY or SUSPEND THEN Modify the state of current task to SLEEP;
ENDIF
ENABLE_ALL_INTERRUPTS;
```

In SDREAM, only the priority task can be suspended and resumed. When the running condition of a task is not satisfied, such as a receive message is not available, this task will be suspended by calling SuspendTask, and the task's state is set to "suspend". If the task in question is the current running task, context switching will occur, and the scheduler is thus called. The algorithm description of the SuspendTask function is given below. It has one parameter: the index identifier Tid on the priority queue.

## SuspendTask

```
DISABLE_ALL_INTERRUPTS;
IF current task is READY THEN Modify the state of current task to SUSPEND;
IF current task is the running task THEN Save current context;
Restore system context;
Run Scheduler ;
ENDIF ENDIF ENABLE_ALL_INTERRUPTS;
```

When the running condition of a suspended task is satisfied, the ResumeTask function is called to resume the execution of this task, and the task's state is set to "ready". If the priority of this suspended task is greater than the one of current running task, context switching will occur, and the scheduler is called. The algorithm description of this function is given hereafter. It has the same parameters as the SuspendTask function.

## ResumeTask

```
DISABLE_ALL_INTERRUPTS;
IF current task is SUSPEND THEN Modify the state of current task to READY;
IF the_priority_of_current_task > the_priority_of_the_running_task THEN Save the context of the running
task;
Restore system context;
Run Scheduler ;
ENDIF
ENDIF
ENABLE_ALL_INTERRUPTS;
```

The service of inter-task communication and synchronization consists of a tuple space, or a collection of tuples, and two system primitives, RCV and SND. These two basic primitives are not only responsible for the task-to-task communications, but also for task-to-device communications, such as the sensors and network mediums. By calling RCV and SND primitives, a task posts a message into a tuple or reads a message from a tuple The messages stored in tuples conform to the format of AHDLC (Adaptive High Level Data Link Control).

When a task wants to extract data from a related tuple, identified by a key, a RCV primitive is called. If a message is available in a tuple, RCV translates the original data from the message in AHDLC format, and then stores the data into the task buffer. Consequently, the RCV primitive returns and this task continues. However, if no message is ready, the task will be suspended until its running condition is satisfied. The algorithm description of RCV primitive is shown below. It has three parameters:

Key : a tuple access identifier;
MsgPtr : the task buffer pointer to store the message;

SP : the maximum message size that the task can receive.

### RCV

```
DISABLE_ALL_INTERRUPTS;
IF (message is NOREADY in the tuple) THEN SuspendTask;
ELSE Extract and store data into task buffer from a message with AHDLC format;
ENDIF
ENABLE_ALL_INTERRUPTS;
```

When a task wants to write data to a related tuple, a SND primitive is called. If the tuple has enough empty space for current data, the SND encapsulates the data into a message in the AHDLC format, and then stores the message into the tuple buffer. The AHDLC format thus allows better utilization of network bandwidth with word-oriented communication. Consequently, the SND returns and the task continues. If a data size is greater than the free space size of the tuple, the task will wait a special waiting time. After a waiting time, if there is still not enough memory, an error will be reported. The algorithm description of the SND primitive is shown below. It has the same parameters as those of RCV. The MsgPtr and Msglen indicate the task's data buffer pointer and data size respectively.

### SND

```
DISABLE_ALL_INTERRUPTS
DO
N--;
WHILE (data_size > free_buffer_size_of_tuple && N > 0)
IF (data_size > free_buffer_size_of_tuple) THEN Err(errocode) ;
ELSE
Encapsulate data into a message with AHDLC format and store it in the tuple;
ENDIF
ENABLE_ALL_INTERRUPTS;
```

The implementation with the SDREAM architecture was ported to two kinds of processor architectures: microcontroller MSP430F149 and digital signal processor TMS320C5410. The memory and power consumption of SDREAM on these two processor architectures was measured, execution times of system primitives and functions calculated, system latencies determined, thereby enabling comparison of the SDREAM architecture with those of other RTOSs and TinyOS.

The SDREAM architecture was implemented and evaluated on two hardware platforms: TMS320C541 0 and MSP430F149. The TMS320C5410 is a 16-bit fixed point digital signal processor having a 100 MHz clock frequency. The TMS320C541 0 has 80KWs (W 'word' = 16 bits) of internal memory (16KWs of RAM and 64KWs of ROM) and three serial ports.

The MSP430F149 is a 16-bit RISC microcontroller having a 8MHz clock frequency. The MSP430F149 has 62KB (1 B=8bits) of internal memory (60KB of flash memory and 2KB of RAM), two serial communication interfaces (USART) and a fast 12-bit Analogic-to-Digital Converter (ADC) (8 channels).

The following Table 5 presents the respective sizes of memory consumption of two SDREAM implementations on the two different processors. The first is a minimal kernel containing the essential components of a real-time kernel: task primitives, scheduler and tuple space. The second is a complete kernel which integrates an embedded TCP/IP protocol stack (PPP, IP/ICMP, TCP/UDP, and HTTP/SNMP) and several applications, including sensor signal acquisition and transmission application, SNMP agent and Web server. It has one periodic task, four priority tasks, including a daemon, and five tuples. The memory consumption of SDREAM is very low, and is ideally suited for tiny embedded devices, especially for a WSN node, which has very severe resource constraints.

Table 5 : comparative memory consumption across processors

| Processor | Code size | Data size |
|---|---|---|
| Minimal implementation | | |
| TMS320V5410 (W, 'word'=16 bits) | 4,393 | 606 |
| MSP430F149 (B, 'Byte'=8bits) | 4,762 | 327 |
| Complete implementation | | |
| TMS320V5410 (W, 'word'=16 bits) | 18,398 | 7,191 |
| MSP430F149 (B, 'Byte'=8bits) | 19,120 | 1,972 |

SDREAM can be configured to operate in low-power mode, thereby supporting the ultra-low power applications of WSN. Table 6 shows the power consumption of different operating modes on a WSN system according to the present invention. The MSP430F149 provides different levels of operation modes to support various requirements for ultra low power consumption. The low-power modes can enable or disable the CPU and the clocks. In addition, specific peripherals can be enabled or disabled to further reduce power consumption. In the general operating mode of MSP430F149, the operating current is on average 2.24 mA at 8 MHz clock frequency and 3V supply voltage. However, in the low-power mode 3 (LPM3), there is only 2 pA power consumption under the same conditions. When the SDREAM system is in the idle state, i.e. running the daemon task, LPM3 is selected by setting several bits in the status register of MSP430F149. Immediately after the bits are set, the CPU and some clocks halt and all internal activated buses stop until an interrupt request or reset occurs. Peripherals that operate with these inactive clocks are also inactive. A WSN system according to the present invention adopts the Bluetooth wireless communication access medium. The BlueWAVE, a Bluetooth serial wireless cable used in a WSN according to the invention, provides different operating modes to reduce power consumption. In the general connected mode, the average power consumption of a wireless connection is 92.5 mA at 3V; while in sleep mode, the average power consumption is only 6.25mA. When there is no data transmission in SDREAM, Bluetooth can be set to sleep mode until a data transmission request occurs. If there is no transmission request in a long-term or the peer is unreachable, the power supply for the Bluetooth can be cut off manually by the user. The Bluetooth is reset until the user requests a new Bluetooth connection.

For most WSN applications, such as environmental data collection, security monitoring, and sensor node tracking, the sensor data sampling frequency is low and SDREAM is in the idle state most of time. SDREAM can thus operate in low-power mode most of the time to reduce overall power consumption. In one preferred device of the invention, the default power source is a Lithium-Ion video battery with the capacity of 700mAH at 3V. A SDREAM application system, implementing 500Hz four-channel data sampling and real-time wireless connection, can run 7.4 hours in the general operating mode, whereas in low-power mode, the runtime is extended to 112 hours or more.

Table 6 : Power Consumption of different Operating modes in a WSN device according to the invention

| Voltage = 3V, Capacity =700mAH | Normal Mode | Low-Power Mode |
|---|---|---|
| MSP430F149 (8Mhz) | 2.24mA | $2\mu A$ (LPM3) |
| BlueWAVE | 92.5mA | 6.25mA |
| Lifetime | 7.4h | > 112h |

The numbers of instruction cycles of the system primitives and functions on two different processors were evaluated respectively. The runtime of each primitive or function was calculated in view of the different clock frequencies on the MSP430F149 (8MHz) and the TMS320C541 0 (100MHz). Table 7 presents the results of the performance evaluation of system primitives and functions. The evaluation version of SDREAM is a complete implementation which is configured with one periodic task, four priority task and five tuples.

Table 7 : Performance Evaluation of system primitives and system functions in SDREAM

| Primitives | Conditions | MSP430F149 | | | | TMS320C5410 | | | |
|---|---|---|---|---|---|---|---|---|---|
| | | Cost (cycles) | | Time ($\mu$s) | | Cost (cycles) | | Time ($\mu$s) | |
| | | Min | Max | Min | Max | Min | Max | Min | Max |
| IntializeTask | | 121 | 156 | 15.125 | 19.5 | 60 | 79 | 0.6 | 0.79 |
| ActiveTask | | 43 | 74 | 5.375 | 9.25 | 43 | 47 | 0.43 | 0.47 |
| DeactiveTask | | 53 | 83 | 6.625 | 10.375 | 49 | 58 | 0.49 | 0.58 |
| SuspendTask | Min : suspend a task in Ready state<br>Max: suspend a running task | 106 | 231 | 13.25 | 28.875 | 75 | 331 | 0.75 | 3.31 |
| ResumeTask | Min : a task having lower priority<br>Max : a task having higher priority than current running task | 77 | 203 | 9.625 | 25.375 | 57 | 318 | 0.57 | 3.18 |
| InitTuple | | 114 | 134 | 14.25 | 16.75 | 134 | 160 | 1.34 | 1.6 |
| SND | Send a message with a length of 8 bytes | 405 | | 50.625 | | 542 | | 5.42 | |
| RCV | Receive a message with a length of 8 bytes | 411 | | 51.375 | | 548 | | 5.48 | |

In this static configurable SDREAM system, the execution time of system functions are determinate, which is between the minimal and maximal times. Moreover, because the size of a tuple message is limited and predefined, the execution times of SND and RCV primitives are thus predictable. The deterministic and predictable behavior of system primitives and functions is the most important property of a real-time operating system.

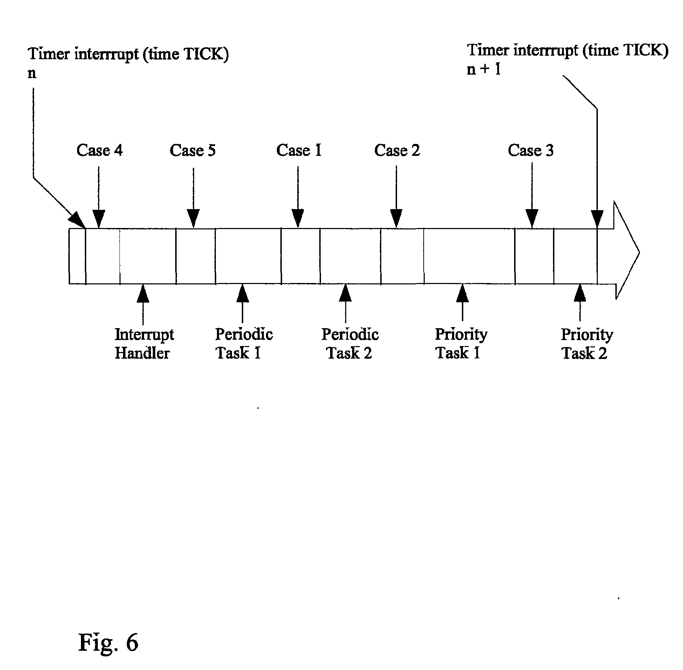

For a real-time operating system, the task scheduling scheme and the interrupt handling mechanism ought to be efficient and time predictable. Several system latencies are used to rate the performances of SDREAM task scheduling and interrupt handling, as shown in Figure 6. The time it takes for the system to switch from one task to another is called the task switch latency, or context switch latency. It is important that this latency is as short as possible to keep the task-switching overhead in the application to a minimum. In SDREAM, since two classes of tasks are defined, i.e. periodic and priority tasks, there are three latencies that can be used to evaluate the task scheduling scheme:

Periodic-to-periodic task switch latency: the time it takes for the system to switch from one periodic task to another periodic task. As periodic tasks don't have a local stack, there is no overhead for a context-switch between periodic task-switching. Moreover, the scheduling sequence of periodic tasks is determined in one-time scheduling. Thus, the periodic-to-periodic task switch latency is virtually equal zero;

Periodic-to-priority task switch latency: the time it takes for the system to switch from a periodic task to a priority task. There is one context-switch between the system and the priority task;

Priority-to-priority task switch latency: the time it takes for the system to switch from one priority task to another priority task. There is one context-switch between the system and the current running task and one context-switch between the system and the next priority task.

Real-time applications need the interrupt events to be handled as soon as possible. The interrupt latencies will be expected to be finite, and never exceed a predefined maximum. Two latencies are used to rate the performance of the interrupt handling mechanism:

Interrupt Response Latency: the time elapsed between the execution of the last instruction of the interrupted task and the first instruction in the interrupt handler. This is an indication of how fast the system reacts to an external event;

Interrupt Dispatch Latency: the time interval to go from the last instruction in the interrupt handler to the next task scheduled to run. This indicates the time needed to go from an interrupt level to a task level.

In order to evaluate system latencies, an SDREAM implementation according to the invention was configured with five periodic tasks, five priority tasks running at different priority levels and five tuples. The evaluation results of task switch latencies and interrupt latencies are presented in Table 8 and Table 9 respectively.

Table 8 : Performance Evaluation of Task Switch Latencies

| | MSP430F149 | | | | TMS320C5410 | | | |
|---|---|---|---|---|---|---|---|---|
| | Cost (cycles) | Time ($\mu$s) | Cost (cycles) | Time ($\mu$s) | Cost (cycles) | Time ($\mu$s) | Cost (cycles) | Time ($\mu$s) |
| | Min | Max | Min | Max | Min | Max | Min | Max |
| Periodic-to-periodic task switch latency | 0(285)[1] | 0(481) | 0(35.625) | 0(60.125) | 0(98) | 0(238) | 0(0.98) | 0(2.38) |
| Periodic-to-priority task switch latency | 284 | 519 | 35.5 | 64.875 | 146 | 299 | 1.46 | 2.99 |
| Priority-to-priority task switch latency | 482 | 645 | 60.25 | 80.625 | 930 | 1015 | 9.3 | 10.15 |
| 1 : the value in parentheses indicates the time of periodic task scheduling | | | | | | | | |

Table 9 : Performance Evaluation of Interrupt Latencies

| | MSP430F149 Time $\mu s$ | | | TMS320C5410 Time $\mu s$ | | |
|---|---|---|---|---|---|---|
| | Min | Avg | Max | Min | Avg | Max |
| Interrupt response latency | 47.72 | 61.25 | 93.75[1] | 2.82 | 2.873 | 4.133[1] |
| Interrupt dispatch latency | 60.25 | 68.25 | 86.5 | 2.56 | 3.2 | 3.68 |

[1] : The max value of Interrupt response latency indicates the case were SDREAM runs in a critical area, especially in RCV or SND primitives.

In order to entirely evaluate the performances of SDREAM, we compared it with other real-time operating systems and the WSN operating system TinyOS. The performance data of some popular RTOSs were obtained from evaluation reports of Dedicated System Experts. The evaluation reports for the following commercial operating systems are currently available:

RTX 4.2 from VenturCom, Inc.
Hyperkernel 4.3 from Imagination Systems, Inc.
VxWorks/x86 5.3.1 from WindRiver Systems Inc.
pSOSystem/x86 2.2.6 from Integrated Systems Inc.
QNX 4.25 from QNX Software Systems Ltd.
QNX/Neutrino1.0 from QNX Software Systems Ltd.

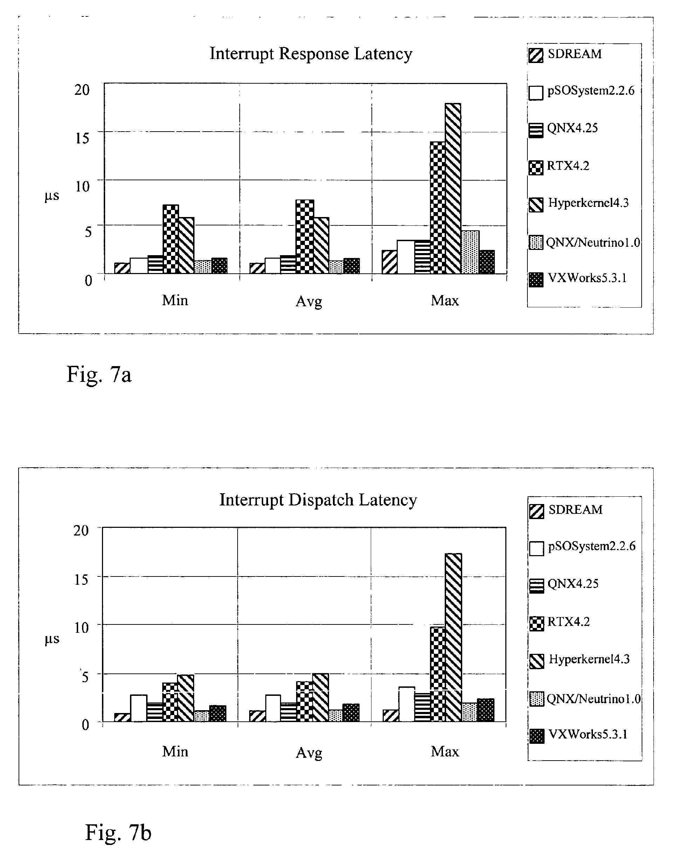

The evaluation platform adopted by Dedicated Systems Experts is an Intel Pentium 200MHz MMX based PC with a Chaintech motherboard. Time intervals are measured using external equipment: the PCI bus analyzer; and system peripherals are simulated by means of another external device: the PCI bus exerciser. The evaluation RTOSs are running with ten different priority-level tasks. In order to compare with the evaluation results of the SDREAM implementation on TMS320C5410, the inventors considered that the P200MMX has at least 300 MIPS (millions instructions per second) and the TMS320C541 0 has no more than 100 MIPS. Hence, the intrinsic execution time of P200MMX is 3 times faster than the TMS320C5410. The comparisons of two interrupt latencies between other RTOSs and SDREAM are shown in Figure 7a and 7b. In the two histograms, we can see that SDREAM has the smallest average interrupt response latency and the smallest average interrupt dispatch latency.

The TinyOS is a naturally multi-tasking event-driven system dedicated to wireless sensor applications. It has been tested on ATmega128 (4MHz clock frequency). The evaluation results are currently available. The minimal SDREAM implementation on MSP430F149 (8MHz clock frequency) was used to compare with the TinyOS. Since SDREAM was tested on a different platform to that on which TinyOS runs, the inventors only compared three system operations: schedule a task, context switch and hardware interrupt. The SDREAM is pre-configured with one periodic task and one priority task for this evaluation. The higher cost of task scheduling in SDREAM compared to TinyOS indicated the overhead of the scheduler function in SDREAM. The operation of context switching happens only between the system scheduler and a priority task. A hardware interrupt includes the hardware (hw) part and the software (sw) part. It was shown that in order to support true real-time multi-tasking operations, SDREAM had a greater system overhead when than TinyOS.

The SDREAM is a true real-time multi-tasking micro-kernel dedicated to embedded wireless sensor devices. A meta language is used to define and to describe the abstract manners of system primitives and operations. In SDREAM, tasks are classified into two categories: periodic and priority. The periodic task has the highest priority level and is responsible for capturing sensor signals or actuating control signals; the priority task has various priority levels and is suitable for time-constrained applications. The two-level task scheduling policy scheme, named "priority-based pre-emptive scheduling", is used for electing a task. SDREAM is a tuple-based message-driven system. The "tuple" is the foundation of task communication and synchronization. A shared data memory, named "tuple space", consists of a set of tuples each having a unique ID: key. All task and tuple members are static pre-allocation by the user at compile time. The interrupt handling mechanism of SDREAM is small and efficient, and has very short and determinate latencies for external events. The SDREAM has two fundamental features: it is both small and efficient. The minimal SDREAM version uses only 5 Kbytes of memory. The optional low-power operation mode enables an application of SDREAM, e.g. a 500Hz four-channel data sampling and real-time wireless connection, to run more than 120h on a wireless sensor device integrating the MSP430F149 microcontroller with a battery of 700mAH and a supply voltage of 3V. The evaluation results of the execution times of system primitives and system functions, task switch latencies and interrupt latencies demonstrate that the SDREAM architecture supports determinate and predictable system behavior, and the maximum response time for external events is under all system loads circumstantial. The SDREAM architecture integrates the advantages of both conventional RTOSes and the TinyOS operating system. Compared to the popular RTOSes, SDREAM has minimum resource consumption (memory and power) and minimum response time for interrupt events. It is thus ideally suited to migration into tiny-resource embedded devices, typically a wireless sensor network node. Even when compared to TinyOS, SDREAM is a true real-time multi-task system and has better scalability and flexibility for different hardware platforms and various applications requirements. SDREAM can run on platforms as diverse as tiny-resource devices to complex distributed systems and can support applications ranging from simple single tasks to real-time multi-tasks.

μRET: Minimal Real-time and Embedded TCP/IP implementation

Over the last few years, the interest for connecting embedded devices to wireless networks has steadily increased. Computers are becoming more and more seamlessly integrated with everyday equipments and prices are dropping. At the same time new wireless networking technologies, such as Bluetooth and IEEE 802.11 b WLAN, are emerging and maturing. Small devices such as sensors can be connected to an existing network infrastructure for example the global Internet, and be monitored from anywhere. With the success of Internet, the TCP/IP protocol stack has become a global standard of network communication. The widespread adoption of the TCP/IP stack in software applications has led to it being implemented in wireless or portable devices that can connect directly to an intranet or even the Internet. While originally developed for low speed networks such as ARPANET, traditional TCP/IP implementations run over a large spectrum of link technologies with vastly different characteristics in terms of bandwidth and bit error rate. Traditional technologies have often paid more attention to providing a solution for the flexibility of network communication in different network environments and traditional implementations, normally Berkeley stacks, are the basis of most of the commercial TCP/IP stacks for embedded systems. However, because real-time and embedded systems face many issues that are unique, a straight port of the traditional stack is not the best solution for the particular needs of an embedded and real-time system. Moreover, traditional TCP/IP implementations consume both CPU and memory resources that are scarce on such embedded devices. To run the full TCP/IP stack a few hundred kilobytes of code and data are necessary. Thus, in practical terms, the full TCP/IP stacks are not adapted to embedded systems because the on-chip memory of most microcontrollers devices is smaller than 100KB. Since embedded devices are often required to be physically small and inexpensive, there is thus a need to provide a minimal implementation of the TCP/IP stack that will be capable of matching the limited resources available. Despite the fact that there are numerous TCP/IP implementations for embedded systems, many of the existing implementations lack research into the simplification mechanisms of the TCP/IP stack. The present inventors have thus conceived and developed a minimal version of a TCP/IP stack, named μRET (Minimal Real-time and Embedded TCP/IP implementation). The μRET according to the present invention was initially implemented for an intelligent wireless ECG sensor that also forms one of the objects of the present invention.

In accordance with a further object of the invention, the design and implementation of μRET are also presented herein, as are the data structures, algorithms and mechanisms, and the general control flows used. The performance of μRET was also evaluated.

The TCP/IP (Transmission Control Protocol/Internet Protocol) protocol stack is probably the most well known communication protocol for the Internet and networks worldwide. Its simplicity and power has lead to its becoming the de facto standard for computer communications in today's networked world. The main design goal of TCP/IP was to build an interconnection of networks, referred to as an internet, or internetwork, that provided universal communication services over heterogeneous physical networks. TCP/IP provides communication services that run between the programming interface of a physical network and user applications. It contains a common interface for these applications, independent of the underlying physical network. The architecture of the physical network is therefore hidden from the user and from the developer of the application. The applications only need code to the standardized communication abstraction to be able to function under any type of physical network and operating platform.

TCP/IP is modeled in layers. By dividing the communication software into layers, the protocol stack allows for division of labor, ease of implementation and code testing, and the ability to develop alternative layer implementations. Layers communicate with those above and below via concise interfaces. In this view, a layer provides a service for the layer directly above it and makes use of services provided by the layer directly below it. However, the layering of TCP/IP is not kept as strict as in other protocol stacks, and it is possible for, e.g. application layer functions to access the internet layer directly. The functionality provided by each layer is :

The network interface layer, also called the data-link layer, is the interface to the actual network hardware. This interface may or may not provide reliable delivery, and may be packet or stream oriented. It takes care of low-level addressing and address mapping.

The main protocols of the network interface layer are PPP, and Ethernet, etc;

The internet layer, also called the network layer, provides the "virtual network" image of an internet. It provides abstract addressing and routing of datagram between different physical networks. The Internet Protocol (IP) is a connectionless protocol that doesn't provide reliability, flow control and error recovery.

The transport layer provides the end-to-end data transfer by delivering data from an application to its remote peer. The Transmission Control Protocol (TCP) provides connection-oriented reliable stream delivery, data suppression, congestion control and flow control. The User Datagram Protocol (UDP) provides connectionless, unreliable, best-effort datagram delivery service.

The application layer is provided by the programs that use TCP/IP for communication.

The interface between the application and transport layers is defined by port numbers and sockets.

The de facto standard for TCP/IP implementations is the one from the CSRG (Computer Systems Research Group) at the University of California at Berkeley. Historically this has been distributed with the 4.x BSD system (Berkeley Software Distribution), and with the "BSD Networking Releases." This source code has been the starting point for many other implementations. Many vendors or free implementations, such as SunOS 4.x, SVR4, AIX 3.2 and Linux were originally developed from the Berkeley sources. These implementations have much in common, often including the same bugs. Since the first widely available release was published in 1983, the BSD TCP/IP implementation has evolved and many versions have been released. Many important TCP/IP features have been incorporated into these new releases. The 4.3BSD release (1986) improved TCP performance, the 4.4BSD Tahoe release (1988) incorporated RTT estimation, congestion control mechanisms and a fast retransmit algorithm, the 4.4 BSD Reno release (1990) then implemented a fast recovery algorithm as well as many other performance related optimizations, such as TCP header prediction, SLIP head compression and routing table change technologies. The last distributed release is 4.4 BSD (1993), which incorporated multicasting and long fat pipe modifications technologies.

As part of the open source movement, the Linux operating system is freely available to everyone and becoming more and more popular. Thousands of programmers are constantly working on the code to implement new features, improve existing ones, and fix bugs and inefficiencies in the code. Detailed sources are obtained for learning more about Linux, from the source code itself to books to "HOW-TOs" and message boards maintained on many different subjects. The kernel of network codes in a Linux operating system is a TCP/IP implementation which originates from the BSD releases. The well documented implementation and the free design mode, study and modification of the TCP/IP implementation under Linux has made this implementation one of the most frequently examined when setting out to develop a new TCP/IP implementation.

The networking function of embedded systems brings has greatly enchanced development of embedded applications and the maturity and progress in networking and VLSI technologies have also contributed to the network ability of embedded systems. However, embedded systems still require small TCP/IP implementations to meet with their constrained hardware resources. Currently, a lot of small TCP/IP implementations have been developed for commercial purposes or in various studies relating to microprocessors and microcontrollers. Some of these implementations are directed to particular hardware materials and system kernels and provide certain network functions, whereas others provide a complete protocols implementation for the common platforms. A. Dunkles implements two small generic and portable TCP/IP implementations, μIP (micro IP) and lwIP (lightweight IP). The lwIP is a full-scale, but simplified TCP/IP implementation that includes IP, ICMP, UDP and TCP. It is modular enough to be easily extended with additional protocols. The μIP is designed to contain only the absolute minimal set of features needed for a TCP/IP stack. The source code is available for both lwIP and μIP. The two implementations have been ported to various 8 bit and 16 bit platforms such as the AVR, H8S/300, 8051, Z80, ARM, M16c, and the x86 CPUs. InterNiche Technologies has designed two commercial TCP/IP implementations for an embedded system: NicheStack and NicheLite. The NicheStack is a small, portable, and RFC compliant implementation of the TCP/IP protocol suite. NicheStack has a small memory requirement. A full featured TCP/IP stack adding the sockets API brings the total ROM and RAM requirements to 51.5 KB on an ARM 32 bit processor. NicheLite is a full featured subset of NicheStack TCP/IP, and was designed for small memory targets and requires only 12 KB of ROM. There are also numerous TCP/IP implementations for embedded systems. Other known implementations are the embedded web servers of Atmel Corporation and A. Dannenberg, the TCP/IP stack of Kadak KwikNET, and the implementations of NexGen, etc.

TCP/IP breaks the data down into a series of packets or frames. Buffers are created to hold the data frames. Because of the inherent asynchronous nature of a network, buffers must be allocated and de-allocated dynamically. They can be deployed from a pre-allocated series of buffer pools or be allocated from the global memory pool when needed. Buffers have variable lifetimes depending on many numbers of factors, such as network throughput, whether the included frame is part of a stream transmission, or whether the packet is a datagram. An understanding of buffer management is therefore fundamental to an understanding of the protocol implementation.

The basic buffering mechanism used in Berkeley-based TCP/IP is called mbufs (memory buffers). Mbufs can be expanded to allow for variable length frames used often in IP-based communications. Small amounts of data can be placed directly in the data area of the mbuf. Larger amounts of data require the mbufs to be extended with clusters. A cluster is a data structure that holds heterogeneous data for extending the data-carrying capability of an individual mbuf. Mbufs can be chained to allow easy implementation of packet queues. The basic mbuf contains some header information with a data area which can be extended with a cluster if the frame can't fit into the data area. Mbufs are also convenient places to put control information or other temporary data used within the protocols. The mbuf supports four data types defined in the m_flags field in the mbuf structure :

0 flags : Mbuf contains only data which can be up to 108 bytes.
M_PKTHDR : Mbuf contains a packet header and up to 100 bytes of data.
M_EXT : Mbuf is extended with a cluster allowing up to 2,048 bytes of data.
M_PKTHDR | M_EXT : Mbuf contains the packet header and a pointer to a cluster, allowing it to contain up to 2,048 bytes of data.

In the Linux kernel, the key to maintaining the strict layering of protocols is the common packet structure, named sk_buff (socket buffer).The primary goal of the sk_buff routines is to provide a consistent and efficient buffer handling method for all of the network layers, and by being consistent, to make it possible to provide higher level sk_buff and socket handling faculties to all the protocols. A sk_buff is a control structure with a block of memory attached. There are two primary sets of functions provided in the sk_buff library:

routines to manipulate doubly linked lists of sk_buffs;
functions for controlling the attached memory.

The buffers are held on linked lists optimized for the common network operations of append to end and remove from start. As so much of the networking functionality occurs during interrupts these routines are written to be atomic. The list operations are used to manage groups of packets as they arrive from the network, and as they are sent to the physical interfaces. The memory manipulation routines are used for handling the contents of packets in a standardized and efficient manner.

The Application Program Interface (API) defines the way the application program interacts with the TCP/IP stack. Two popular APIs for applications using the TCP/IP protocols are called sockets and TLI (Transport Layer Interface). The former is sometimes called "Berkeley sockets" or BSD sockets, indicating where it was originally developed. The latter, originally developed by AT&T, is sometimes called XTI (X/Open Transport Interface). The BSD socket is the most popular and commonly used API for TCP/IP which is implemented in most Unix systems and has heavily influenced the Microsoft Windows WinSock API. It abstracts the network communication so that sending or receiving data from the network is no different from writing or reading from an ordinary file. From the application's point of view, for example, a TCP connection is just a continuous stream of bytes rather than segmented. Even though the BSD socket API is not formally defined as a standard API, the success of BSD has resulted in a large number of applications written for the BSD socket API.

The BSD TCP/IP implementation is a complex incorporation of multi-layer network protocols, which integrates various network standards and network technologies. This feature of BSD stack makes it flexible for the network communication between different network environments. However, this complicated implementation is resource hungry, which is obviously unacceptable for embedded system. The key issue of small implementations is therefore to reduce the bloat in the BSD stack and to behave in accordance with the actual circumstances of application. Although numerous small TCP/IP implementations have various simplified models, there are still certain simplification rules that can be followed. These rules are based on the several assumptions about the communication environments.

Firstly, the most common assumption is that the remote peer of embedded system, such as PC, runs a full-scale and standards compliant of TCP/IP implementation. By relying on the standards compliance implementation of the remote host, even an extremely simplified and standards non-compliant TCP/IP implementation will enable embedded system communicate with remote host. The usual simplifications are to tailor the TCP/IP stack and to remove certain communication mechanisms. One of the notable implementations is the iPic web server, which implements a web server on a chip PIC 12C509A with the code size of 256 bytes (12 bit). The files for the web server are stored on an EEPROM chip. In such an implementation, UDP is not necessary for a web server and the retransmission mechanism can not be made by the TCP module in the embedded system because nothing is known about the active connection. Another example is the Atmel TCP/IP implementation which removes TCP's congestion control mechanism. This implementation can work well when connected within a single network connection at a time. By removing certain protocol and several mechanisms, the complexity of an implementation is reduced, and the memory requirement is thus decreased. Secondly, another common simplification is to support embedded systems that handle one network connection at a time. This is a sensible simplification for many applications which dedicate to one special network transaction. This assumption will also greatly decrease the usage of RAM. Texas Instrument's MSP430 TCP/IP stack provides a TCP connection each time for a web server. This assumption has proven to be effective for an embedded system with scarce resources. The third common simplification is to pre-allocate frame buffers statically rather than to allocate them dynamically from the global heap at run time (via malloc). The header length of protocols, including IP, UDP and even TCP, is fixed if the implementation removes the options of protocols' headers. The data length of a frame is predictable, which is no more than the MTU (Maximum Transfer Unit) or MRU (Maximum Receive Unit). This method is useful to simplify the memory management of the implementation and enhance its effectiveness, particularly for the network transaction of constant data length. The $\mu$IP implementation of A. Dunkles gives an example of statically configuring a frame buffer at compile time, in which the number of the TCP listening port, the TCP connection and ARP table entries are pre-configured. In a fourth modification, the IP fragment reassembly module is optional and can be removed for many applications. TCP/IP implementations that are able to correctly reassemble fragmented IP packets are usually too large in terms of program memory and RAM requirements. The fragment and reassembly module is also the most complex part of the buffering and memory management mechanism. If particular applications can guarantee that no fragmented IP packets will occur in embedded systems, the reassembly function can be removed. Moreover, if the length of transmitted data is constrained to be no more than the MTU, the fragment function can also be removed. Finally, as the cost of data copying is expensive in a real-time embedded system, one simplification is to copy data as few times as possible and thus minimize the overhead of copying data. The data within each frame can be maintained in the same buffer so it doesn't need to be copied and re-copied by the CPU at each stage of the protocol. The networking chip's DMA places the packets directly in the managed buffer pool where the packet is passed up through the stack by manipulating pointers and not by copying data. Also, some vendors have extended the MBUF mechanism of BSD to allow data sharing between kernel space and user space where STREAMS protocols are also present in the system. For example, the NicheStack and NicheLite implementations are declared by InterNiche Technologies to support ZERO data copy for ultra fast performance.

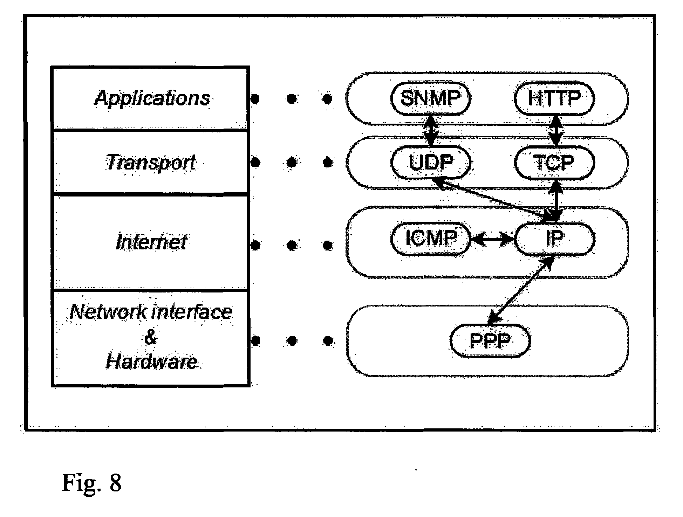

TCP/IP implementations are designed and modularized in a layered fashion as a hierarchy architecture described in Figure 8, where each protocol layer solves a separate part of the network communication problem and each protocol is separately encapsulated as a unique communication module. This modularization approach can simplify the TCP/IP implementation in which it one can add or remove protocol modules. Since each protocol is implemented as a module, every protocol layer module is thus composed of some functions and can provide uniform entry points for other modules, applications, and network interfaces. Traditional TCP/IP implementations keep a strict division between the protocol layers. Implementing the protocols in a strictly layered way can bring additional communication overhead between the protocol layers. This overhead degrades the overall performance of TCP/IP implementation, especially for the low-resource embedded devices. Moreover, in most implementations, the application layer and the lower protocol layers are strictly divided. The low protocol layers are implemented as a part of the operating system with entry points for communication with the application layer process. From the application program's point of view, there is no difference between network communication and inter-process communication. It implies that the application program is unaware of buffer mechanisms used by the lower layers and the memory spaces between application and system kernel are completely separated. Hence, when an application sends data, this data only has to be copied once from the application space into the system kernel space before being processed by network code. To overcome the problems caused by the strictly layered approach, most embedded TCP/IP implementations adopt relaxed layered schemes for the communication between the applications and the lower layer protocols. There is no strict protection barrier between the kernel and the application tasks. Therefore, an application task can directly read and write to the buffers of kernel space and share memory with networking code. By accessing shared memory, applications can more efficiently reuse buffers, and thus save the overhead of data copying. Moreover, some TCP/IP implementations violate the separate rule of protocol module and provide "wickets" for protocol modules. In this situation, a protocol module can directly extract information from other modules by not only the entry points but also the "wickets". By adopting the "wickets", the implementation can reduce function calls and more efficiently reuse buffers.

One object of the present invention is therefore a $\mu$RET implementation that can provide network function for a intelligent wireless ECG sensor, also an object of the invention. This particular application has a certain number of network connections, i.e. one TCP connection and one UDP connection at a time, and has a unique network medium, i.e. a wired or wireless serial link. The data sizes of sent and received IP packets are determined to be no more than the MTU and MRU, and the remote peer is a standard machine which runs a full-scale and standards compliant TCP/IP implementation. These application conditions distinctly satisfy with the simplification assumptions of the TCP/IP stacks. The layered protocol design idea is adopted for the design of the $\mu$RET implementation. Each protocol is implemented as a single module, with a few functions acting as entry point of each protocol. In order to improve performance in terms of processing speed and memory usage, the $\mu$RET implementation adopts a relaxed layered scheme for communication between the application and the lower layer protocols, and provides "wickets" for data exchanges among the lower layer protocols modules. The $\mu$RET implementation consists of several protocol modules - PPP, IP, ICMP, UDP, and TCP - which are illustrated in Figure 8.

The buffering and messaging subsystems are an important component of protocol implementation, which directly influence the efficiency of network communication. The key point of the buffering subsystem is the definition of packet structure. The design of the packet buffer adheres to two criteria: firstly, the packet buffer should be accommodated to different protocol formats and very varying data sizes; secondly, the buffering subsystem should be able to simply and effectively reuse the buffer and avoid data copying. Due to the pre-configured application features of wireless device according to the invention, and in order to improve efficiency and reduce memory usage, a simple buffering mechanism was designed for the μRET. The mechanism supports only static pre-allocated memory. The packet buffers, sockets, network interface tables, and route tables are all allocated before run-time. Since there is no need for dynamically handling memory allocation and de-allocation, memory management is thus extremely simple. The messaging subsystem presents the message handling functions of the protocol modules. In some particular situations, certain network functions of the TCP/IP stack can be removed. For example, TCP congestion and flow control mechanism is not indispensable if the network link can guarantee a reliable connection; and IP fragment reassembly function is not necessary if the data size can be controlled by the application programs of the peer-pairs. As a protocol implementation for the present invention, μRET has a sensitive application environment that can satisfy various assumptions of simplification. In this way, all the unnecessary network functions are not implemented in μRET, thus μRET may be considered as having a very simple and effective message subsystem.

The μRET implementation according to the present invention provides a simple packet structure, named ippacket, to handle IP packet buffers (see Figure 3.3) [27].

```
            IPPACKET

STRUCTIPPACKET
{
struct IPHDR i ph; /*IP head*/
union{
struct UDPHDR udph; /*UDP head*/
struct ICMPHDR icmph; /*ICMP head*/
struct TCPHDR tcph; /*TCP head*/
}trah;
unsigned char * dat_ptr; /*Data pointer*/
short dat_len; /*data size*/
short ipp_len; /*ip packet size*/
unsigned char flag;
/*0000 /*relative socket index*/
0 /*unused*/
1 /*transport layer head flag.
1: used; 0: unused*/
0 /*ippbuf unit use flag.
1: used; 0: unused*/
1*/ /*packet type flag.
1: in; 0: out */
};
```

The ippacket is a rather simple structure and supports only static memory pre-allocation. In view of the fact that it is only intended to handle 2 or 3 simultaneous network connections and short message sizes in the μRET, there are four ippacket buffers pre-allocated for network communication. These four ippackets constitute an ippacket queue and the index is used to indicate a ippacket member. The ippacket structure consists of an IP header structure, a variable transport layer header, a data pointer, two length fields, and a flag field. The IP header (iph) has a fixed length of 20 bytes (8bits/byte), and the IP options are not implemented in μRET. The transport layer header structure (trah) encapsulates three protocol headers: TCP, UDP and ICMP. The TCP header (tcph) has a fixed length of 20 bytes. In μRET, the TCP options only exist in the TCP SYN segment, which are stored into the TCP data buffer, as described further. The UDP header (udph) has a fixed length of 8 bytes. The ICMP is an Internet layer protocol, but in terms of the encapsulation sequence, it acts more like a transport layer protocol. Depending on the message types, the ICMP header (icmph) can have varying lengths. μRET only provides one ICMP service ("Ping") which has a fixed length of 8 bytes. The dat_ptr pointer points to the associated data buffer of an IP packet. The data buffer exists in either user space or kernel space. The dat_len field contains the length of data buffer. The ipp_len field contains the total length of an IP packet, which is the sum of the dat_len, and the length of iph and trah. In the flag field, the first four bits contain an index value of relative socket of the IP packet. The sixth bit indicates whether or not there is a transport layer protocol in the IP packet. The seventh bit is used to mark if current IP packet buffer is used or not. The last bit of flag field describes the type of IP packet: '0' indicates input packet and '1' represents output packet.

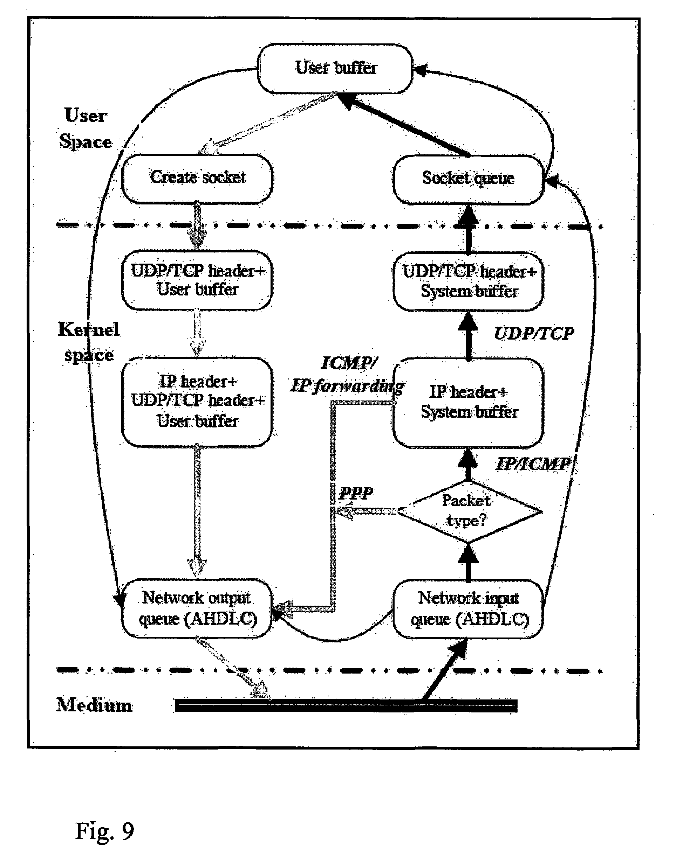

The relaxed layered scheme and the "wickets" technique are used in μRET of the present invention to avoid data copying and reuse shared memory. The message traffic flows of μRET describing how data is copied and memory usage are shown in Figure 9. There is only one data copying operation for outgoing messages in μRET. Data is copied

directly from the application program into the network output queue. While for incoming messages, in view of the different message types, there are one or two data copying operations. If an incoming message is a PPP negotiation frame or ICMP datagram or IP forwarding packet, it means that this packet is for a "handshake" or testing ("ping" service) or forwarding. Data is therefore copied directly into the network output queue. Whereas, if the incoming message is a UDP datagram or TCP segment, data is first copied from the network input queue into the socket queue to wake up a suspended application process, and then the invoked application process will copy data once more into its application buffer for subsequent processing. Typical network applications included in µRET comprise a web server (TCP) and a SNMP agent (UDP).

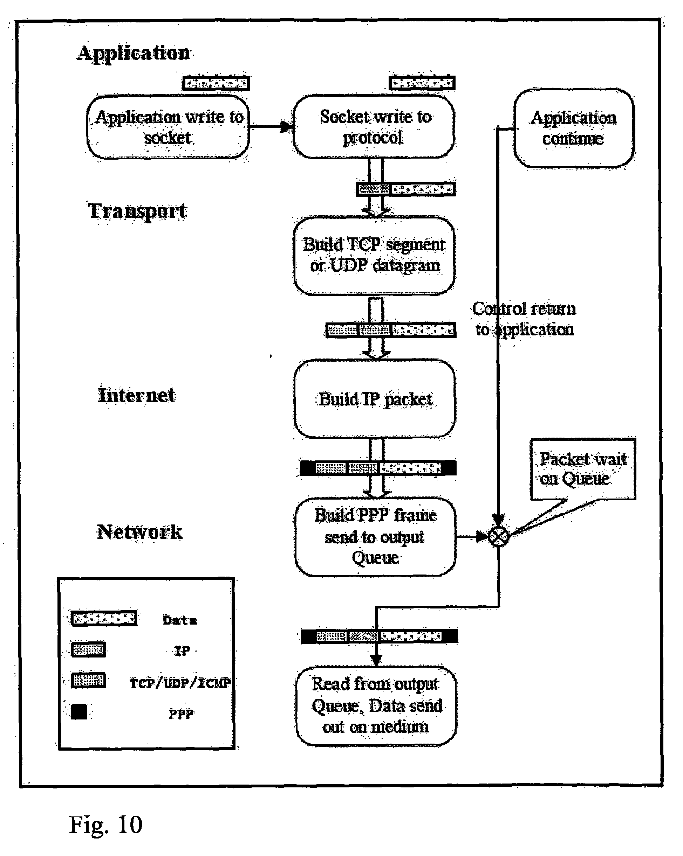

An outgoing message flow begins with an application process to write data to a socket. A socket is allocated from the pre-allocated socket queue for a network connection. The socket output function verifies the status of the socket, examines its protocol type, and sends the data to the transport layer routine (such as TCP or UDP). Subsequently, this transport protocol allocates an ippacket buffer from the pre-allocated ippacket queue for the outgoing packet, records the address pointer and the size of the application data, and fills in its header information (such as port number, data length and checksum) before passing the ippacket to the internet layer (usually IP). The IP output functions fill in more of the buffer with its own protocol headers (such as the IP address, lengths, and checksum). It may also fragment the packet if required. Next IP route function searches a matching route and network interface. Then the IP layer passes the packet to the related network interface layer. The network layer interface protocol, PPP in µRET, encapsulates IP packet to PPP frame with the AHDLC format and copies the frame onto the network output queue. At this time, the application process will return and the device driver of the physical medium undertakes the subsequent communication job, which includes reading data from the network output queue onto the physical device or writing data from the medium onto the network input queue. The send message modules are shown in Figure 10.

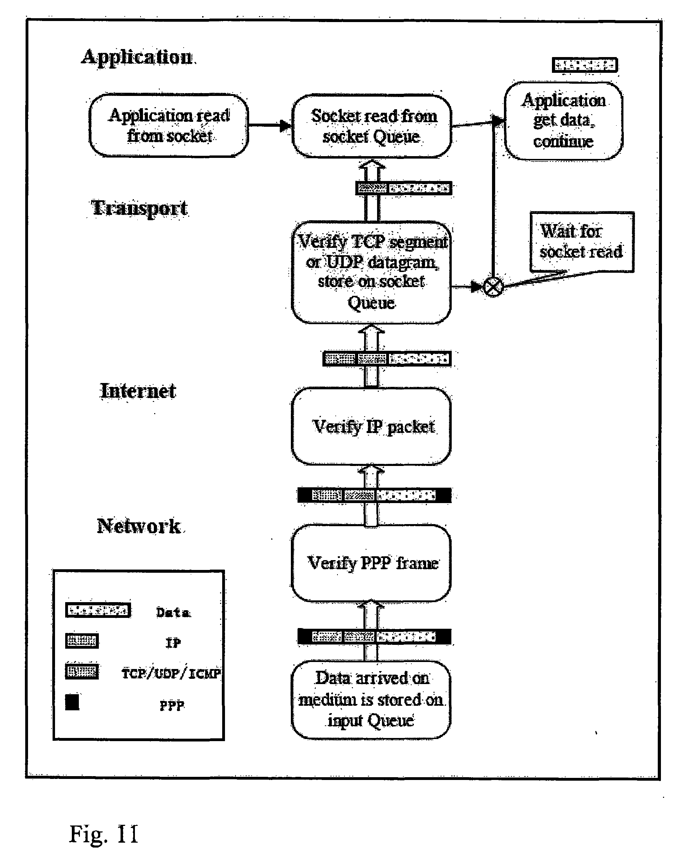

An incoming message flow begins when a message is ready in the network input queue. The PPP module verifies and extracts a message from the received frame, and identifies the message type. If the received message is a PPP negotiation frame, i.e. LCP and IPCP, the PPP negotiation automaton is called to establish, configure or terminate a PPP connection. Otherwise, if the received message is an IP packet, a free ippacket member is allocated from the ippacket queue to store this IP packet, and then the PPP protocol passes it to the IP layer. The IP layer examines the packet for errors and routes it; the packet will be sent up to the transport layer (such as TCP or UDP), or to the ICMP protocol module for the "Ping" service, or (down) to network interface layer for IP forwarding packets. The transport layer again checks for errors, looks up a socket pair associated with the port specified in the packet, and stores the packet in the socket queue. Once the packet is in the socket queue, the kernel will wake up the corresponding application process. As a consequence, the application process may make or return from a read system call that copies data from the socket queue into its own buffer. The message receiving modules are shown in Figure 11.

The purpose of the network interface layer in the TCP/IP protocol stack is to send and receive IP datagrams for the IP module. TCP/IP supports different network interface layer protocols, depending on the type of network access medium being used (physical layer). A common network interface structure has been defined for different protocols adapted to physical devices in µRET. The IP module does not concern the practical network interface protocol or the physical device. The transmission and reception of IP datagrams are implemented by the output and input functions of the network interfaces. These input and output functions have uniform encapsulation formats for all network interfaces. The Point-to-Point Protocol (PPP) is a network layer protocol used to control the delivery of IP datagrams between two hosts interconnected by a point-to-point link. It is described in IETF document (Internet Engineering Task Force) RFC 1661 and RFC 1662 (Request For Comments). In embedded systems, the point-to-point line, also named serial line, is the most widely used to connect an embedded device with remote hosts. The wireless serial connections, for example, 802.11 x and Bluetooth, and the wired serial connections, for exampel, classical modem or ADSL, can be implemented by PPP. The purpose of designing PPP into µRET was to provide an ability of point-to-point link connection for embedded systems. Since a remote machine that acts as a PPP server runs a full-scale and standards compliant of PPP protocol, a simplified and standards incompliant PPP protocol will be able to run in the embedded device.

In µRET, physical medium are presented by a network interface structure similar to that of BSD, shown below. There are two network interfaces supported by µRET: a serial link interface and a loopback interface. Since there is no Ethernet device in the systems according to the present invention, the most common Ethernet network layer protocol is not implemented in µRET.

## New_ndevice

```
struct new_ndevice
{
unsigned char i n dex;
unsigned char n ame[2];
struct ip_addr if_addr;
struct ip_addr if_broadaddr;
struct ip_addr if_mask;
int (*if_output)(struct ppp_state *state,int ipp);
void (*if_input)(unsigned char *buf, int len);
};
```

Two network interface structure buffers are pre-allocated in SRAM (static RAM) to store the interface information. The index field is used to indicate the position of the interface in a network interface array. Each network interface has an abbreviation name to identify the type of physical access medium, which is stored in the name field, e.g. bl(Bluetooth), wf(IEEE 802.11 b), sl (serial line), md(modem) and lb (loopback). The three IP addresses ip_addr, ip_broadaddr and ip_mask are used by the IP layer when sending and receiving packets. Each network interface must have a unique IP address as the identified flag. The IP address of the loopback interface is always "127.0.0.1". The IP address of the serial link interface is allocated automatically by the remote PPP server or set manually by the user. The if_output and if_input pointers point to the output and input function addresses of network interface. A network interface is connected to a physical access medium through the if_output and if_input functions. When a data frame has been received, the function pointed by if_input is called. The two arguments are respectively the address pointer and size of receive data buffer. For the serial link interface, the input function unwraps the incoming PPP frame, and then calls the PPP input function which is used to handle the PPP frame. For the loopback interface, since it is a virtual interface, the input function of the IP layer is called directly. When an IP packet is to be transmitted, the output function of the network interface is called by the IP layer. The first argument to the output function, its state, is the pointer of the PPP state structure data. It contains the status information of the PPP connection and the address pointers information of network input/output queues. The second argument, ipp, is the index of this IP packet in the ippacket array. For the serial link interface, the output function encapsulates the IP packet into a PPP frame in the AHDLC format, and simultaneously copies it onto the network output queue. For the loopback interface, the first argument (state) is NULL, and its output function copies the IP packet directly onto the network input queue.

PPP has three main components:

A method for encapsulating datagrams over serial links
A Link Control Protocol (LCP) for establishing, configuring, and testing the data-link connection;
A family of Network Control Protocols (NCPs) for establishing and configuring different internet layer protocols.

In μRET, only IPCP (IP control protocol) is implemented to support IP layer interconnection. RFC 1661 specifies the encapsulation method and the link control protocol. RFC 1662 describes the use of HDLC-like framing for PPP encapsulated packets. RFC 1332 specifies the network control protocol for IP.

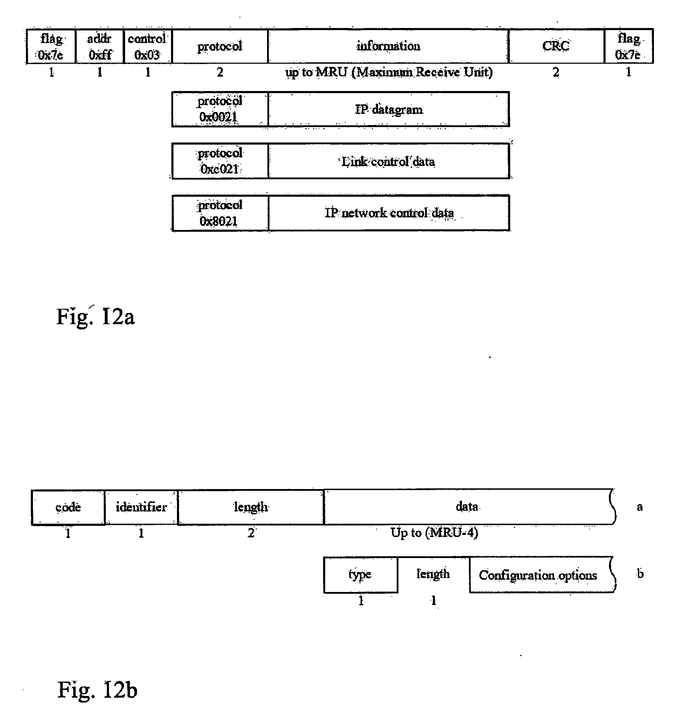

PPP encapsulation provides for multiplexing of different network-layer protocols simultaneously over the same link. The format of the PPP frames was chosen to look like the ISO HDLC standard, named AHDLC (Adaptive High-Level Data Link Control). Figure 12a shows the format of PPP frames. Each frame begins and ends with a flag byte whose value is 0x7e, which is used for frame synchronization. The address byte 0xff and control byte 0x03 are always constant. A protocol field is used to identify the type of PPP frame. Three types of PPP frames are supported in μRET: a value of 0x0021 means the information field is an IP datagram, a value of 0xc021 means the information field is link control data, and a value of 0x8021 is for network control data. The CRC field (or FCS: Frame Check Sequence) is used for cyclic redundancy check, to detect errors in the frame. A fast FCS implementation and computation method is provided in RFC 1662. In μRET, fast FCS calculation mode is supported, which improves performance by using additional 512 bytes of memory for a FCS lookup table.

The Link Control Protocol (LCP) is used to negotiate the configuration of the connection link. A LCP packet is encapsulated into the PPP information field, where the PPP Protocol field indicates type 0xc021. A summary of the Link Control Protocol packet format is shown in Figure 12b.

The Code field identifies the type of LCP packet. The LCP packet types defined in RFC 1661 include the following LCP codes: Configure-Request(1), Configure-Ack(2), Configure-Nak(3), Configure-Reject(4), Terminate-Request(5), Terminate-Ack(6), Code-Reject(7), Protocol-Reject(8), Echo-Request(9), Echo-Reply(10), and Discard-Request(1). LCP packets are classified into 3 classes:

Link configuration packet used to establish and configure a link (code 1 ~ 4);
Link Termination packets used to terminate a link (code 5, 6); and
Link Maintenance packets used to manage and debug a link (code 7 to 11).

The identifier field is used to match requests and replies of the LCP packet. The length field indicates the length of the LCP packet, including the Code, Identifier, Length and Data fields. The Data field has zero or more bytes. In most LCP packets, the data field is filled with LCP Configuration Options which allow negotiation of modifications to the default characteristics of a point-to-point link. The format of configuration options is shown in Figure 12b. For example, for a given LCP configuration option, e.g. MRU, the value of its type field is 1, which indicates the type of configuration options. The length field of MRU is 4, which indicates length of this configuration option including the Type, Length and Data fields. The data field of MRU is two bytes, and specifies the maximum number of bytes in the Information and Padding fields.

The IP Control Protocol (IPCP), described in RFC1332, defines the manner in which IP datagrams are delivered over the link established by PPP. An IPCP packet is encapsulated into the PPP information field, where the PPP Protocol field indicates type 0x8021. The IPCP is responsible for configuring, enabling, and disabling the IP protocol modules on both ends of the point-to-point link. The IPCP packet format and packet exchange mechanism are exactly the same as the Link Control Protocol. IPCP Configuration Options allow negotiation of desirable Internet Protocol parameters. IPCP uses the same Configuration Option format defined for LCP. The most well known IPCP Configuration Option is the IP-address which provides a way to negotiate the IP address to be used on the local end of the link.

In order to establish communications over a point-to-point link, each end of the serial link must first send LCP packets to configure and test the data link. After the link has been established, the peer may be optionally authenticated.Then, PPP sends NCP packets to choose and configure one or more network-layer protocols. Once the IP layer protocol has been configured, IP datagrams can be delivered over the point-to-point link. RFC 1661 proposes the standard for PPP finite-state automaton. Based upon the standard automaton, μRET implements a simplified finite-state automaton to negotiate, configure and manage a point-to-point link, which is defined by events, actions and state transitions.

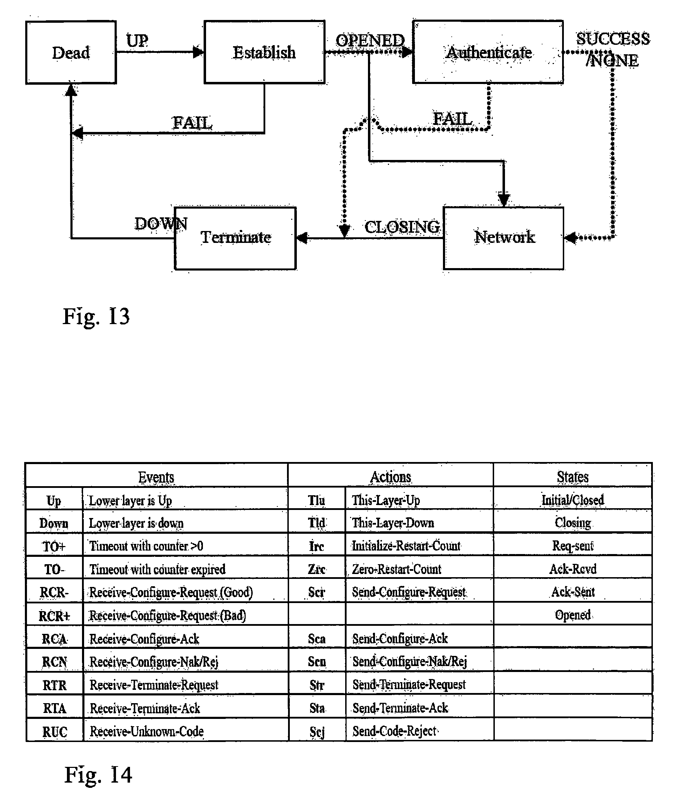

In the process of configuring, maintaining and terminating the point-to-point link, the PPP link goes through several distinct phases which are specified in the simplified phase diagram of Figure 13.

The point-to-point link begins and ends necessarily with a Dead phase. When an external UP event (such as carrier detection or user configuration) indicates that the physical-layer is ready to be used, PPP will proceed to the Establishment phase. In the Establish phase, LCP is used to establish a connection through an exchange of LCP Configuration options. The Authenticate phase which is in advance of the Network phase can provide authentication from the peer. The Authentication phase is optional. If link establishment is completed, LCP enters the Opened state and PPP proceeds to the Network phase directly. In the Network phase, IPCP is used to negotiate the network configurations. After IPCP has reached the Opened state, PPP will deliver IP datagrams. PPP can terminate the link at any time. In the Terminate phase, LCP is used to close the link through an exchange of Terminate packets. PPP then returns to the initial phase: Dead.

The finite-state automaton is defined by events, actions and state transitions. Events include reception of external commands such as Open and Close, expiration of the restart timer, and reception of packets from a peer. Actions include the starting of the restart timer and transmission of packets to the peer. States indicate the different connection status of LCP and IPCP. μRET contains a simplified PPP automaton, the events, actions and states of which is a subset of the standard automaton of RFC 1661. These events, actions and states are specified in Figure 14.

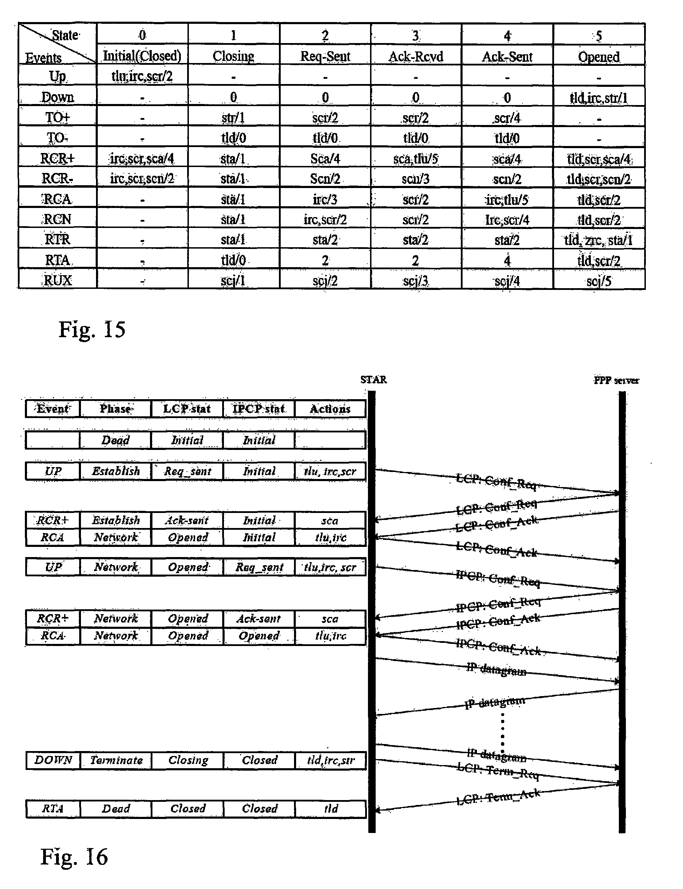

The state transition table of the PPP automaton of μRET is shown in Figure 15. States are indicated horizontally, and events are read vertically. State transitions and actions are represented in the form action/new-state. Multiple actions are separated by commas; multiple actions may be implemented in any convenient order. The dash ('-') indicates an illegal transition.

Figure 16 shows how to configure the LCP and IPCP options, and how to establish and terminate a PPP link in active mode. The active mode is the most common operation mode in the device according to the present invention, here given the designation "STAR" for convenience, in which a point-to-point link is opened and closed by a STAR user. The interrelation of phases, events, actions and state transitions are illustrated as the description of the state transition table. The initial phase of the PPP link is Dead, and the LCP and IPCP states are Initial. After LCP negotiation, the LCP connection has been established. The phase of the PPP link proceeds to Establish, and the LCP state is changed to Opened. Subsequently, exactly the same operations are carried out to establish the IPCP connection. The PPP phase then proceeds to Network, and IPCP enters Opened state. From now on, IP datagrams can be delivered in the established PPP link. In the active mode, a STAR user requests the termination of the PPP connection by sending a Term_Req LCP packet. The PPP phase proceeds to Terminate, the IPCP returns to its initial state and the LCP state is changed to Closing. If Term_Ack packet is acknowledged by the peer or a timeout event is happened, the PPP connection will be completely closed, and the whole PPP link returns to the initial point.

IP is the workhorse protocol of the TCP/IP protocol suite. All TCP, UDP and ICMP data will be transmitted as a formatted IP packet. The IP protocol hides the underlying physical network by creating a virtual network view. It provides an unreliable, best-effort and connectionless packet delivery service which means that the packets sent by IP may be lost, arrive out of order, or even be duplicated. The IP module assumes that higher layer protocols will address these anomalies. RFC 791 is the official specification of IP. IP provides three important definitions. First, the IP protocol defines the format of header encapsulation and data delivery mechanism. Second, IP performs the routing function, choosing a path over which data will be sent. Third, IP includes a set of rules that embody the idea of unreliable packet delivery. The μRET of the present invention implements only the most basic functionality of IP, which provides packet sending, receiving and forwarding ability.

For incoming IP packets, the input function of the IP layer, new_ip_input(), is called by the PPP input function. Here, the initial sanity check of the IP version field and the header length is done, as well as computing and checking the header checksum which uses standard techniques. Next, the function checks the destination address to see if there is a match the IP addresses of the network interfaces to determine if the packet is intended for the host. As mentioned in a previous section of this patent specification, there are only two network interfaces supported by μRET: serial link interface and loopback interface, which are stored in the pre-allocated network interface array. If the incoming packet is found to be destined for this host, the protocol field of the IP header is used to decide to which higher level protocol or transport layer, control should be passed. The μRET supports three types of transport layer: UDP, TCP, and ICMP, since in μRET, ICMP is implemented as a transport protocol.

An outgoing data generated by applications and wrapped by a transport layer protocol will be sent to physical media over the IP layer. The outgoing data is handled by the output function of IP layer, new_ip_output(). Here, all IP header fields are filled in and the IP header checksum is computed. Then, it uses the function new_ip_route() to find the appropriate network interface to transmit the packet on. When the outgoing network interface is found, the packet is passed to the output function of the network interface, which takes two arguments as described in the network interface structure. In μRET, the size of outgoing data is limited to be no more than MTU, which thus avoids the overhead of IP packet fragmented operation.

## NEW_ROUTE

```
struct new_route{
unsigned long ro_dst; /*destination host adress or net address*/
unsigned long ro_src; /*interface addr*/
unsigned long ro_mask; /*destination net and subnet mask*/
unsigned long ro_gate; /*gateway address*/
unsigned short ro_flag; /*route flags*/
int r o _ifdex; /*the index of interface device*/
#define NEW_RU 0x01 /*route can be used*/
#define NEW_RG 0x02 /*route to gateway, if no ,route to host*/
#define NEW_RH 0x04 /*ro_dest is a host address, if no, a net address*/
#define NEW_RS 0x08 /*route static*/
#define NEW_RC 0x10 /*route can be cloned*/
#define NEW_RL 0x20 /*route include datalink lay data*/
#define NEW_RR 0x40 /*route loopback*/
};
```

The route information is stored on a route table structure buffer shown above. A route table array that has five route table members is pre-allocated in μRET. In the route table, the ro_dst, ro_src, ro_mask and ro_gate fields describe respectively the destination host address, the network interface address (source host address), the destination address netmask and the gateway address. The ro_flag field indicates the characteristics of route table defined in table structure. The ro_ifdex field is the index pointer of the network interface. Each route table is associated with a unique network interface. The IP module can add, delete or modify a route table member. As the number of route table members is small, the route algorithm is thus fairly simple: if the masked destination address of IP packet is equal to the masked interface address of a route table member, a network interface is chosen; whereas if no match is found, a default network interface, i.e. point-to-point link, is used.

If an incoming packet is not for this host, it means that no network interface matches the destination address of the incoming packet. Hence this packet should be forwarded by new_ip_forward(). The IP header is changed, the TTL is decreased to indicate one more incidence of IP forwarding, and the IP header checksum is recomputed. Then, the new_ip_output() is called to transmit the forwarding packet to the remote peer via the default network interface.

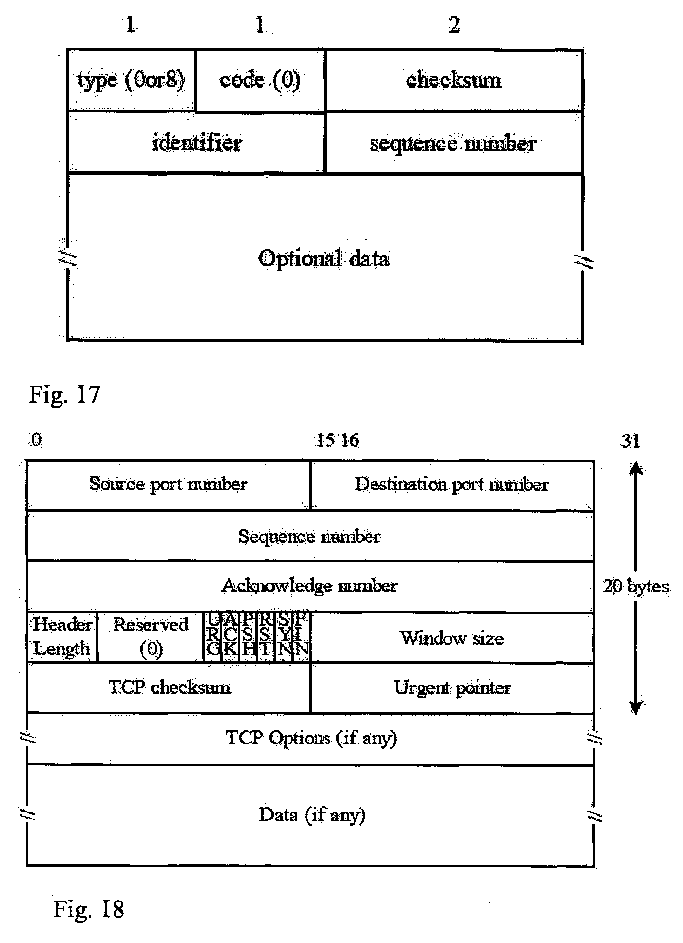

The ICMP protocol described in RFC 792 is used for reporting error conditions and for querying host parameters. The most frequent scenario for ICMP is to probe the remote host or network, if reachable, by using ping program. The ping program is the simplest of all TCP/IP applications which uses ICMP echo mechanism. It sends ICMP datagrams to a specified destination host and measures the RTT (Round Trip Time) to receive a response. The ICMP implementation in μRET is very simple in that it only supports the ICMP echo message, i.e. the ping program. Figure 17 shows the format of the ICMP message in the present invention for echo request and echo reply. The value of the type field is 8 for an echo request (ping request), and 0 for an echo reply (ping reply). The code field contains the error code for the datagram reported, and for an echo message, the value of this field is always 0. The checksum field contains the checksum for the ICMP message starting with the type field. The identifier field is set to be an identifier of the ping process, for the case where multiple instances of the ping program might be running at the same time on the same host. The sequence number starts at 0 and is incremented every time when a new echo request is sent. These two values are used to identify an ICMP echo datagram and they are returned in the echo reply. The data field for the ping program is normally the timestamp of this ping request in sender.

ICMP is an internet layer protocol, but in the present invention it acts more like a protocol of the transport layer. As the ICMP module of μRET according to the invention does not deliver ICMP error messages generated in IP, TCP or UDP, it means that the transport layer protocols have no need to call ICMP functions. An input function of ICMP, new_icmp_input(), is activated by when an ICMP echo request message is received from remote hosts. This function automatically generates an ICMP echo-reply message in response to the echo-request. Replies to echo messages are constructed by simply swapping the source and destination IP addresses of echo request message and rewriting the ICMP header with the Echo-Reply message type. The ICMP checksum is adjusted using the same method as described for the IP header checksum. Finally, the constructed echo reply message is passed into the IP layer and transmitted by new_ip_output().

UDP is a simple, datagram-oriented transport layer protocol: each output operation of a process produces exactly one UDP datagram, which causes one IP packet to be sent. UDP provides an unreliable datagram delivery service and the application function is responsible for the reliability of data exchange. RFC 768 is the official specification of UDP. The encapsulation of UDP is fairly simple. The UDP header is composed of the port numbers of the source host and the destination host, the length of the UDP header and the UDP data in bytes, and the UDP checksum. The basics for calculating the UDP checksum are similar to the one for the IP header checksum, but some differences are worthy of mention. First, the UDP checksum covers the UDP header and the UDP data, which is unlike the IP checksum that covers only the IP header. Second, the length of the UDP datagram can be an odd number of bytes, while the checksum algorithm adds 16-bit words. The solution is to append a pad byte of 0 to the end, if necessary, just for the checksum computation. Third, both UDP and TCP include a 12-bytes pseudo-header with the UDP datagram (or TCP segment) just for the checksum computation.

The input and output processing of UDP is straightforward in μRET. When applications generate outgoing data, the output function of UDP new_udp_sendmsg()is called. This function immediately calls another output function new_udp_output()(in order to have a uniform style with TCP, we define two UDP output functions). Here, an ippacket buffer is allocated to accommodate the UDP datagram. The UDP checksum is computed and UDP header fields are filled. UDP pseudo header can be stored into the IP header field of the ippacket structure buffer. If the application program does not specify the IP address or the port number of the sender, the default IP address and the port number will be designated to this packet and be attached to the socket-pair. Finally, the UDP datagram is turned over to new_ip_output () to transmit. When a UDP datagram arrives, the input function of UDP new_udp_input() is called. Here, the UDP checksum is checked and the datagram is de-multiplexed. The new_udp_lookup() is used to lookup an associated sock-pair for the received datagram in socket queue. When a socket-pair is found, the UDP data is copied to the related socket buffer. In next task scheduling, the UDP function new_udp_recvmsg() copies data from the socket buffer to the receiving buffer of the application. Consequently, the suspended application is returned and continues with the next operation. If no socket-pair is found, the UDP datagram is discarded silently.

TCP provides a connection-oriented, reliable, flow-controlled and ordered byte-stream service between the two end points of an application. TCP is the most complex protocol in the TCP/IP protocol stack. It uses the unreliable IP mechanism for communication and uses time with a retransmission mechanism to achieve reliability. The original specification for TCP is RFC 793. In view of the actual application circumstances of the system according to the invention, the TCP implementation has been greatly simplified in μRET. First, the TCP module in μRET acts as a TCP server, which responds to a TCP request from a TCP client and gives TCP acknowledgement. Since the remote peer runs a full-scale and standards compliant of TCP/IP implementation, only part of the functions needs to be implemented for TCP establishment and termination. Next, because there is only one TCP service i.e. a web server in a STAR system according to the present invention and only one TCP connection at a time in any current TCP application, some complex TCP communication mechanisms, such as retransmission, flow control, congestion control, and sliding window, can thus be simplified or removed.

The unit of transfer between two machines using TCP software is called a segment. Segments are exchanged to establish connections, to transfer data, to send acknowledgements, to advertise window sizes and to close connections. Each segment is divided into two parts, a TCP header followed by data. The TCP header carries the expected identification and control information. The way TCP achieves reliability is in the format of the TCP header. Figure 18 shows the TCP segment format used in the present invention.

The Fields, source port and destination port contain the TCP port numbers that identify application programs at the ends of the connection. The sequence number field identifies the sender's byte stream of data in the segment. The acknowledgement number specifies the number of bytes that the source expects to receive next. The header length field contains an integer value that specifies the length of the segment header in 32-bits. The header length covers the 20 bytes common TCP header and the varied TCP options. In the TCP implementation of the invention, only one TCP option, MSS (Maximum Segment Size) is defined for the TCP SYN segment. The TCP options are stored into a data buffer in μRET, so that the TCP header has a fixed-length which satisfies the transport layer header format of the ippacket structure. The 6 bits reserved field is reserved for future use and is filled with zero.

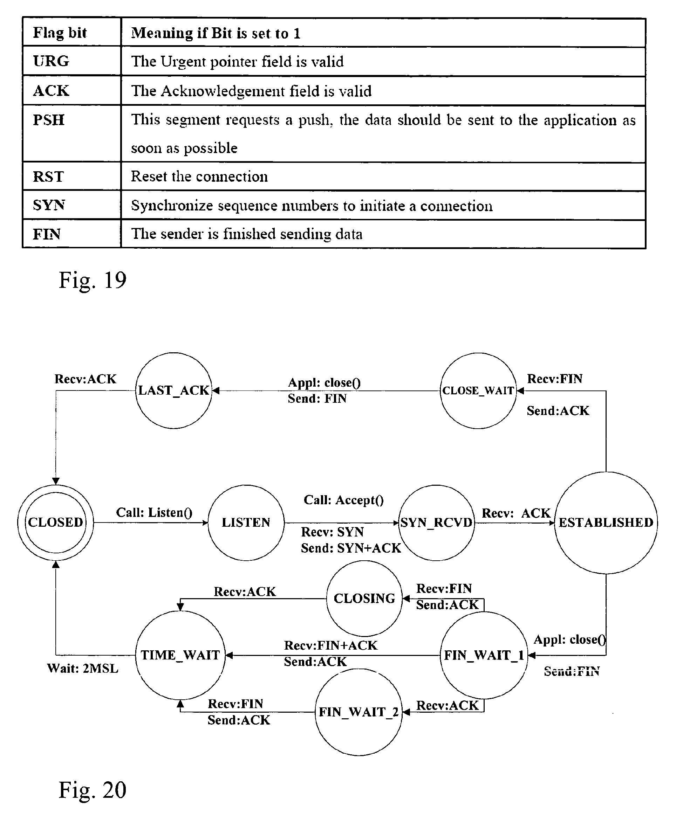

Six flag bits are used to determine the purpose and the contents of the TCP segment. These six bits tell how to interpret other fields in the header as illustrated in Figure 19. The TCP implementation of the present invention responds to the ACK, RST, SYN, FIN, and PSH segments. Since there is only one TCP application and one TCP connection at a time, the URG flag is meaningless for the identification of urgent data and the urgent pointer field is thus of no use. TCP software advertises how much data it is willing to accept every time it sends a segment by specifying its buffer size in the Window size field. The window size of the TCP segments in the present invention is fixed and equals the MSS minus the size of the general TCP header (MSS-20). The checksum covers the TCP segment: the TCP header and the TCP data. The TCP checksum is calculated similarly to the UDP checksum, using a pseudo-header as described above. The urgent pointer field is insignificant in our implement, which is ignored for the incoming segments and is filled with zero for the outgoing segments.

The lifetime of a TCP connection is divided into three phases: (1) opening the connection to create a full-duplex byte stream; (2) transferring data in one or both directions over this connection; and (3) closing the connection. A three-way handshake mechanism is utilized to facilitate the set up and closure of the TCP connection. Many of TCP's actions, in response to different types of segments arriving on a connection, can be summarized in a state transition diagram. The TCP implementation in μRET is a subset of the TCP standard, which implements the basic functions of a TCP server: passive opening TCP connection, transferring TCP segments, and active or passive closing TCP connection. Figure 20 shows the various state transitions of the TCP server according to the present invention. The CLOSED state is the begin and end point of a TCP connection. Before estimating a TCP connection, socket function new_socket() is called to create a socket, and then new_bind() is called to bind this socket with a specific port:

TCP server passive open: the application process calls socket function new_listen() to listen for connection requests from TCP clients, the connection state is changed to LISTEN. Then, socket function new_accept() is called to accept connection requests from TCP clients. If the opening request segment, with seq number of J, from a TCP client is received, a SYN segment, with a seq number of K and ack number of J+1 is sent back to the TCP client to actively set an active opening of the TCP client. The connection state changes to SYN_RCVD. When an ACK segment, with the ack number of K+1, is received, the TCP connection is established and the state is changed to ESTABLISHED. From now on, TCP data segments can be exchanged between TCP client and server.

TCP server passive close: when a FIN segment, with seq number of M, is received, it means that the client has begun to close the TCP connection. An ACK segment, with ack number of M+1, is sent to the TCP client. The connection state is changed to CLOSE_WAIT. Then, socket function new_close() is called to terminate the connection, which sends a FIN segment, with seq number of N, to activate the active closure of the TCP client. The connection state is changed to LAST_ACK. If last ACK segment, with ack of N+1, is received from the TCP client, the TCP connection is completely terminated and the connection state is returned to CLOSED.

TCP server active close: The application process calls the socket function new_close() to terminate a TCP connection. A FIN segment, with seq number of N, is thus sent to notify the client of a request to close the TCP connection. The TCP connection state is changed to FIN_WAIT_1 and the TCP client begins a passive closure. After one of the three following phases, the TCP server enters the TIME_WAIT state. Then, after waiting a time interval of 2MSL (2 times the Maximal Segment Lifetime), the TCP connection is completely terminated and the connection state is returned to CLOSED.

If a FIN segment is received first, with seq number of M, from the TCP client, the TCP server responds with an ACK segment, with seq number of M+1, and the TCP connection changes state to CLOSING. Then a received ACK segment, with ack number of N+1, will change the TCP connection state to TIME_WAIT.

If an ACK segment, with ack number of N+1, is received first, the TCP connection will change to FIN_WAIT_2. This is a half-closed state, in which the TCP server can still receive the data segments from the TCP client. When a FIN segment, with seq number of M, from a TCP client is received, the TCP server will send back an ACK segment (with ack number of M+1) and the TCP connection processes to TIME_WAIT.

If an ACK+FIN segment, with ack number of N+1 and seq number of M, is received, the TCP server responds with an ACK segment, with ack number of M+1, and the TCP connection directly proceeds to TIME_WAIT.

The function new_tcp_sendmsg() is called by the socket function to handle TCP data sending. Here, the data is divided into appropriate sized chunks, of a size less than MTU-28, and is attributed ordered sequence numbers. Next, in a control loop, new_tcp_output() is called to send each data chunk. In new_tcp_output(), an ippacket member is allocated to accommodate the TCP segment. The TCP header fields are filled and the checksum is computed. The SYN segment is handled differently. The SYN segment does not contain TCP data, but has TCP options, for example MSS. As the TCP header defined in the ippacket structure has a fixed length, TCP header options thus have to be stored in the data buffer, but are processed as the part of header. Like the process of the UDP output function, the IP address and port number of the sender must be attached to the socket-pair before calling the new_ip_output() function to transmit a TCP segment.

When a TCP segment arrives, the IP layer calls the new_tcp_input() function. The TCP checksum is checked and the segment is de-multiplexed. The new_tcp_lookup() function is used to lookup a socket-pair for the received segment in the socket queue. When a socket-pair is found, and depending on the segment type, different processes are performed. If the received segment is a SYN segment, FIN segment or an ACK segment for SYN and FIN requests, the passive mode of the TCP server for opening and closing a TCP connection is applied. If the received segment is a data segment, the data is copied to the corresponding socket buffer. The suspend function new_tcp_recvmsg() will be returned until the TCP connection is terminated, which means that all of the TCP segments of this TCP connection have been received. Then, the corresponding application program is invoked.

Most TCP implementations use the sliding window mechanism for sending data. In the STAR system of the present invention, there is only one TCP application and only one TCP connection at a time. In the web server application, the HTML page is stored in a pre-allocated data buffer. A TCP connection will be established when the user requests the HTML page through an Internet browser program, such as Internet Explorer or Netscape Navigator. The new_tcp_sendmsg() function is called to divide the HTML page buffer into several appropriated data chunks. There are thus several data segments that need to be transmitted for this service. These data segments are sent successively without waiting for an acknowledgement for each segment. The μRET implements the sliding window mechanism for TCP segments, and only needs a few lines of code to manage the data pointers of the TCP segments, avoiding increased complexity of the μRET implementation.

Since only the functions of a TCP server are implemented in μRET, the TCP implementation therein maintains three timers for each connection: a retransmission timer, a delayed timer and a 2MSL timer. The three timers are driven by a periodic system timer. Every time the periodic timer is invoked, these three TCP timers' values are decremented. If a timer's value reaches zero, the timer is considered to have expired and actions are invoked. The retransmission timer is set to the timeout each time the TCP sends data. If the segment is not acknowledged by the other end when the retransmission timer has expired, the TCP module retransmits the segment. The default retransmission timeout is set to 2.84s or 200 time-ticks for the Texas Instruments Digital Signal Processor TMS320C5410. Moreover, each time that the timer expires, its retransmission timeout is increased by 10 time-ticks. If the retransmission times arrive at the maximum threshold value, in this case 3 times, a FIN segment is sent to the other end, the TCP connection is terminated abruptly and the connection state is set to CLOSED. The delayed timer is set when the TCP server sends data segments which should have been acknowledged by the TCP client, and normally caused by the sliding window. The delayed timer has a longer timeout than, but its expired action is the same as, that of the retransmission timer. The default delayed timeout is set to 5.68s. The 2MSL (Maximum Segment Lifetime) timer is set when the the TCP connection state is TIME_WAIT. The TCP server has to wait an interval time of 2MSL in order to make sure that all data segments on the link will be received. When this timer has expired, the connection state is changed to CLOSED to terminate the connection. The default 2MSL timeout is set to 30s. The actual retransmission operation is fairly simple in μRET because the data buffer of the TCP application is pre-configured and always kept alive. Hence there are no additional buffer allocation/de-allocation operations for retransmission and no need to build a new segment. In fact, segment retransmission is similar to a normal segment transmission which further reduces complexity of the TCP implementation in μRET.

The Round-Trip time (RTT) is normally used to determine the retransmission timeout and to evaluate network communication capacity in most TCP/IP implementations. In μRET, the ping program (ICMP echo message) can be used to estimate RTT. In an exemplified STAR system of the present specification, the TCP application is extremely simple (single-user web server) and its physical network medium is a unique point-to-point link, the default timeouts of the three TCP timers and the network traffics are thus predictable and predefined during compile time, so that there is no need to estimate the retransmission timeout using RTT.

The purpose of TCP flow control mechanism is to allow communication between hosts with widely varying memory size. The flow control mechanism can be implemented by altering the window size field of the TCP header.

The value of this field indicates the available buffer space of the sender. In the present invention, μRET does not integrate a flow control mechanism and the value of window size field remains constant. The sizes of the network input/output buffer determine the maximum size of the TCP segment. Additionally, the current TCP application of the STAR system in accordance with the invention only has a few segment exchanges, and each time only one TCP connection is allowed. There is thus no advantage to be gained from implementing window size control. By limiting the numbers of simultaneous TCP segments, the congestion control mechanism changes the data rates of the sender and the receiver to meet network capacity and to avoid potential congestion. Since the TCP application of the STAR system according to the invention only supports one TCP connection at a time, there is in essence no possibility for a potential congestion situation.

The standard BSD socket API adopts "stop and wait" semantics. It requests a data copy between the application program buffer and the system protocol buffers. The reason for data copy is that the application program and the TCP/IP stack usually reside in different protection domains. In most cases, the application program is a user process and the TCP/IP stack is the part of the system kernel. The user buffer space and the kernel buffer space are usually strictly protected and can not be shared with each other in most systems. It is for this very reason that the BSD socket API provides a high-level abstraction layer to manipulate the data exchanges between applications and network protocols. Therefore, the standard BSD socket API is not suitable for the purposes of the present invention. However, the BSD socket API is a well-known API, and has become a de facto industry standard. Lots of applications have been developed based on BSD socket API. It was thus considered appropriate to implement an API in the present invention which is compatible with the BSD socket API. In most small embedded systems, system kernels are designed to support direct data exchanges between applications and kernel by avoiding extra data copy operations and reducing memory usage. This is also an important advantage of the real-time embedded system SDREAM described above and one of the objects of the present invention. This feature makes it possible to implement an API which supports direct data exchanges between applications and network protocols. A simplified variation of the BSD socket API is thus implemented in μRET. It adopts completely compliant interfaces and call modes with the BSD socket API, but can also directly support data read and write operations among different data spaces.

Each network connection must have a unique socket-pair. The socket structure of μRET is shown below.

## Socket structure

```
struct new_socket
{
int skfd;                          /* socket index in sock queue*/
int used;                          /* used flag. 0: unused; 1 used*/
struct new_skaddr srcskaddr;       /* source socket-address*/
struct new_skaddr dstskaddr;       /* destination socket-address*/
unsigned char * dbuf;      /* data buffer pointer*/
int d len;                         /* data length*/
int t ype;                         /* socket type: DGRAM,STREAM,RAW*/
int p r ot;                /* transport protocol: udp, tcp, raw*/
int dev_if;                /* device interface index*/
struct new_route * ro;     /* route table pointer*/
unsigned char state;               /* tcp socket state*/
};
```

This smart structure contains all the information of a network connection. The socket-address structure (new_skaddr) is composed of an IP address and a port number. The two socket-addresses of the source and the destination hosts, i.e. the srcskaddr and dstskaddr fields in socket structure, are combined as a socket-pair which is used to exclusively identify a network connection. The socket-pair is important to de-multiplex the incoming TCP segments or UDP datagrams for their corresponding applications. It is also useful to lookup a suitable route table and a network interface for outgoing packets. The values of dbuf and dlen fields are the address pointer and the size of the data buffer of the application, respectively. These two values are passed to an allocated ippacket member, which means that the protocol layers can directly manipulate the data buffer of applications and no data copying is needed here. There are three different types of socket interfaces which are identified in the type field: The stream socket interface (SOCK_STREAM), the datagram socket interface (SOCK_DGRAM) and the raw socket interface (SOCK_RAW). The protocol field is used to indicate the types of lower layer protocols: TCP for SOCK_STREAM, UDP for SOCK_DGRAM and ICMP for SOCK_RAW. The value of the dev_if field points to the index of the related network interface. The ro pointer points to the index of the related route table. The state field is only reserved for the SOCK_STREAM interface and the TCP protocol, which indicates the TCP connection state. The skfd field is the index of the socket in the socket queue. The used field then indicates the status of a socket. The skfd and used values are useful for designating a new socket or to search a related socket from socket queue.

The simplified variation of the BSD socket API is composed of seven basic socket calls, which allow application programs to communicate with any available transport layer protocol and underlying network service. In view of the functions of the socket calls, they can be classified into 3 classes: 1) socket allocation: new_socket(); 2) connection management: new_close(), new_bin(), new_listen(), new_accept(); 3) data delivery: new_sendto(), and new_recvfrom().

Socket system calls are frequently used for the connect-oriented TCP protocol and the connectionless UDP protocol. The TCP implementation of the present invention is designed to support applications of the TCP server, and thus the connection request call of a TCP client, i.e. new_connect(), is not implemented. Connectionless socket calls are simpler, in that the listen/accept/connect stages can be avoided.

In a application protocol layer, a SNMP agent and a minimum web server are implemented as the instances of UDP and TCP applications respectively. The SNMP agent and the web server run in the SDREAM system configured as two system priority tasks.

The Web (WorldWide Web, or WWW) is probably the most important network application of Internet. A web application is based on a typical client/server model and adopts HTTP (Hypertext Transfer Protocol) for web communications. The web client is normally an Internet browser program, such as Internet Explorer, Netscape Navigator, Opera, Konqueror, Mozilla, Mozilla Firefox, Lynx, etc. The web server listens to the well-known TCP port 80 and returns a HTML page to the client. HTTP is the application-level protocol which specifies the communication mechanism between web server and web client and runs over the TCP protocol. First, the client requests a web service. Through the TCP negotiation mechanism, the client establishes a TCP connection with the web server. The browser of the client sends a "GET" request automatically to and then reads the responses from the web server. The web server closes the TCP connection to indicate the end of the HTML page transfers. The most frequently used response data of web server is the HTML (Hypertext Markup Language) document which is described in RFC 1866. The minimum web server of the present invention implements only part of the functions of HTTP version 1.0. It responds only to "GET" requests from the client. The web data that includes the status information and the configuration parameters of the STAR system according to the invention are displayed in client's browser interface. The source code of the web server used in the implementation according to the present invention, in which the socket system calls of the TCP connection are presented, is shown hereunder for illustrative purposes.

## Web server implementation code

```
/***************************************************************
*
* Web Server
*
***************************************************************/
const unsigned char WebHead[] =  /*HTML page header*/
{
            "HTTP/1.0 200 OK\r\n"
            "Content-Type: text/html\r\n""\r\n"
};
const unsigned char WebBody[] =  /*HTML page body*/
{
            "<html><head><title>Hello, world!!</title></head>
            <body><center>Welcome, this is my first page!!</center></body>
            </html>\r\n""\r\n"
};
void HttpServer(void)
{
            int                                     err, requst_id, tcpdatlen, tcpcurnum;
            unsigned char *              tcpdatptr;
            struct new_sockaddr              my_addr, client_addr;

            while(!IS_PPP_OPEN());       /*Verify if the PPP connection link is opened*/
            while (1)
            {
                        if (tcpskfd == -1){
                        tcpskfd = new_socket(NEW_AF_INET,NEW_SOCK_STREAM ,0);
                        /*Allocate SOCK_STREAM socket*/
                        if (tcpskfd == -1) {
                                    ErrTyp = ERR_SO_SOCK;
                                    ErrMnm(); goto fini;
                        }
                        new_aton("10.0.0.2",&my_addr.sin_addr);
                        my_addr.sin_port = HTTP_PORT;
                        err=new_bind(tcpskfd, &my_addr);            /*Bind Well-known HTTP port*/
                        if (err == -1){
                                    ErrTyp = ERR_SO_BIND;
                                    ErrMnm(); goto fini;
                        }
                        }
                        while (1){
                        err= new_listen(tcpskfd, 1);       /*Listening*/
                        if (err == -1){
                                    ErrTyp = ERR_SO_LISTEN;
                                    ErrMnm(); goto fini;
                        }
                        err = new_accept(tcpskfd, &client_addr); /*Establish Connection*/
```

```
            if (err == -1){
                            ErrTyp = ERR_SO_ACCEPT;
                            ErrMnm(); goto fini;
            }
            err = new_recvfrom(tcpskfd, requst_id, 1, &client_addr);
             /*Wait client's 'GET' request*/
            if (err == -1){E rrTyp = ERR_SO_RECV;
                            ErrMnm(); goto fini;
            }
            if (request_id != HTTP_GET_REQUST){
                            ErrTyp = ERR_HTTP_TYPE;
                            ErrMnm(); goto reset;
            }
            tcpdatptr = (unsigned char *)&WebHead[0];
            tcpdatlen = sizeof(WebSite1);
            err = new_sendto(tcpskfd,tcpdatptr,tcpdatlen,&client_addr);
                            /*Send HTML page head*/
            if (err == -1){
                            ErrTyp = ERR_SO_SEND;
                            ErrMnm(); goto fini;
            }
            tcpcurnum = 0;
            while (tcpcurnum <= TCPNUM){
             /*Send HTML page body: fragment in application layer*/
                            if (tcpcurnum == TCPNUM){
                            tcpdatptr = (unsigned char *)&WebBody
                            [tcpcurnum*TCPMSS];
                            tcpdatlen = sizeof(WebSite2) -tcpcurnum*TCPMSS;
                            }
                            else{
                            tcpdatptr = (unsigned char *)&WebSite2
                            [tcpcurnum*TCPMSS];
                            tcpdatlen = TCPMSS;
                            }
            err = new_sendto(tcpskfd,tcpdatptr,tcpdatlen,&client_addr);
            if (err == -1){
                            ErrTyp = ERR_SO_SEND;
                            ErrMnm(); goto fini;
            }
            tcpcurnum++;
        }
reset:              new_close(tcpskfd);              /*Close the TCP connection*/
        }
fini:      new_tcp_init();                  /*Initialization TCP */
        }
    }
```

The μRET implementation of the present invention integrates network management functions for small embedded devices. Network management of a TCP/IP implementation consists of network management stations, known as managers, communicating with network elements, known as agents. Network embedded systems act as SNMP agents to respond to managers' requests and to report the embedded system status. TCP/IP network management generally has three components:

A Management Information Base (MIB) that specifies what variables the network elements maintain. RFC 1213 defines the second version of this, called MIB-II.

A Structure of Management Information (SMI), which is a set of common structures and an identification scheme used to reference the variables in the MIB, is specified in RFC 1155.

The Simple Network Management Protocol (SNMP) details the format of the SNMP messages and defines the exchange mechanism between the managers and the agents. RFC 1157 specifies the protocol of SNMP version 1 and RFC 1901 defines the SNMP version 2. SNMP messages are normally encapsulated in UDP datagrams to be transported over the network.

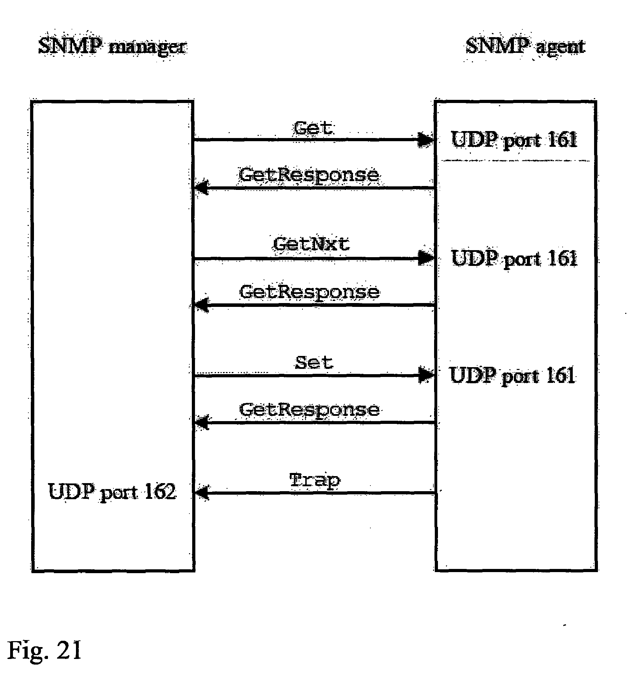

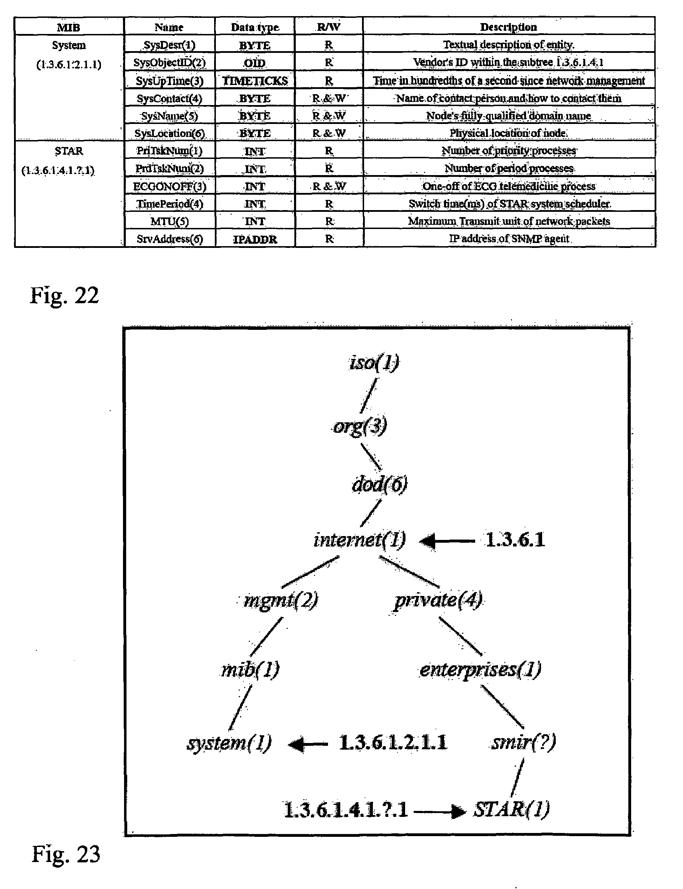

The SNMP agent used in the present invention implements the SNMP protocol of version 1 and version 2c. It supports five basic SNMP operators, i.e. GET, GETNEXT, SET, GETRESPONSE and TRAP, shown in Figure 21. The manager sends its three classes of requests to the agent (UDP port 161), while the agent sends traps to the manager (UDP port 162). The SNMP agent contains two MIB groups: a public system group and a private STAR group. The system group is a basic MIB-II group which provides the vendor's information, while the STAR group is a private MIB group which includes the status information of the STAR system according to the invention. The two MIB group variables and their SMI definitions are shown in Figure 22. Figure 23 shows the tree structure of the two MIB groups.

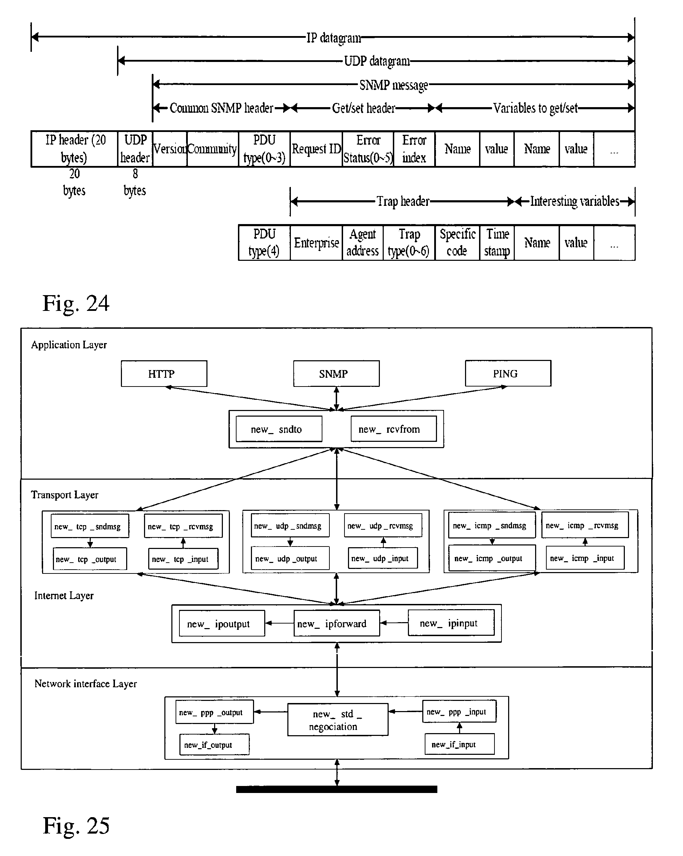

The SNMP manager gets the MIB variables of the SNMP agent by the GET or GETNEXT command and modifies these variables by the SET command. The SNMP agent also allows adding new MIB group or variables by the SET command from the SNMP manager. If something has happened to the agent that the manager might want to know about, the SNMP agent voluntarily sends a TRAP message to the manager. The STAR system error codes, communication KEY/community, and other configuration/management information can be set as MIB variables. By getting and setting the MIB variables, the SNMP manager has the ability to remotely configure and manage the STAR system. The formal specification of SNMP uses Abstract Syntax Notation 1 (ASN.1) and the actual encoding of the bits in the SNMP messages uses the corresponding Basic Encoding Rules (BER). The format of the SNMP message is shown in Figure 24. In this figure, the version equals the SNMP version number minus one. The community is a character string that is a clear-text password between the manager and agent. In this SNMP agent, we have defined three community strings: public, private, and smir, each having different read and write privileges. The PDU indicates the type of SNMP message. For the GET, GETNEXT, and SET operators, the request ID is set by the manager, and returned by the agent in the GETRESPONSE message, which is used to identify the SNMP message. The error status and error index is used to indicate which type of error and which variable is in error, when an error occurred. A list of variable names and values follows in the GET, GETNEXT, and SET requests. The SNMP agent actively sends a trap message to the manager if something has happened on the agent that the manager might want to know about. The PDU of trap message is always 4 (trap). The enterprise field is the vendor's object identifier. The agent address is the IP address of the network device. The specific code indicates the trap type which identifies the specific trap events. The Time stamp represents the number of hundredths of a second since the agent initialized. The source code of the SNMP agent used in a STAR device according to the present invention, in which the socket system calls of the UDP connection are presented, is given below as an example :

```
/*****************************************************************
 *
 * SNMP Agent
 *
 *****************************************************************/
void SnmpAgent(void)
{
int err, nflg=0;
struct new_sockaddr my_addr, client_addr;
while(!IS_PPP_OPEN()); /*Verify if the PPP connection link is opened*/
new_snmp_init(&rcvpdu); /*Init Snmp Agent*/
err=new_snmp_trap(&rcvpdu,SNMP_TRAP_COLDSTART, 0); /*trap--CodeStart*/
if (err <= 0) goto fini;
while (1)
{
if (snmpskfd == -1)
{
snmpskfd = new_socket(NEW_AF_INET,NEW_SOCK_DGRAM ,0); /*allocate new socket*/
if (snmpskfd == -1) {
ErrTyp = ERR_SO_SOCK;
ErrMnm(); goto fini;
}
new_aton("10.0.0.2",&my_addr.sin_addr);
my_addr.sin_port = 161;
err=new_bind(snmpskfd, &my_addr); /*bind socket with SNMP_PORT
```

```
if (err == -1){
ErrTyp = ERR_SO_BIND;
ErrMnm(); goto fini;
}
}
if (nflg == 1){
err=new_snmp_trap(&rcvpdu,SNMP_TRAP_WARMSTART, 0); /*trap--WarmStart*/
if (err <= 0) goto fini;
}
while (1)
{
err = new_recvfrom(snmpskfd, &SnmpSpace[0],MAX_SNMP_SPACE,&client_addr); /* Receive SNMP
message*/
if (err == -1){
ErrTyp = ERR_SO_RCVF;
ErrMnm(); goto fini;
}
err = new_snmp_parse(&rcvpdu,&SnmpSpace[0], err); /* Parse snmp packet*/
if (err <= 0){
if (err == -2)
err=new_snmp_trap(&rcvpdu,SNMP_TRAP_AUTHFAIL, 0); /*trap--Authfail*/
continue;
}
err = new_snmp_build(&rcvpdu); /*bind pdu according to the get/set request*/
if (err <= 0) continue;
err = new_sendto(snmpskfd,&SnmpSpace[0], err, &client_addr); /*send Get-Response*/
if (err == -1){
ErrTyp = ERR_SO_SEND;
ErrMnm(); goto fini;
}
}
fini:
new_close(snmpskfd); /*close the this SNMP socket connection */
snmpskfd = -1;
nflg =1;
err=new_snmp_trap(&rcvpdu,SNMP_TRAP_LINKDOWN, 0); /*trap--LinkDown*/
}
}
```

The complete function call flowchart of the μRET according to the present invention is shown in Figure 25. UDP, TCP and ICMP function calls are presented by three different applications: a SNMP agent (UDP), a web server (TCP) and a ping routine (ICMP).

The μRET of the present invention has been ported to two different processor architectures: microcontroller MSP430F149 and digital signal processor TMS320C5410. The following part of the description deals with measurement of the memory usage of μ RET in the two processor architectures, and compares μRET with other TCP/IP implementations with respect to code sizes and protocol functions. Then, the performance of μRET was evaluated.

The applicants analyzed the codes of μRET with respect to code size and data size of compiled objects. The code was compiled for two processor architectures: Texas Instruments (TI) digital signal processor TMS320C5410 and Texas Instruments microcontroller MSP430F149. The code for the first processor TMS320C5410 was compiled with the 'C5000 Code Composer Studio v1.20' IDE (integrated development environment) tool and the TMS 320C5410 EVM development motherboard, with compiler optimization option turned on. The codes for the second processor MSP430F149 was compiled with the 'MSP IAR C Compiler' development tool, with compiler optimization option turned on.

The TMS320C5410 is a 16-bit fixed point digital signal processor having a 100 MHz clock frequency. The TMS320C5410 has 80KW (W 'word' = 16 bits) internal memory (16KW of RAM and 64KW of ROM). The MSP430F149 is a 16-bit RISC microcontroller having a 8MHz clock frequency. The MSP430F149 has 62KB of internal memory (60KB of flash memory and 2KB of RAM). Analysis of compiled code size in the TMS320C5410 processor showed the SNMP module to have the greatest code size because this module requires the greatest memory space to be pre-allocated for the SNMP MIB variables. The PPP module represents approximately 40% of the total code size due to the complex PPP state automaton. An 11*6 two dimension data matrix was pre-allocated to store PPP state transition table and PPP two-way negotiation mechanism (LCP and IPCP), thereby increasing overhead of the PPP module. The TCP module represents approximately 20% of the total code size because of the complex mechanisms for the connection-oriented, reliable service. It has the largest size of all of the transport and internet layer modules of μRET. In view of the feature of 16-bit per word of the TMS320C5410, one way of reducing overhead for μRET is to use the first 8 bits of a word memory. For example, in TMS320C5410, a 'char' has 16 bits but only tail 8 bits are practically used and another 8 bits are thus wasted. Hence, using the idle 8 bits of each char is one significant way of decreasing memory usage.

Analysis of code and data sizes of each protocol module for the MSP430F149, was carried out. Due to the minimal system resources in this processor, the two application layer protocol modules, SNMP and HTTP, were not contained in the μRET when compiled for the MSP430F149. The code size for each protocol module was noticeably greater due to the fact that the MSP430F149 processor does not naturally support 32-bit operations and does not have an 8 bit idle space for a char, when compared to the TMS320C541 0 processor. Thus, any 32-bit operations will be expanded by the compiler into many more assembler lines in the MSP430F149 processor. Since the TCP module performs many 32-bit operations, it had the correspondingly greatest code size out of all of the protocol modules.

Performance of memory usage was analysed by comparing μRET with other TCP/IP implementations with respect to memory consumption and protocol functions,. Without the application layer SNMP and HTTP protocol modules, μRET only uses 13.7KB of memory for the MSP430F149 and 10.2KW of memory for the TMS320C5410. Compared to full-scale and generic TCP/IP implementations, such as KwikNet (158KB or 124.6KB), NicheStack (47KB) and NexGenIP (34.5KB), μRET of the invention therefore has far lower memory requirements. Additionally, when compared to known lightweight implementations, such as μIP (7.2KB), lwIP (13.8KB or 22.6KB), and Nichlite (10KB), μRET has far more protocol modules (PPP). In fact, if the PPP module is removed, μRET requires only 6.1 KB or 9.1 KW respectively, and can thus compete with any of the known lightweight TCP/IP implementations.

In order to meet different TCP applications and various hardware platforms, the μRET implementation can be further adapted to be able to meet the capacities of other processors. For example, in one preferred embodiment of the present application, the STAR device uses a MCU CP3000, providing more powerful computation capability and more internal memory. This MCU is designed to support different wireless sensor network applications. In view of variable network communication capacity and a large number of WSN nodes in the WSN applications envisaged by the invention, the μRET implementation for this processor provides RTT estimation ability and supports flow and congestion mechanisms. Again, the μRET implementation can be adapted to take into account network security and various authentication methods or encryption when connecting embedded systems to a WAN (wide area network) or even globally to the Internet. The μRET implementation is thus fully modular. Not only is it possible to add or remove each protocol module easily, but also each function component can simply be added to or removed from any particular protocol module. It is thus possible to modify the μRET implementation in such a way as to select those protocol modules and function components that provide efficient and reliable communication capability adapted to different platforms and applications.

In still yet a further object of the present invention, the applicants have devised embedded real-time diagnosis techniques for an Automated ECG Diagnosis (AED) system, and a corresponding system, known herein as the Star system.

Cardiovascular variables, such as heart rate, arterial blood pressure and the shape of the QRS complexes in the cardiac electrical signals, are almost "periodical", showing some variability on a beat to beat basis. This variability reflects the interaction between perturbations to the cardiovascular variables and the corresponding response of the cardiovascular regulatory systems. Therefore, the analysis of such variability can provide a quantitative and non-invasive method to assess the integrity of the cardiovascular system. The ECG (electrocardiogram), that is the body-surface manifestation of the cardiac electrical potentials, is the most prescribed diagnostic measure in medicine and is routinely used to diagnose heart disease, identify irregular cardiac rhythms (arrhythmias), evaluate the effects of drugs, and monitor surgical procedures. The magnitude, conduction, and duration of these potentials are detected by placing electrodes on the patient's skin. The ECG trace or plot is a series of waves that represent the electrical events in the heart. By examining the sequence of events on the ECG, it is possible to diagnose cardiac arrhythmias. Developments in computerized patient monitoring systems over the recent years have made remarkable demands on automatic ECG analysis systems. Many efforts have been focused on the enhancement of the efficacy and the accuracy of such AED systems and a variety of AED algorithms have been presented based upon different domains and/or techniques by researchers. The AED is one of real-world application of automated pattern recognition. The essential problem of pattern recognition is to identify an object as belonging to a particular group. In AED, it is to classify a cardiac cycle into a particular group of cardiac status by observing statistical features and morphological features of ECG signals. Assuming that each group of cardiac status has exclusive attributes, the problem of assigning an unlabelled cardiac cycle to a group can thus be accomplished by determining the attributes of this cardiac cycle (pattern) and identifying the group of which those attributes are most representative (recognition). Given the goal of classifying an object, i.e. the status of a cardiac cycle, based on their attributes, the functionality of an AED can be divided into two basic tasks: the description task generates attributes of an object using feature extraction techniques, and the classification task assigns a group label to the object based on those attributes with the rhythm classification. The description and classification task work together to determine the most accurate label for each unlabelled object analysed by an AED algorithm. This is accomplished with a training phase that configures the algorithm based on a collection of objects whose labels are known,i.e. a training set.

In terms of signal capture techniques, cardiac electrical signals are categorized into two classes: one from body-surface electrodes which is the most commonly performed cardiac test, named ECG (Electrocardiograph); another from implantable leads placed inside a patient's heart, which is therefore an invasive cardiac signal, called EG (Electrogram). The electrogram is mostly used in ICDs (implantable Cardioverter Defibrillators) that are implanted inside the body and can deliver pulses or shocks to treat rapid heart rhythms by continuously checking the heart's electrical signals. In contrast to ECG signals, the electrogram obtained from ICDs are generally of a higher quality and stability, and are free of disturbances originating from external situations, devices and patients, which include baseline drift, muscle tremor, electrical noise and motion artefacts. These external factors are mainly interferences attenuating and polluting the electrical signals. The ICDs, as a therapy for cardiac arrhythmias, are suitable for those patients having serious rapid heart rhythms such as VT and VF, but do not prevent and cure the underlying disease that causes cardiac arrhythmias. Moreover, the disturbance to daily movement, expensive fees, and even malfunctions, make ICDs generally unsuitable as a general cardiac care system for most people. The ECG signals are further classified into two types in view of the application environments: resting ECG (RECG) and ambulatory ECG (AECG). RECG is used for stationary hospital-bedside monitoring and diagnostics. It is a short-term (several hours) stable ECG record typically obtained from an ECG machine. AECG is generally used for long-term heart monitoring services normally acquired from sensors outside of laboratories or hospital care units. Compared to AECG, a resting ECG has a generally cleaner and more stable signal, and is free of the interferences caused by motion artefacts, which are the critical problem of AECG. Using RECG signals, AED algorithms have higher accuracy, but the counterpart thereto is that patients' motions are completely constrained. Moreover, cardiac arrhythmia events do not always occur in a limited clinical detection period and the decision-making rules of heart diseases are not entirely the same in a situation of movement when compared to a resting situation. RECG is thus suitable for monitoring in a clinical resting environment, but not illfitted to the requirements of daily real-time heart care. Since AECG signals are acquired outside of a laboratory, they are generally contaminated by environment factors, such as the electronic noises of nearby circumstances, the muscle tremor of patient and the unstable connections between electrodes and skin caused by patient movement. Compared to RECG, AECG signals show more non-stationarities, which are influenced by noises of transient phenomena or slow variance (trends). Noise causes significant problems in the identification of heart rhythms, so a signal preprocessing procedure is necessary to reduce the interferences of noise and to prepare clearer signals for the subsequent analysis tasks.

To date, there are three approaches to acquiring an AECG :

Holter monitoring generates a continuous recording of the ECG, usually for 24 hours. It does not constrain the daily motions of patients. The test is useful for detecting cardiac arrhythmias that may not appear during a resting ECG at the doctor's office. However, the short-term detection, say over 24h, may also miss some infrequent cardiac arrhythmias and the lack of real-time diagnosis prevents the implementation of real-time heart care.

The event recorder is carried over a period of days or weeks, and is used to record cardiac arrhythmias that occur infrequently. When symptoms occur, the patient activates the recorder which records and stores several minutes of ECG signals. Like Holter, this device lacks the ability to carry out real-time diagnosis and its performance depends only on the subjective interpretation of patients' feelings and is therefore unreliable.

The exercise ECG test, or treadmill test, allows cardiologists to record ECG signals during the stress of exercise. It can help bring on cardiac arrhythmias that may not occur during a resting ECG. The drawbacks of the device are its limitations in time and space because this test can practically only be done in hospital and can not run for long periods of time.

Due to the fact that severe noise pollution often occurs in AECG, the AED algorithms for AECG are different to those of RECG and EG. The traditional techniques of treating AECG signals are limited in time and / or space, and consequently most of them do not support real-time ECG detection and diagnosis and are unsuited to real-time long-term heart care.

ECG signals are acquired through electrode pads that are attached to the patient's chest, generally in a anterior-anterior placement. Since Ambulatory ECG takes place over a relatively long period and under the influence of the exterior environment, there is a higher probability of contamination by various interferences caused by noise. An artefact is an electrical signal caused by noise, and which is unrelated to cardiac behaviour. If artefacts cannot be effectively removed from or reduced in the ECG, they will cause incorrect detection of heart rhythms, and lead to inappropriate cardiac arrhythmia interpretations. For this reason, many AECG signals are digitized and processed to remove or reduce the effects of certain types of electrical interference before ECG detection and diagnosis is carried out on said signals. Sources of artefacts can be characterized as either controllable or uncontrollable. For example, direct manipulation of the electrode pads or the movements of the patient are examples of artefacts that can be controlled. Correspondingly, electrical interference and patient muscle tremors are sources of artefacts that are generally not within the control of the patient.

Baseline drift originates from high skin impedance caused by unstable electrode contact and poor skin preparation. This interference can be effectively eliminated by clinical means shown as follows:

de-greasing skin with alcohol and drying the skin with gauze, which lowers skin resistance;
employing the electrodes that are made of silver coated with silver chloride with stable impedances reducing slow drift;
attaching gel-impregnated foam pads that decrease movement artefact and lower skin impedance;
securely connecting electrode to skin and lead wire to electrode.

The technical solution is to employ a high-pass filter with cut-off frequency at 0.1 Hz to reduce low-frequency baseline drift.

Electrical interference caused by the electrical noise from nearby equipment may contaminate the ECG with a 50 Hz (France) or 60 Hz (America) signal. This interference is attenuated by a narrow stopband filter called a notch filter, which is tuned to remove the 50 or 60 Hz signals.

Muscle tremor present when a patient is tense, cold, or affected with muscle or nerve disorders may contaminate the ECG with high-frequency baseline noise. A low-pass filter can remove much of the noise. Unfortunately, the frequency spectrum overlaps the ECG frequency, so it is not possible to remove all of it without destroying ECG data. A 40 Hz low-pass filter is used in the present invention to minimize artefact from muscle tremor.

Electrodes on hairy chests and loose skin or acute motion can cause the electrodes to fall off or result in poor contact with the patient. Motion artefact commonly has an irregular rhythm and an irregular morphology that introduces increased difficulty in recognising QRS complexes. By monitoring the impedances between the electrodes and the patient, a procedure has been developed by the present applicants that can detect conditions that could compromise the algorithm results. For example, a pad that is not making good contact with the patient interferes with ECG data acquisition. The impedance data in this case suddenly changes to very high values and back again. Similarly, when a pad comes off completely, the impedance data suddenly changes to infinite. The procedure of the present invention monitors the impedance data for this type of change and alerts patient to reattach pads, if necessary. Moreover, the motion artefacts which have QRS-like waveforms will confuse the primary decision of the location of the QRS complex in the signal, and will require subsequent processing to confirmed and correct the detected QRS series. For the diagnosis algorithm in ambulatory ECG signals, one of the crucial problems is the discrimination of the QRS complex from motion artefacts. In fact, for each component of the present AED method, a solution is provided to eliminate the disturbance of motion artefacts.

Heart rhythms have certain attributes that can be used to recognize cardiac arrhythmias. These attributes are quantified by the measurements extracted from AECG signals. They are classified into two categories:

statistical features;
morphological features.

Statistical features reveal the quantitative changes of heart beat and are used to characterize heart rate variability (HRV), while morphological features describe the morphological interrelation between rhythms. The varieties of morphologies are related to the rhythm waveforms and their implications with respect to cardiac behaviour. These relationships are clearly understood by cardiologists and can be represented by foundational attributes, such as extreme amplitude, slope and duration, etc.

The time that elapses between consecutive heart beats, and is defined as that between two P waves, is named atrial heart rate when the P wave describes the atrial depolarization of cardiac behaviour. Considering the difficulty of locating the P wave in ambulatory data, it is ventricular beat-to-beat interval that is practically used to define the heart beat interval, named ventricular heart rate. The QRS complex represents ventricular depolarization. Compared to the P wave, the R wave has cleaner amplitude and a better frequency resolution, thus making it the signal of choice to detect the ventricular heart rate. As the time interval between the P and R waves can be assumed and has been shown to be constant, the atrial heart rate can be considered to be equal to the ventricular heart rate. The time intervals of consecutive heart beats are thus named RR interval time series or RR intervals. At the moment, the sequence of successive RR intervals is the simplest, but the most important time series that can be used to characterize HRV, either in time or frequency domain. In ECG signals, defining the positions of two consecutive R waves as $s(t)$ and $s(t+1)$,

t=1...N, where t is the time parameter of a series and N is the length of this series. The expression RR(t) = s(t) - s(t+1) is represented for RR intervals. A heart rate time series [min$^{-1}$] is calculated by the expression HR(t) = 60 *($F_{sample}$/RR (t)). The expression HRavg = (1/N)*$\Sigma_{i=1}^{N}$HR(t) shows the mean heart rate. These expressions reveal a non-linear relation of ECG time series and the results indicate the statistical features of AECG.

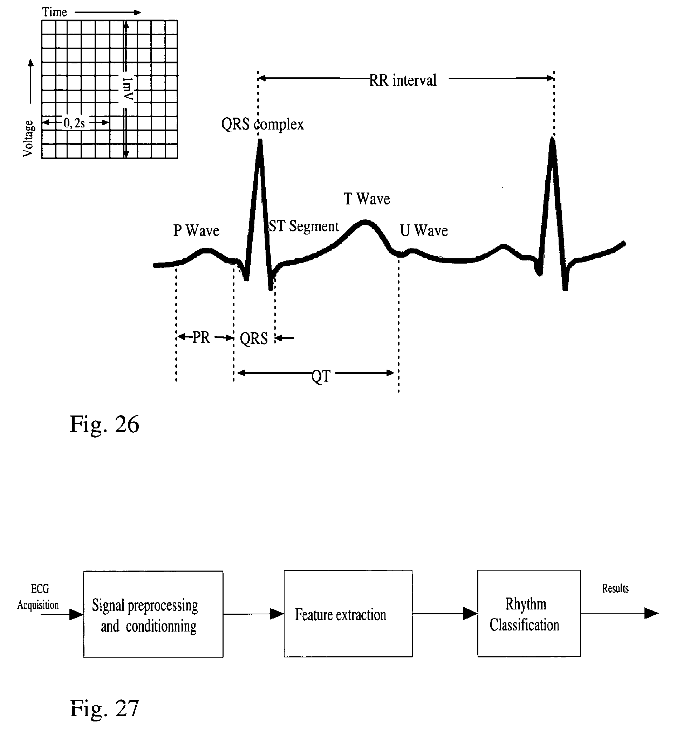

The morphology attributes of AECG are used to classify the QRS complexes of ECG signals. The different phases of a cardiac cycle in ECG are represented by their corresponding wave components. The P, QRS and T waves are represented respectively as atrial depolarization, ventricular depolarization and ventricular repolarization, as shown in Figure 26. The length of the QRS is defined as the time interval between the onset of Q wave (Qi) and the end of S wave (St), and is designated LQRS. It represents the duration of ventricular depolarization, and is usually used to measure the changes occurring with the ventricular depolarization and repolarization period. However, due to poor SNR (Single-to-Noise Rate) and the polymorphic nature of the QRS complex in AECG signals, it is nearly impossible to locate precisely the Qi and St points. In the AED method of the present invention, the time interval between the end of Q wave (Qt) and the onset of S wave (Si) is used to estimate LQRS.

In ECG signals, defining the positions of Qt and Si points of each QRS complex as Qt(t) and Si (t), t=1...N, the expression LQRS(t) = 1000 *($F_{sample}$ /(S, (t)-$Q_t$(t)) indicates the LQRS time interval in millisecond. The other morphological attributes, such as the amplitude of R wave (VR), the two slopes of QRS (SP, SN) then are extracted by studying the QRS complex.

The AED system according to the present invention replaces the cardiologist in the diagnosis of ECG signals. The diagnosis of cardiologists is rooted in their knowledge gained through experience of electrocardiographs obtained from long-term studying. In contrast to human diagnosis, the AED system of the present invention uses a dedicated algorithm to process ECG signals collected by sensors, resulting in a rhythm classification and arrhythmia interpretation. The AED algorithm of the present invention is commonly divided into two tasks: a description task and a classification task. The description task transforms the ECG signal into features, i.e. the characteristics that are derived from ECG and are representative of the heart rhythms. The description task can involve several different but interrelated activities:

signal preprocessing to modify the data to correct deficiencies in the ECG signals due to the limitations of the sensor and the interferences of noise, and/or to prepare the conditioning signals for subsequent activities later in the description task or in the classification task;
feature extraction is the process of generating features to be used in the classification task. According to diagnosis techniques, the features can either be derived from time domain data directly, and/or are generated by performing manipulations and /or transformation on the data.

Feature extraction is an important aspect because the features made available for discrimination directly influence the efficiency of the classification task: features which truly discriminate among groups will assist in identification, while the lack of such features can impede the classification task from arriving at an accurate identification. The end result of the feature extraction task is a set of features, commonly called a feature vector, which constitutes a representation of the data. In the AED system of the present invention, two classes of features, i.e. the statistical features and the morphological features, are extracted from the AECG signal. The classification task uses a classifier to map a feature vector to a group. Such a mapping is specified either by hand, or induced from a training set in a training phase. Once formulated, the mapping can be used to assign identification to each unlabelled feature vector subsequently presented to the classifier. In the AED system, the rhythm classifier identifies rhythms based on the heart rate and the morphological attributes of QRS complex, and consequently interprets corresponding cardiac behaviours.

The signal features of AECG determine the complexity of the diagnosis techniques that are incumbent in accurate diagnosis. Compared to RECG and EG, AECG has more disturbances caused by a variety of noises. Due to their irregularity and QRS-like features, motion artefacts reduce the performance of ECG diagnosis. In the past twenty years, automated ECG diagnosis techniques have improved vastly. Based on various pattern recognition techniques and distinctive theoretical foundations, a large set of feature extraction techniques and rhythm classification techniques have been developed. However, because most of the actual AECG devices do not provide the ability to carry out real-time long-term diagnosis, most of the diagnostic algorithms focus only on the reliability and accuracy of diagnosis, in which the efficiency and real-time requirement cannot be studied correctly. Thus, one of the objects of the present invention is to develop an AED algorithm suitable for real-time continuous cardiac arrhythmia detection and diagnosis in sensor devices and on remote telemedicine platforms. In order to overcome the shortcomings of conventional ECG monitoring methods, the present invention provides a diagnosis system that can support long-term, i.e. from a few days to weeks, real-time ECG monitoring and allows daily patient movements in exterior environments. The AED method of the present invention is thus free of the limitations in time and space that are known in current implementations. In addition, in order to provide a cardiac diagnosis function for a sensor system, the algorithm is both simple and efficient, and can be implemented in VLSI or integrated into an embedded system.

The architecture used in the present invention is shown in Figure 26. It consists of three components:

Data acquisition and signal conditioning;
Feature extraction;
Cardiac arrhythmias classification and interpretation.

The signal preprocessing and conditioning component acquires the ECG signal from sensors and removes unwanted noises and artefacts. The "clean" ECG signals are prepared for the feature extraction component. In the feature extraction component, the algorithm yields feature sets from the measurements of AECG signals that are used to discriminate heart rhythms. Finally, in the rhythm classification component, the classifier uses the feature sets to classify rhythms and to interpret cardiac arrhythmia events.

The electrocardiograph, as a non-invasive and easy-acquired cardiac test, is routinely used to diagnose heart disease and monitor cardiac behaviours. The features of the ECG signals, including the variety of heart beat and morphologies, and their implications with respect to cardiac activities are well understood by cardiologists. The automated pattern recognition techniques have been widely used in ECG diagnosis in recent years. A variety of diagnostic techniques based upon the theories of pattern recognition have been proposed, and a lot of automated ECG diagnosis systems have been developed practically. A heart beat, or a cardiac cycle, consists of several phases that include atrial depolarization, AV conduction, ventricular depolarization and ventricular repolarization. In a ECG signal waveform, a cardiac cycle is divided into three main waveform components: P wave, QRS complex and T wave. The characteristics of a cardiac cycle are classed into two categories: statistical features and morphological features. Insofar as the pattern recognition concept is concerned, a cardiac cycle is defined as an object; the features extracted from ECG signals are named a feature vector; a group is a pattern template with respect to one class of cardiac behaviour. The feature vector of each group has exclusive feature values. Through the experience of cardiologists, or through training rules obtained by studying a training set, a cardiac cycle can be classified into a group of cardiac status via feature vector template mapping.

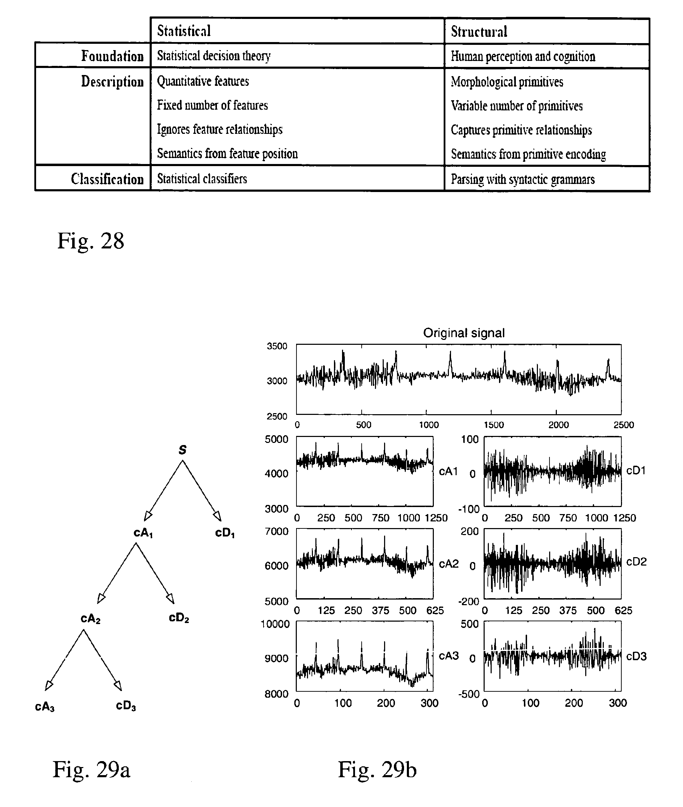

According to the classification methods of pattern recognition, there are two fundamental approaches to implementing an AED system: statistical and structural. Each approach employs different techniques within the description and classification tasks which constitute a whole algorithm for the AED system. Statistical pattern recognition draws from established concepts in statistical decision theory to discriminate among signals from different groups based upon quantitative features of the signals. There is a wide variety of statistical techniques that can be used within the description task for feature extraction, ranging from simple descriptive statistics to complex transformations. The typical techniques include mean and standard deviation computations, Fourier transformations, wavelet transformations, etc. The quantitative features extracted from each object for statistical pattern recognition are organized into a fixed length feature vector where the implication of each feature is determined by its position within the vector. The collections of feature vectors generated by the description task are then passed to the classification task. Statistical techniques used as classifiers within the classification task include those based on similarity, e.g. template matching, nearest neighbor; probability, e.g. Bayes rules; boundaries, e.g. decision trees, neural networks; and clustering, e.g. k-means, hierarchical. Structural pattern recognition, sometimes referred to as syntactic pattern recognition due to its origins in formal language theory, relies on syntactic grammars to discriminate among ECG data from different groups based upon morphological interrelationship or interconnection. Structural features often referred to as primitives, represent the signal building blocks and their relationship. Morphologies used to extract structural features from the signal result in primitives, such as edges, curves, and regions. Structural feature extraction techniques include chain codes, piecewise-linear regressive and curve fitting. The classification task comes to an identification using parsing: the extracted structural features are identified as being representative of a particular group if they can be successfully parsed by a syntactic grammar. A summary of the differences between statistical and structural approaches to pattern recognition is shown in Figure 28.

The essential dissimilarities are twofold: (1) the description generated by the statistical approach is quantitative, while the structural approach produces a description composed of building blocks; and (2) the statistical approach discriminates based upon numeric differences among features from different groups, while grammars are used by the structural approach to define a "language" encompassing the acceptable configurations of primitives for each group.

The quantitative property of statistical pattern recognition makes it sensitive to numerical changes, hence statistical methods are suitable for discrimination among groups based on statistical features of ECG signals- i.e. heart rate. However, this approach is not adapted to discrimination among groups based on the morphological features of ECG signals, because it normally lacks an efficient method to reflect the morphological building blocks and their interrelationships. The morphological features of ECG signals can be described by the fundamental primitives of the structural approach. The syntactic grammars for ECG diagnosis are readily available based on the rules gained through the experience of cardiologists. However, due to the fundamental complications associated with the implementation of the description and classification tasks, the usefulness of the statistical pattern recognition approach is also limited. For the purposes of the present invention, the applicants have adopted the statistical pattern recognition technique, but morphological features are quantified according to a set of independent definitions and the rules of experience of cardiologists.

The statistical and structural approaches adopt different signal features because of their different theoretical foundations, and thus employ different diagnostic techniques to implement both the description and classification tasks. The approximate periodic property of ECG signals reflects the regularity of cardiac behaviours, which is the foundation of ECG diagnosis. In view of the signal features and theoretical foundations, the diagnostic techniques can be classified into several categories: time domain, frequency domain, time-frequency, wavelet, neural networks and chaotic modelling.

In cardiac contraction, heart electrical signals originate from the sinoatrial node, and then rapidly spread from atrial to ventricular via an A-V pathway. Hence cardiac electrical potentials have rapid changes and high magnitude. While in cardiac expansion, cardiac electrical potentials will rapidly return to the baseline potential and keep to this potential until the next heart beat. Observing the potential changes of ECG signals is thus the main measurement of time domain analysis. The changes of cardiac electrical potentials are quantified and used to extract the statistical features from ECG signals in the time domain. Fortunately, in contrast to other domain analyses, the statistical features in the time domain have been studied in detail. Time domain analysis is based on peak measurement, which evaluates each event of a heart beat in an ECG signal. The evaluation methods comprise feature extraction (pattern) and template matching (recognition). The template in time domain analysis can either be defined as the sample set of a waveform complex, or the particular extracted statistical features. The common statistical features of the ECG signals include extreme amplitude, an optional extreme slope/curvature, and the duration of QRS, etc, which can be obtained by detecting the waveform of the complex. By tracking the change of ECG time series, the template matching method requires huge computation to obtain the correlation coefficient between signal and template. In one known method, the template matching algorithm is expressed as follows, and is designated correlation waveform analysis (CWA).

$$\rho = \frac{\sum_{i=1}^{N} \left( (t_i - \bar{t})(s_i - \bar{s}) \right)}{\sqrt{\left( \sum_{i=1}^{N} (t_i - \bar{t})^2 \sum_{i=1}^{N} (s_i - \bar{s})^2 \right)}}$$

Where :

$\rho$ = the correlation coefficient
N = the number of template points
$t_i$ = the template sample point
$s_i$ = the signal sample point under analysis
$\bar{t}$ = the average value of the template samples, and
$\bar{s}$ =the average value of the signal samples.

The correlation coefficient falls within a range $-1 < \rho < +1$, where +1 indicates a perfectly matched signal and template. Applying to a data set with the length k, the CWA has a computational complexity of $O(k*(n^4+n^2+1))$. A simplified implementation of the CWA is provided to decrease computation overhead. Since the ECG signals in the expansion time have electric amplitudes nearly equal to the zero isoelectric baseline, it has no contribution to recognition of rhythm. Therefore, only the ECG signals in the contraction time are applied to perform template matching in the simplified CWA. For a healthy adult, the heart rate (HR) is around 60bmp to 100bmp under normal conditions and the length of QRS complex is no more than 200ms, which means that there is only 20% to 33.3% (0.2*HR/60) computation cost for this simplified CWA compared to those of classical CWA. This simplified implementation needs reliable QRS position information before effecting template matching. The position information can be obtained by analysing the morphological attributes of QRS complex, based on the mean and standard deviation computations. The relative formulation is well known and doesn't require any complex calculation. When adopting a statistical approach to define morphological features of a QRS complex and to describe a pattern template in time domain analysis, the computational complexity can be markedly decreased to $O(n)$. A heart rhythm can be classified basing on the predefining syntax analysis of the statistical features. From this point on, the statistical features indicate the original statistical features and the morphological features defined by the statistical approach. In time domain analysis, the statistical features are directly extracted from original ECG signals, which include the peak-to-peak amplitude, the waveform slope, the length of the QRS complex, the sequence of slope patterns, and the sequence of amplitude threshold intercross points. Time domain techniques have the advantage of relatively easy implementation. They have thus been widely applied in real-time AED systems. The main drawback of time domain analysis is the limitations of its anti-noise ability. The ECG signal, especially in AECG, is a faint electrical signal of cardiac activity which is often diminished or modified by a variety of noises caused by physical and technical effects. It is thus necessary to perform signal preprocessing and conditioning to get better SNR signal for the subsequent feature extraction and rhythm classification tasks.

The ECG signal has statistical features in the frequency domain that consists of a variety of spectral components. The different waveforms of ECG signal, i.e. P wave, QRS complex, and T wave, have their particular spectra that are respectively represented as the atrial frequency and the ventricular frequency. For a healthy adult, the frequencies of atrial and ventricular are approximately equivalents that are equal to 1 to 1.7 Hz. Because the P and T wave are often undiscernible from artefacts in AECG, the practical cardiac frequency is represented by the ventricular frequency (QRS). The interferences caused by noise are unavoidable in AECG. The different origins of interference have different frequency components. For example, the frequency of electrical interference is 50 Hz (France) or 60 Hz (America), the frequency of baseline drift is normally less than 0.1 Hz, and muscle tremor has a frequency of more than 40Hz. Therefore, and in accordance with the present invention, a band-pass filter which has the frequency range of 0.1 to 40 Hz is used to remove the spectral components of noise. The theory of frequency domain analysis is based on the assumption of signal stationarity. The ECG signal is a discrete-time random process that is thus assumed to have wide sense stationarity. The power spectral density (PSD) of a wide sense stationary random process is the Fourier transformation of the autocorrelation function. Defining x(t) as a ECG signal, where t = 0,1,2,... the autocorrelation function is defined by $r(t_1, t_2) = E[x(t_1)x(t_2)]$. For a wide sense stationary process, the mean is constant and the autocorrelation depends only on the time difference $\tau = t_1 - t_2$, so that $r(t_1, t_2) = r(\tau)$, $r(\tau) = E[x(t+)\times(t)]$. The PSD is thus denoted by :

$$S(\omega) = \sum_k r(k) e^{-jwk} \quad \text{(Fourier)} \quad S(z) = \sum_k r(k) z^{-k} \quad \text{(Z)}$$

Frequency domain techniques aim to analyse the frequency components of ECG and their relative power spectral density. Applying to a data set of length k, Fourier transform has a computational complexity of O(klogk). The assumption of frequency domain analysis is based on the ECG signal having wide sense stationarity. In practise, however, because of its wide origin and the nature of its information, the ECG signal can hardly be regarded as a wide sense stationary process under any condition. It is often necessary to divide the ECG recording into shorter stationary sequences. The identification of non-stationarity which includes transient phenomena, i.e. spikes and slow variance, i.e. trends, is thus more difficult. Frequency domain analysis is often proposed for the classification of rhythms, but little success has been solidly demonstrated for the recognition of ventricular tachycardia (VT). Examination of longer segments of 1000ms to 15,000ms yields the same phenomenon because the power presented in small visually distinctive high frequency notches is insignificant compared to the remainder of the signal, and the changes in polarity of the waveform, easily recognized in the time domain, are simply not revealed by frequency analysis.

Wavelet analysis is a new and promising technique for analysing ECG signal. Like Fourier analysis consists of breaking up a signal into sine waves of various frequencies, similarly, wavelet analysis is the breaking up of a signal into shifted and scaled versions of the original (or mother) wavelet. The continuous wavelet transform (CWT) is defined for a signal (x(t)) as the sum over all time of the signal multiplied by scaled, shifted versions of the wavelet function ($\Psi_{a,b}(t)$) :

$$C(a,b) = \int_{-inf}^{+inf} x(t) \, psi_{(a,b)}(t) \, dt$$

Where a and b is the scaling and shifting factor respectively. The results of the CWT are many wavelet coefficients C(a, b), which are a function of scale and position. Wavelet functions are located in time by the shift factor b, while the scale factor a indicates the width of the wavelet. Actually, there is a correspondence between wavelet scales and frequency as revealed by wavelet analysis:

Low scale a => Compressed wavelet => Rapidly changing details => High frequency w.
High scale a => Stretched wavelet => Slowly changing, coarse features => Low frequency w.

In order to improve the efficiency of wavelet analysis, discrete wavelet transforms (DWT) are introduced. It determines scales and positions based on powers of two so called dyadic scales and positions. The DWT represents a signal (x[n]) defined at n sequential points at n points in times as a linear combination of a family of wavelets ($\psi_{j,k}[n]$) :

$$x[n]=\sum_{j,k\in Z} C_{jk}\psi_{j,k}[n], a=2^j, b=ka, j,k\in Z$$

Where $C_{j,k}$ is the coefficient of the wavelet function $\psi_{j,k}[n]$. The DWT produces a family of hierarchically organized decompositions termed approximation and detail respectively. The approximations are the high-scale, low-frequency components of the signal. The details are the low-scale, high-frequency components. The wavelet decomposition tree shows in Figure 29a (order=3). The $cA_x$ are the approximation components of ECG, and $cD_x$ are the details components (see Figure 29b).

Since wavelets have scale aspects and time aspects, wavelet analysis can reveal the frequency and time characteristics of ECG signals. The motivation for applying the wavelet transform to the analysis of ECG signal lies mainly in the monitoring of non-stationary signals and the long-term evolution of the power spectrum, such as the removal of baseline wandering, ECG de-noising and QRS complex detection. The capacity of wavelet decomposition lies in the removal of undesired components, essentially noise, from the signal by a thresholding operation. A new de-noising technique has also been described which uses a low pass filter (LPF) named Daubechies filter to smooth waveform transform coefficients instead of the thresholding of Donoho's method. There is also a rhythm classification technique for the discrimination of ventricular tachycardia from supraventricular tachycardia (SVT) based on wavelet coefficients. Applying to a data set of length k, the wavelet transform has a computational complexity of O(klogk). The statistical features of wavelet transform, such as wavelet coefficients and power spectrum, describe the local "frequency" and "morphology" properties of a non-stationary time series in the neighbourhood of each time point. The wavelet transformation employs basis waveforms generated by scaling and translating a mother wavelet, thereby enabling the technique to extract features that are both global and local within a time series. The basis waveforms, however, are derived from a single morphology as defined by the mother wavelet. The main advantage of wavelet analysis is that there is no need for any pre-processing tool for ECG fluid point detection, since the wavelet algorithms can work without any additional information intervention. However, wavelet decomposition and reconstruction of the approximation must be performed more than once, with the result that computational overhead is significantly higher in comparison to other methods based mainly on polynomial interpolation.

Time-frequency analysis has emerged for monitoring the spectral properties of the signal as time elapses, especially, when "long" time periods are under consideration. The temporal location of the spectral components may give more information than one single spectrum. Time-frequency spectral analysis is an advanced spectral analysis method which can generate multiple spectra over time instead of a single spectrum for the whole data segment, as is the case with the FFT (Fast Fourier Transform Algorithm) method. Furthermore, time-frequency analysis based on the so called "quadratic Time-Frequency Distribution (TFD)" can provide good resolution in both time and frequency. As an example of quadratic TFDs, the Wigner distribution (WD) is given:

$$Wx(t,w)=\int x(t+v)x*(t-v)\exp(-iw\tau)d\tau, v=\tau/2$$

The Smoothed Pseudo Wigner-Ville Distribution (SPWVD) was used in other work to analyse the spectral components of heart rate variability (HRV) during anesthesia. The discrete Wigner distribution (DWD) was applied to a non-stationary time series, and a modified algorithm was proposed for auto- and cross-DWD. The cross-terms were suppressed by means of a smoothing data window and a Gauss frequency window. This approach has been employed further in a study of the influence of respiration on heart rate, showing its ability to estimate spectral changes in non-stationary RR interval time series.

Classification of the electrocardiogram using neural networks has become a widely used method in recent years.

The efficiency of these classifiers depends upon a number of factors including extracted features, system architecture and network training. The statistical features must be carefully selected from feature vectors. The features that are crucial for identification rhythm will be kept and then the others will be excluded from feature vectors. A well-chosen training algorithm, results in an ANN which is capable of generating a non-linear mapping function with the capability of representing relationships between given ECG features and cardiac disorders. In designing an ANN, the key factors with regard to the system architecture include: the appropriate number of hidden layers, the nodes numbers of each layer, the activation function and its configuration. Golam Hosseini et al presented an extraction technique of the features for ANN-based ECG classifiers to enhance the performance of automatic detection and classification of cardiac arrhythmias. Chris. D. Nugent et al developed prediction models, based on Neural Networks and Genetic Programming respectively, to indicate the point at which training should stop for Neural Network based Electrocardiogram classifiers in order to ensure maximum generalization. The results show that both approaches provide close fits to the training data. Farrugia et al designed an artificial neural network classifier to recognize tachycardias from the ventricular intra-cardiac electrogram. It performed classification on a set of easily extracted features that characterize waveform morphology and rate.

Chaos theory is a new branch that is applicable to the modelling of non-linear dynamical systems. The most recent discussions have applied chaotic modelling to non-stationary time series, in particular the analysis of ECG. Cohen et al studied the Poincaré equation :

$$x(t) = Ax(t - 1)(1 - x(t - 1)), 2 \leq A \leq 4$$

and some of its solutions as an example of a chaotic system. The first order difference plot was introduced by presenting x(t+1) vs. x(t), and given as a measure of the degree of chaos in a system. In Huikuri et al., an ellipse was fitted to the plot and the centroid of this ellipse and the lengths of both axes were determined. The second-order difference, obtained by presenting (x(t+2)- x(t+1)) vs. (x(t+1) - x(t)), would show the degree of theoretical chaos. A quantitative measure of the degree of variability in a second-order difference plot, the measure of central tendency (CTM), takes the form :

$$CTM = \frac{\sum_{i=1}^{N-2} \delta(d(t))}{N-2},$$

$$\delta(d(t)) = \begin{cases} 1, & if \ \sqrt{(x(t+2)-(x(t+1))^2 +(x(t+1))-x(t))^2} \ < \ r \\ 0, & otherwise \end{cases}$$

Where N is total number of time series values and r is the radius of the central area. Several popular methods exist for studying chaos in cardiovascular time series, but the indices proposed for measuring it should be applied cautiously and results obtained should be interpreted with care. The basic condition is the need for large amounts of data which make these methods not well-suitable for real-time analysis. The problem also lies in discriminating a chaotic system from a random one, and calculation of the correlation dimension.

The objective of developing a real-time embedded ECG diagnosis system determines that the AED methods according to the invention must be both simple and efficient. A simple algorithm means two basic things: the first is to provide easy implementation of the algorithm, which ought to have a compact system architecture and thus capable of being integrated into microprocessors or be carried out by VLSI; the second is to ensure low computational overhead of the algorithm, so that it only requires minimal resources and power consumption. An efficient algorithm will enable accuracy and reliability of an AED system that is the crucial to a cardiac surveillance system. Based on the considerations mentioned above, the applicants' method employs techniques of time domain analysis that ensure simplification of the method. In order to improve system efficiency, some particular techniques are adopted that are presented hereafter.

ECG features, statistical features and morphological features defined by statistical methods, are extracted from ambulatory ECG in a features extraction module of the AED method of the invention. There are three main phases in this module: first, a QRS detection procedure locates the positions of QRS complexes and then obtains the statistical features of ECG signals, i.e. heart rate. Consequently, the QRS extraction procedure analyses the morphological attributes of the QRS complex and extracts the associated morphological characteristics, such as the peaks of the R wave, slopes and duration of QRS complex. Finally, the QRS correction procedure identifies the detected QRS complexes and corrects fault detections based on the standard feature vector. The two main problems of ambulatory ECG processing, i.e. the interferences caused by motion artefacts and the unreliability of the QRS complex, need to be handled specially in the features extraction module.

Due to the non-stationary and easily-disturbed natures of ambulatory ECG data, ECG acquisitions must be de-noised before further making detection. Most artefacts, such as baseline shift, electrical noise and muscle tremor interference, can be effectively eliminated or reduced by choosing suitable filters.

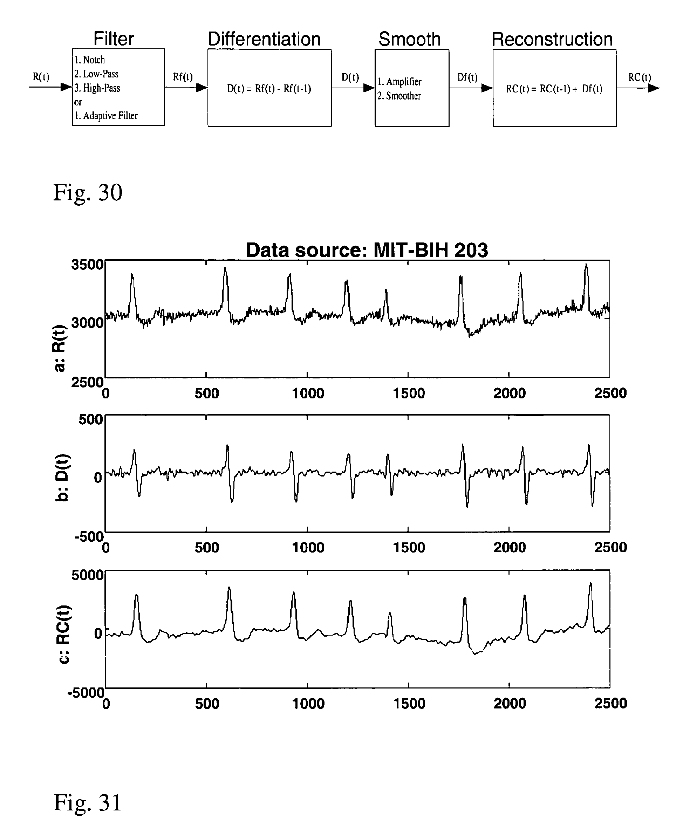

There are three ECG time series used in the method according to the invention that are represented as R(t), D(t) and RC(t) respectively. R(t) represents the raw ECG acquisitions acquired directly from a wireless ECG sensor (WES) via electrodes, which is generally contaminated by various noises. D(t) indicates the differential signal based on R (t). It represents the deviation change between two consecutive samples and is expressed as D(t) = R*(t) R*(t-1), R*(t) = FILTER(R(t)), where FILTER is the operation of a set of de-noising filters. RC(t) is the reconstruction signal based on the differential signal D(t) and its expression is RC(t) = RC(t-1)+SMOOTH(D(t)), where SMOOTH is the operation of a set of simple smoothing and amplifying filters. The signal preprocessing module is shown in Figure 30, and describes the relationships of the three ECG time series.

In view of the spectral components of AECG, the high-frequency components of noise, i.e. electrical noise and the interference caused by muscle tremor, can be effectively removed by operation of a low-pass filter and/or notch filter. The electrical noises which have the frequency of 50 Hz (France) or 60 Hz (America) are eliminated by a notch filter, and the interferences caused by muscle tremor are removed by a low-pass filter with a cut-off frequency of 40 Hz.

It should be noted that it is possible that the frequency spectrum of high-frequency noises overlaps the ECG frequency. From the point of view of wavelet analysis, the high-frequency components reflect the details of signal, and the low-frequency components manifest the approximations of signal. The low-frequency component can be used to reveal the features and trends of ECG signals. In the present application therefore, the high-frequency components of signal are removed. The AECG often overlaps with the low-frequency components of noises, especially the motion artefacts and interferences due to baseline wandering. Because the features that are used to classify rhythm are extracted from low-frequency components of ECG signals, low-frequency interferences have serious repercussions on the performance of the diagnostic algorithm. In practise, a high-pass filter with a cut-off frequency of less than 0.1 Hz simply eliminates very low frequency components of ECG signal, but cannot sufficiently reduce the influences of baseline wandering and motion artefacts . The solutions used in the present invention to reduce low-frequency noise in our algorithm are twofold:

    differential series D(t) are adopted to locate QRS complexes and estimate heart rate;
    piecewise segments of RC(t) series are used to extract morphological features.

Differential operation effectively removes the interference of baseline drift of ECG series, but distorts the morphologies of the ECG signals. The differential series D(t) have a stable baseline reference and distinctive magnitude of amplitudes of electrical , potentials in QRS length, shown in Figure 31 b. Once these signals are de-noise filtered, smoothed and amplified, the reconstruction signal RC(t) is cleaner and has a better SNR than R(t), as shown when comparing the raw signal R(t) in Figure 31 a with that of the filtered and reconstructed signal in Figure 31 c. The piecewise segment in RC(t) has a length of 200ms and the onset of the segment is determined by each QRS position obtained in the D(t) series. The influences of low-frequency noise can thus be effectively reduced in a segment with a length of only 200 ms.

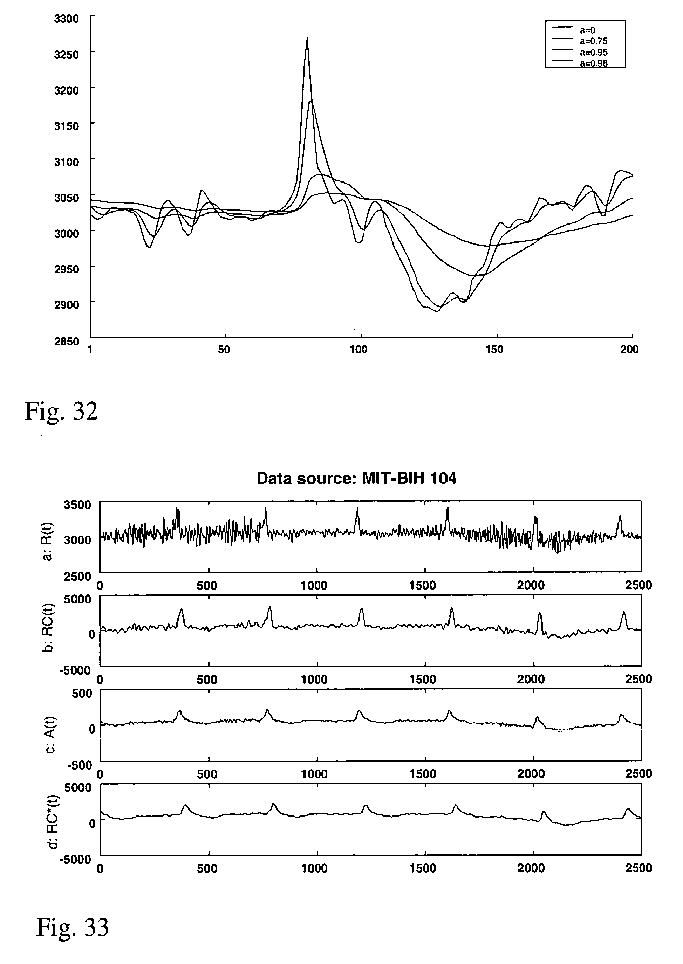

Conventional filter operations of ECG series, e.g. notch filter, low-pass filter, and high-pass filter, effectively remove or reduce most interference. However, motion artefacts, being both irregular in their occurrence and morphological attributes, can not be eliminated by these kinds of filters, see for example in Figure 33a, and 33b. These artefacts can cause significant problems in QRS detection when QRS-like artefacts are encountered, with the result that it is necessary to provide a method for eliminating motion artefacts in order to guarantee the accuracy of diagnosis. One of the solutions to eliminate motion artefacts is wavelet analysis. It removes artefacts by decomposing and then recomposing ambulatory signals. Multi-order wavelet transformation can effectively decompose high-and low-frequency components of ECG signals. However, the high-orders of wavelet transformation will cause serious waveform distortion, and bring huge computational complexity as well as huge resource consumption. Consequently, wavelet analysis is obviously not fit for a real-time embedded AED system. In the method of the present invention, an adaptive filter (AT) is used to reduce motion artefacts. The resultant signal series, named A(t) or Aecg(t), are generated by performing AT operation for the original ECG series R(t). The expression of AT is

$$\begin{cases} Aecg(0)=R(0) \\ Aecg(t)=\alpha * Aecg(t-1)+(1-\alpha)R(t) \end{cases} \quad 0 < \alpha < 1, \ t=1\cdots N \ ,$$

where $\alpha$ is the balance coefficient of AT, which is an important factor in influencing the performance of AT. If the value of $\alpha$ is increased, the previous estimated value Aecg(t-1) then gives greater proportionality in the estimation of Aecg(t). The result of this is that the obtained A(t) is stable and does not reveal changes in the ECG signals. In contrast, if the value of $\alpha$ is reduced, the current original value R(t) will then have greater proportionality, and the obtained A(t) will be more dynamic and adaptive to the changes in ECG signals. Thus, the value of $\alpha$ is carefully selected to make A(t) both stable and adaptive to the changes of R(t). The value of $\alpha$ is relative to the sample frequency of ECG signals. In the STAR system of the present invention, the sample frequency is set to 500 Hz to obtain good signal resolution. The waveforms of the QRS complex with varying adaptive filter coefficient $\alpha$ are shown in Figure 32. The coarse solid line represents the original complex R(t). If $\alpha < 0.5$, the Aecg(t) series has a curve that almost matches the original R(t), and so at this coefficient value AT does not reduce motion artefacts. The thin solid line represents the QRS complex with $\alpha$ =0.75, which has an approximate waveform with R(t). The dashed line represents the QRS complex with $\alpha$ =0.98, in which the motion artefacts are removed by AT, but the waveform is excessively smoothed and thus insensitive to the changes of ECG. The dash-dot line represents the QRS complex with $\alpha$ = 0.95, in which not only motion artefacts are removed by AT, but also the changes of complex can be monitored. One can thus see from the above that the value of the adaptive filter coefficient $\alpha$ can be chosen to be comprised between about 0.5 to about 0.98, but is preferably chosen to comprise a value of about 0.75 to about 0.95, and more preferably is selected to be 0.95.

A comparison of various filter techniques and adaptive filtering is shown in Figure 32, which illustrates an ECG signal series with different filters. Signal (a) is the original ECG signal which is polluted by noise. Signal (b) represents the reconstructed series RC(t) mentioned above. The filters adopted in the "FILTER" operation include a notch filter, a low-pass filter and a high-pass filter. Figure 32b clearly shows that the RC(t) signal still contains interference generally caused by baseline drift and motion artefacts. Signal (c) of Figure 32 is the adaptive filter signal Aecg(t) when the original signal R(t) is directly filtered by AT, and which has markedly better signal quality than RC(t). Signal (d) is the reconstructed signal RC*(t) based on the adaptive filter signal Aecg(t). In contrast to initially reconstructed signal RC(t), signal RC*(t) shows a much improved signal quality and better signal to noise ration (SNR), in which motion artefacts are effectively eliminated. Another advantage of the AT used in the present invention is that it is simple and compact, and therefore enables AT to be implemented by VLSI or directly integrated into an embedded system.

QRS detection is a crucial component of the AED system. In the present method of the invention, QRS detection is the basic foundation stone to the later feature extraction module, the accuracy of the QRS detection having a direct bearing on performance of the feature extraction method. Over the past twenty years or so, much research has been directed to the reliability of QRS detection from ambulatory ECG signals. Some algorithms use information from a frequency domain or a wavelet description of the signals, while others make use of the information derived directly from the ECG signals' time domain description. In order to reduce the overhead of the ECG signal analysis method, the procedure of the preset invention detects QRS complexes directly from ambulatory ECG time domain signals, as this results in lower computational costs than detection of a frequency domain or wavelet analysis. In the present invention, the applicants have come up with a new class of optimum QRS detector which efficiently cope with noise, artefacts and variability of ECG morphology by exploiting a self-adaptive threshold method, hereafter abbreviated to SAT, and a particular state transition recognition procedure, hereafter abbreviated to STR. The SAT method is used to estimate the peaks of ECG sub-segments and the means of contextual thresholds, thereby allowing for effective evaluation to obtain the optimum thresholds in segment space. The STR procedure traces the changes of waveform of signal series and identifies QRS complexes based on the optimum thresholds and the particular rules of state transition.

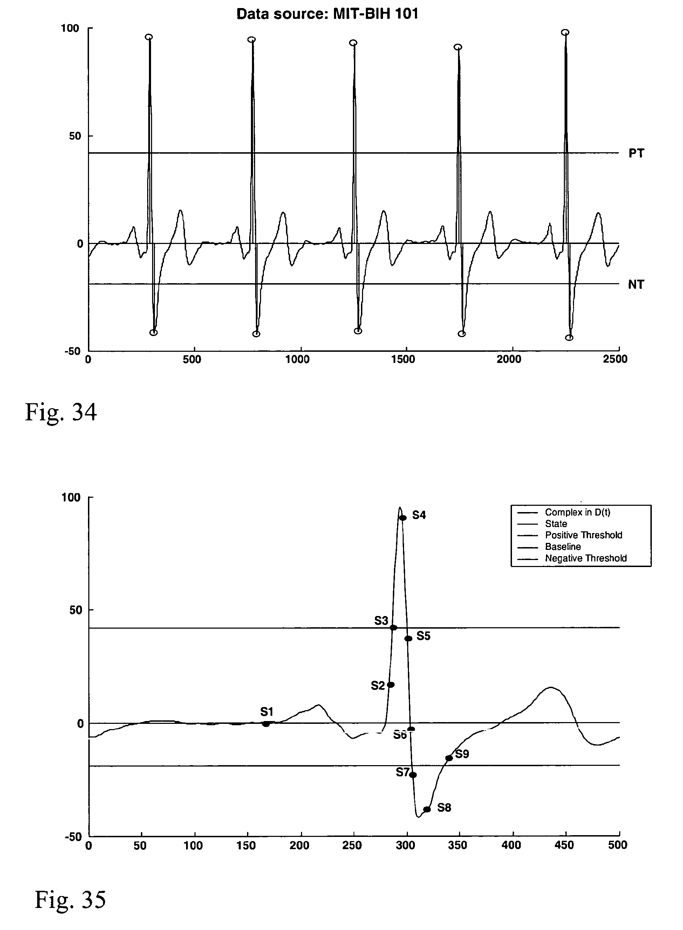

A short-term redundant data, for example a segment of 5 seconds of ECG data, is important in QRS detection. Firstly, the short-term segment enables the contextual correlative analysis of complexes, and reduces the interferences of baseline drift. In view of the low-frequency nature of baseline drift, a short-term segment has fewer disturbances caused by baseline wandering than an equivalent long-term signal. The redundant data enable the QRS detector to identify the current QRS complex by comparing with the preceding and following QRS complexes. This property is very useful when removing motion artefacts. Furthermore, in view of the unpredictability and variability of network quality, appropriate data redundancy is one important element for the data retransmission mechanism and reliable network communication. Having due regard to algorithm performance, network reliability and resource costs, the segment length was defined in the present invention as 5 seconds. Since in the STAR device according to the present invention the sampling frequency of is 500 Hz, any given 5s segment will have 2500 sample points. This data segment is forms a diagnostic unit of the QRS detector, and is called a Diagnostic Segment Window, hereafter abbreviated to DSW. As is apparent from the baseline stability shown in Figure 34, the differential series D(t), which represents the changes of the series A(t), is used to locate QRS complexes.

The waveform of the QRS complex in AECG presents rapid fluctuations and high amplitudes of electrical potential, these features being correctly represented by the differential series D(t). The signals of the QRS complex have higher absolute amplitudes compared to the signals of other phases in a cardiac cycle of D(t) series, see Figure 34. The schema of QRS detection is to carry out a search for the optimum pair-peak for each QRS complex, i.e. the positive and negative peaks of a cardiac cycle. In a DSW, there are generally multiple pair-peaks because several heart beats will occur over a period of five seconds. These pair-peaks make up a pair-peak group in a DSW. Based on the pair-threshold obtained from the pair-peak group of a DSW, the STR procedure described hereinafter is then able to locate the QRS complexes. The absolute amplitude of each peak is generally greater than the associated absolute threshold in D(t). Furthermore, because the offset of location between the series D(t) and A(t) is a constant, we can thus obtain the positions of QRS complexes in A(t) by locating the complexes of D(t).

The SAT method aims to determine the optimum pair-threshold, which is estimated from two elements: the mean of the pair-peak group, both positive and negative, of a DSW and the pair-threshold of the previous DSW. The pair-threshold results from the means of the negative and positive pair-peaks group of a DSW. In order to accurately estimate these pair-peaks, the diagnostic segment window is divided into 5 sub-segments each having a period of one second. It is based on the fact that the average heart rate of a healthy adult is 60 bmp to 100bmp, thus each sub-segment usually contains one heart beat. Since the differential signals of the QRS complex have the maximum absolute amplitudes in a cardiac cycle, a pair-peak can thus represent a QRS complex in the sub-segment and then be used to estimate the thresholds. Furthermore, the shorter the length of the sub-segment, the less interference of baseline drift the sub-segment it will show. A sub-segment with a length of one second can thus be regarded as a stationary series. In order to improve the reliability of the pair-threshold, the SAT method is used to handle some abnormal case scenarii. In general, these abnormal peaks are caused by uncontrollable noise, particularly interference due to motion artefacts, which are typically representative of transient spikes. This class of artefacts, occurring randomly and transiently, has more acute changes of waveform and higher amplitudes than normal QRS complexes. In this case, the pair-peak obtained in a sub-segment is an artefact which has very high amplitude. In a case where the heart beat of patient is less than 60 bmp, it is possible that no heart beat will be present in a given sub-segment. In this case, the SAT method will extract a pseudo pair-peak at this sub-segment. This pseudo pair-peak thus has very low amplitudes, which are close to the value of the baseline. The abnormal pair-peaks can be removed from the pair-peaks group by comparing their amplitudes with others before estimating the mean amplitudes. Because of the regularity of ECG rhythms and the correlation of ECG signals, the previous pair-threshold is used to evaluate and adapt the current pair-threshold. For example, where the electrode pad has completely detached from the person under surveillance, signal amplitudes will change suddenly and then come back to the baseline potential, and consequently the estimated pair-threshold will be significantly different to the previous pair-threshold. Thus, the correlation evaluation procedure of the present invention is used to manage abnormal cases by analysing the differences in thresholds. The pair-threshold, defined as T, is expressed as $T = \rho * M$, where M represents the mean of the pair-peak group of a DSW, and $\rho$ is the threshold coefficient. The threshold coefficient $\rho$ is thus an important factor that influences accuracy of the estimation procedure. Empirical studies by the applicants have led to the selection of a value of 0.45 as the optimal threshold coefficient. The estimation steps of the pair-threshold are described below :

Detect and record five pair-peaks from the relative sub-segments in current DSW;
Calculate the relative mean of positive and negative peaks, and then exclude the peaks that are too far from the means;
Repeat step 2 for the remaining pair-peaks;
Verify current pair-threshold with the previous estimated pair-threshold

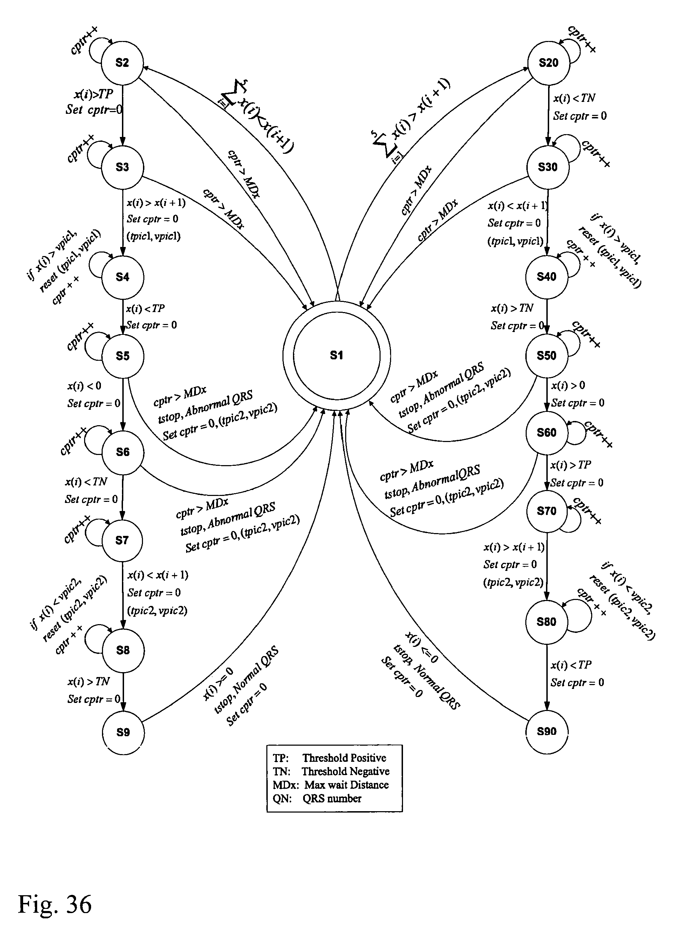

With regard to the morphological properties of a QRS complex in a D(t) series, the complexes are categorized into two groups: positive and negative. The different states are thus defined to outline the phases of a QRS complex in D(t). Figure 36 shows the positive states of a QRS, S2 to S9, and the correspondingly identified S20 to S29 represent negative states. An adaptive and self-corrected procedure, named STR (State Transition Recognition), has been developed by the applicants to automatically track the changes of signal series, to correct error detection and to record detected complexes. The state transitions are based on three basic reference lines: baseline, positive threshold and negative threshold. The state transition diagram, shown in Figure 36, illustrates the transition rules of QRS complex. A data structure named ECG_COMPLEX has been defined by the applicants to record the information of a QRS complex, as illustrated below. It includes the type of QRS complex and the positions of key points, i.e. onset point, peaks and end point. The STR procedure will generate a QRS complex result list, in which each member is a detected QRS complex.

### ECG_COMPLEX

```
Struct ECG_COMPLEX
{
int tstart;
int tpic1;
int vpic1;
int tpic2;
int vpic2;
int tstop;
int etype;
int pattern[MLQRS];
}
```

The objective of a feature extraction procedure is to obtain the measurements of features information from a AECG. The ECG features, including statistical features and morphological features, are used to classify heart rhythms and interpret cardiac arrhythmias. Since the QRS detection procedure according to the invention has located the QRS complexes, the statistical features, i.e. heart rate or RR interval, can be obtained by calculating the time distance between two consecutive complexes. The main problem of the QRS extraction procedure is thus to extract quantified morphological features from the complex. The present invention provides a Geometric Analysis Method, hereafter abbreviated to GAM, to simulate and quantify morphological features.

GAM extracts morphological features from an ambulatory ECG de-noised series RC(t). Due to the constant offset distance between RC(t) and the differential series D(t), the positions of complexes in RC(t) can be determined according to the complexes detected in D(t). GAM analyses the morphology of each QRS complex in a sub-segment of 300ms, which is equivalent to 150 samples in STAR device of the present invention, and which sub-segment begins from the onset point of the complex. There are two advantages to building a complex sub-segment: the first is to reduce interference from noise, especially baseline drift; the second is to exclude the influence of the preceding and folowing complexes. The period of 300ms is an optimum length which is longer than the duration of the QRS, but shorter than the duration of the occurrence of the next complex. Hence, the artefacts detected in the QRS detection procedure can be removed by analysing the duration of the QRS and the RR length.

The baseline and fiducial peaks are the foundation of GAM. The onset point of a QRS complex is supposed to the zero electrical baseline point as it represents the onset position of the changes in QRS detection procedure. GAM provides a policy to detect changes in waveform and to estimate the baseline point and fiducial peaks. The policy is composed of the following three phases. The changes in a complex are caused by either ventricular depolarization or artefacts. Changes that are too small or rapid and that are generally caused by noise, i.e. muscle tremor or motion artefacts, can be removed from records by comparing their amplitude and their length of time with a normal QRS complex. The evaluation steps of the baseline and peaks of complex are shown as follows, where Tmax and Tmin indicate the positions of maximum and minimum peaks, and Vmax and Vmin are the amplitudes of these two peaks :

Trace the changes of sub-segment data and record peaks information. The peak estimation criterion is a continuous three incremental samples and subsequently a continuous three decremental samples, and vice versa.

Estimate Tmax and Tmin peaks and calculate the slope of each peak. The R wave fiducial point will be detected from the two peaks. The slope reflects the gradient of the complex.

Remove the peaks which have too small/large slopes and too dull/steep changes of amplitudes between two consecutive peaks .These kind of peaks are generally caused by noise.

Repea steps 2 and 3 in the remaining peaks set and then obtain more accurate Tmax and Tmin.

By studying the morphologies of the V1 to V6 ECG leads and the morphologies of ventricular arrhythmias, QRS complexes of cardiac rhythms can be categorized into 6 classes:

Positive single-peak;
Negative single-peak;
Positive multi-peaks;
Negative multi-peaks;
Positive-negative peaks; and
Negative-positive peaks.

GAM provides a sub-procedure to do this classification task based on the set of peaks obtained in the previous phase. The operations of the classification sub-procedure are shown below:

Verify the Tmax and Tmin peaks. The criterion is that the Tmax and Tmin peaks must have rather high absolute amplitude and absolute slope compared to those of other peaks and the Tmax and Tmin peaks of a previous QRS complex are also used to verify these two peaks. The aim of this step is to exclude the pseudo-Tmax/Tmin caused by noise.

Pre-estimate the type of complex (rhythm). The criterion is to compare the absolute amplitudes of Tmax and Tmin. If Vmax >> Vmin, set class 1; else if Vmax << Vmin, set class 2; else, continue to next step.

Estimate the type of complex (rhythm). For class 1 or 2, search for the second peak that has similar peak characteristics to the Tmax (1) or Tmin (2). If one is found, adjust class to class 3 or class 4, the second peak is then marked as Tmax2 or Tmin2. For other situations, if Tmax >= Tmin, set class to class 5, else set class to class 6. In classes 3 to 6, there are two peaks to indicate the peak features of a complex. The two values named Tpeak1 and Tpeak2 are thus defined to mark the positions of two fiducial peaks, where Tpeak1 is set to less than Tpeak2. The R peak is one of two fiducial peaks, which is estimated according to the class of morphology and the contextual complexes.

The evaluation criteria for the peaks of Tpeak1 and Tpeak2 are shown below. If necessary, the baseline position of the current QRS complex can be adjusted according to Tpeak1 and its previous peak. The characteristic VR (Amplitude of R wave) of a complex can thus be determined in this phase.

## Estimation criteria for Tpeak1 and Tpeak2 :

```
if class 1 or 2  then
            TR=Tmax/Tmin;
            Tpeak1=TR, Tpeak2=TR;
else if class 3  then
            TR=Tmax;
            Tpeak1=MIN(Tmax, Tmax2), Tpeak2= MAX(Tmax, Tmax2);
else if class 4  then
            TR=Tmin;
            Tpeak1=MIN(Tmin, Tmin2), Tpeak2= MAX(Tmin, Tmin2);
else if class 5  then
            if (Vmax>Vmin) TR=Tmax;  else TR= Tmin;
            Tpeak1=Tmax, Tpeak2=Tmin;
else if class 6  then
            if (Vmax>Vmin) TR=Tmax;  else TR= Tmin;
            Tpeak1=Tmin, Tpeak2=Tmax.
end if
```

The triangular-like or triangular-component morphology of a QRS complex makes it possible to extract features by a geometric analysis method (GAM). This method consumes relatively little resources, which means that it is ideally suited to embedded real-time diagnostic applications. Another advantage of GAM is its prediction ability, making it equally suited to estimating key sample points of a QRS complex under noisy conditions.

The key sample points of a complex include the R wave peak, the end point of Q wave (Qt) and the onset point of S wave ($S_i$). The R wave peak is obtained from Tpeak1 or Tpeak2 as described above and comprises a single peak or a multitude of peaks. The measurement and the detection phases of the Qt and $S_i$ points are illustrated as follows:

Define two-level thresholds for left and right sides of the R wave in a QRS complex respectively. The left two-level thresholds are defined as LH=1/4*Vpeak1,
LL=3/4*Vpeak1; correspondingly, the right-level thresholds are RH=1/4*Vpeak2, RL=3/4*Vpeak2.
Calculate the intersections between threshold values and the complex signal. Generally, there are 4 intersections which are used to estimate the approach lines of left and right side waveforms. The slopes of two approach lines represent two other characteristics of the complex: SP (Positive Slope) and SN (Negative Slope).
Obtain the length of the QRS (LQRS), which is the distance between two intersections on the baseline and two approach lines.

Once these steps have been completed, the morphological features of complex have been extracted from a ambulatory ECG. However, due to the non-stationary and easily disturbed features of a ambulatory ECG, the results are preferably evaluated further before interpretation of cardiac arrhythmia.
The most obvious error of ECG signals is incorrect electrode placement on the chest of a person under surveillance. Since QRS complexes vary with the position of the electrodes, each ECG lead views the heart at a unique angle in a unique electrode placement. The method of the invention interprets cardiac arrhythmia on the basis of the morphological features of each ECG lead, and so correct electrode placement is important in order to be able to accurately classify rhythms. In one preferred embodiment of the invention, and before starting the diagnostic algorithm, the system should indicate to the person under surveillance to attach the electrodes in the correct positions.
A further problem to be solved is the accurate and effective detection and extraction of a QRS complex from an ECG series contaminated by artefacts. The applicants of the present invention have therefore developed QRS correction methods. Since all detected complexes are supposed to be caused by true heart beats, GAM extracts the features from all detected complexes. Because of the irregular morphology and irregular occurrence of motion artefacts, the feature values of artefacts have obvious differences with those of a normal QRS complex, and are determinant for the recognition of an artefact signal. The method of the invention comprises a two-level correction mechanism to remove artefacts and correct QRS complexes.

The first level of correction, also named pre-correction, also forms the basis of second level correction. It aims to detect artefacts from single-lead ECG signals. The decision factors include the signal quality ratio, the feature vector of the current complex and its class-complex. As mentioned in the description of the QRS extraction procedure, each complex is classified into a class-complex in terms of its morphological features. Consequently, the feature values of the normal complex are used to calculate the feature mean of a class-complex. By comparing the feature values of the current complex and its class-complex template, the correction procedure can recognize most of the irregular complexes. The method can also help evaluate the standard QRS type of an ECG lead. As the changes of a QRS complex under clean conditions are regular, the number of peaks of a QRS complex, obtained in the feature extraction procedure, is regarded as a quality index that is used to quantify signal quality of the QRS complex. The result of the peak numbers of current complex divided by the peak numbers of normal QRS complex indicates the quality ratio of the complex. This quality ratio is one of the factors for level-1 QRS correction. If the quality ratio is much greater than 1, AECG signals are severly polluted by noise. The criteria for level-1 correction are presented and explained below.

## Level 1 correction criteria

```
State = 0; % Artifact Flag
if (RRcur< 0.8* RRnormal ) then
Slopecur = Max(SP, SN); % max slope of current complex
Dev = Slopecur / Slopenormal ; % Slope ratio
if (Dev<0.3 || Dev >3 ) then
State = 2;
end if
Peakcur = Max(|Vpeak1|, |Vpeak2|); % max peak of current complex
Dev = Peakcur / Peaknormal ; % Peak ratio
if (Dev<0.3 || Dev >3 ) then
State = 2;
end if
if (LQRScur>100) then % the duration of QRS
State = 2;
end if
if (State==2 && Numpeak>10) then % noise situation
State = 1;




end if
if (Numpeak > 12) then % strong noise situation
State = 1;
end if
if (RRcur< 0.3* RRnormal) then % impossible RR
State =1;
end if
end if
if (Numpeak > 8 && (Tpeak1 > 50 || Tpeak2 > 100)) then %inaccurate baseline
State = 1;
end if
```

An artifact flag is attached to detected artefacts for subsequent correction. An artefact is an irregular occurrence, and thus it also causes an irregular RR which may have a similar RR length to tachycardia arrhythmia. The correction procedure of the invention therefore handles cases of short RR. The artifact flag is initialized to 0 at the beginning of level-1 correction.

The morphological features of the current complex will be compared with the feature mean of the associated class-complex. If an abnormal complex is found, the artefact flag is set to the transient state 2 because tachycardia arrhythmia may be causing similar abnormal morphologies.

When the artefact flag is set to state 2 and the signals are obtained under noisy conditions, as determined by the quality ratio, the artefact flag is set to 1. If the signals are obtained under high noise conditions or RR is too short, the artefact flag is also set to 1.
If the signals are obtained under a weak noise condition and the R peak position has a large offset in the sub-segment, the artefact flag is also set to 1. This case may be caused by the inaccurate baseline of complex.

The level-1 correction phase sometimes leads to incorrect determinations when considering various uncertain factors, particularly interference caused by noise. For example, in some cases, ventricular arrhythmias have similar characteristics to artefacts, therefore leading to a potentially incorrect decision relating to the rhythm class. The applicants thus take two other criteria into account in order to correct these potential errors: contextual decision and multi-lead fusion. In order to simplify the method and improve efficiency, these two methods are combined together to discriminate artefacts from ventricular arrhythmia. These two methods are also used to diagnose and interpret cardiac arrhythmia.

Level-2 correction adopts contextual analysis to aid in decision-making. A missed or additional QRS complex can bring transient irregular change of the RR interval time and consequently heart rate will change acutely. This sudden change is possibly reflected by some forms of heart diseases, but most of such changes result from various technical and physiological disturbances. Clinical experience has revealed that sudden change of the RR interval has a contextual correlation and that a normal RR interval is regular and periodic. Hence, the preceding and subsequent RR interval values and the average RR interval values can be used to estimate the current QRS complex.

Multi-lead monitoring provides the ability to continuously view cardiac behaviour from multiple angles. The greater the number of ECG leads used, the greater the information obtained for cardiac arrhythmia analysis. Multi-lead ECG signal analysis therefore improves the reliability of the method of the invention. When meeting the abnormal rhythm or irregular RR interval, data segments of other leads in the same DSW will be processed to assist in making a correct decision. In fact, the influence of a patient's motion is generally limited to a certain electrode and causes artefacts only in one of the leads; the other leads generally remain unaffected. This means that, even if one lead's signal is completely destroyed by noise, the diagnostic algorithm will still continue to work.

There are two potentially abnormal situations that require level-2 correction: the first is a pseudo-complex caused by QRS-like artefacts; the second is a loss of the true QRS complex. The level-2 QRS correction procedure is shown and explained below.

## Level 2 correction

```
if (Additional Interval)  then
Estimate Pnxt = Ppre+ RRavg;
Call CHECK(Pnxt) to verify complex at Pnxt position(multi-leads);
 if (Find a complex)  then
Call DETECT(Pcur), analysis methods contain
comparing with pattern Template, calculating signal
pollution ratio of current window, analyzing contextual
complexes.



 else
Call CHECK(Pcur);
 if (Find a complex)  then
Call DETECT(Pcur);
 end if
end if
else if (Missing interval)  then
Estimate Pnxt = Ppre+ RRavg;
 while (Pnxt < Pcur)  do
Call CHECK(Pnxt);
 if (Find a complex)  then
Call ADD(Pcur);
 end if
Pnxt = Pnxt+ RRavg;
 end while
end if
```

The correction process can be described as follows :

Decide the class of the abnormal situation by evaluating the RR interval of the current complex;
If RR interval is too short, the complex is perhaps an additional pseudo-complex, so then

(a) estimate the position of expected complex resulting from the position of previous complex and the average RR interval: Pnxt = Ppre+ Rravg;
(b) search the expected complex at the position of Pnxt in multi-lead ECG signals. If one is found, then the current complex is more likely to be an artefact, call Delete(Pcur) to exclude from current complex; else if one is not found, then the current complex is either arrhythmia or artefact, need to further Check(Pcur) before calling Delete(Pcur). If RR interval is too long, it is possible that a complex interval is missing, then

(a) estimate Pnxt as the method in step2-(a)
(b) if Pnxt < Pcur, search the expected complex at the position of Pnxt in multi-lead ECG signals. If one is found, then call Add(Pnxt) to supply a complex, and then continue step 3-b) until Pnxt > Pcur.

Based on the feature values extracted from ECG signals by the feature detection procedure, a self-diagnosis expert system has been implemented to classify heart rhythms and interpret cardiac arrhythmias. The diagnostic rules of the expert system rely on the rules of experience gained from self-learning capability of the system and cardiological definitions. Cardiac arrhythmias originated from the atria, conduction system and ventricles, and show up as a change in heart rate and rhythm. Cardiac arrhythmias that are associated with the symptoms of heart diseases can be classified and recognized, and their diagnostic criteria obtained by studying the characteristics of ECG signals, empirical clinical experience.

An arrhythmia is a change in the normal rate or control of the heart's contractions. Cardiac arrhythmias are classified into two classes: those originating in the atria and those originating in the ventricle. Furthermore, in view of the change in heart rate, cardiac arrhythmias have two modes: bradycardia and tachycardia. Not all cardiac arrhythmias are harmful or associated with heart disease. For example, sinus arrhythmias, which may cause anxiety during an episode, are for the majority of people completely harmless. However, studies have confirmed that some cardiac arrhythmias, especially ventricular arrhythmias, are associated with heart disease and require medical treatment to keep the heartbeat regular. The various categories of cardiac arrhythmia are described below:

Sinus arrhythmias are the cyclic changes of sinus rhythm under the influence of the SinoAtrial (SA) node. Common in children and often found in adults. This kind of cardiac arrhythmia is generally benign and includes following classes: Sinus Bradycardia (SB) Sinus Tachycardia (ST)
Sick sinus syndrome: The sinus node does not fire its signals properly, so that the heart rate slows down. The identifiable classes include: Dropped beats (DB), Sinus pause (SP), and Sinus arrest (SA)
Heart blocks: interference with the conduction process in the AV node (atrioventricular node), bundle of His (or atrioventricular bundle), or the bundle branches can lead to this phenomenon. Heart blocks have similar features of heart rate to Sick sinus syndrome, the identification of which depends on the recognition of the P wave.
Premature beats: ectopic (out of place) beats occur when a focus stimulates an early contraction of the myocardium. Where the focus is in the atria, atrial ectopic beats occur with normal ventricular operation. Where the focus is in the ventricular myocardium, broad QRS complex beats occur. There are thus two identifiable classes shown: Premature atrial contractions (PAC), Premature ventricular complexes (PVC) or extrasystole ventricular (ESV)
Supraventricular arrhythmias are caused by non standard conduction and origin within the atria. The main classes are: Supraventricular tachycardia (SVT) or Atrial tachycardia (AT), Atrial Flutter and Atrial Fibrillation
Ventricular arrhythmias are caused by an irregular depolarization and repolarization pathway in the ventricle. In ventricular rhythms, the depolarization wave spreads through the ventricles irregularly and therefore takes a slower pathway. The QRS complex is thus wide and abnormal. Repolarization pathways are also different, causing the T wave to have an unusual morphology. The classes of ventricular arrhythmias are: Ventricular tachycardia (VT) and Ventricular Fibrillation (VF)

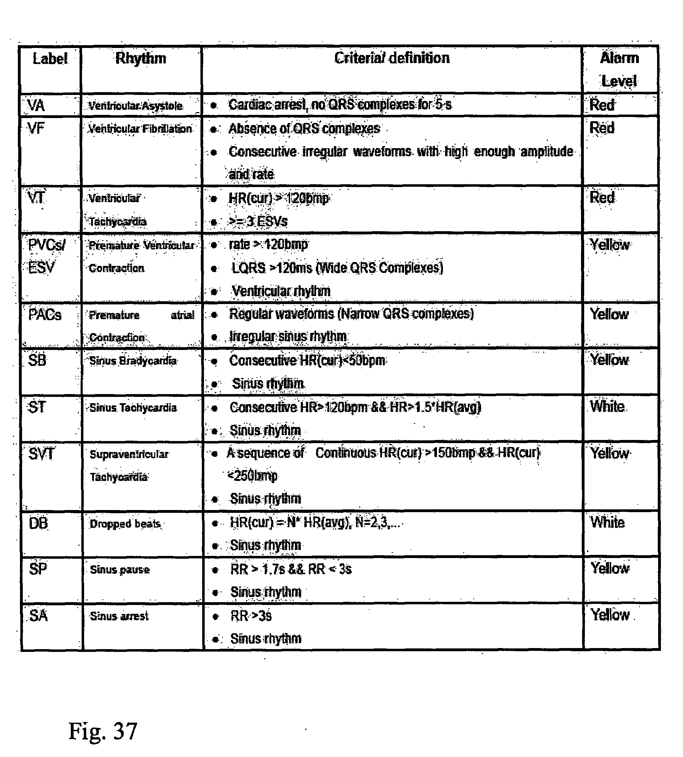

Heart rhythms can be classified into two categories: if originating in the atria, normally from the SA node, the QRS complex is a sinus rhythm; but if originating in the ventricles, the QRS complex is a ventricular rhythm which is generally an aberrant beat. This implementation of classification is based on the extracted feature values obtained in the extraction task described above. The diagnostic criteria are based on the expert knowledge and rules obtained by studying a training set. In the system according to the present invention, cardiac arrhythmias are graded into three alarm levels : red, yellow and white, to identify the priority of each arrhythmia alarm with the gravity of its corresponding heart disease. The diagnostic criteria and alarm levels of cardiac arrhythmias are defined in Figure 37.

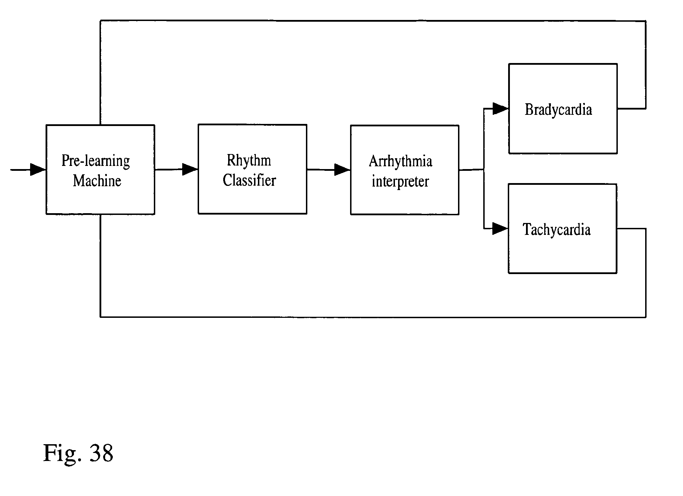

The determination system of the present is composed of three phases: a pre-learning machine, a rhythm classifier and an arrhythmia interpreter. Figure 38 shows the diagnostic module. Based on the clinical cardiological experience and the results of the training procedure, the pre-learning machine builds and estimates the diagnostic rules for every lead ECG signal of a person under surveillance. The rhythm classifier classifies each detected heart rhythm into one of two categories : sinus rhythm or ventricular rhythm. Depending on the rhythm type and the diagnostic rules, the cardiac arrhythmia interpreter correlates cardiac arrhythmia with the symptoms of the corresponding heart disease. The interpretation is preferably carried out separately for the two types of cardiac arrhythmias, i.e. bradycardia and tachycardia, although it is within the skilled persons remit to envisage a single interpretation step for both types.

On initialisation of the system according to the invention, there is a procedure to correct electrode placement which displays 10 seconds of ECG signals. This 10 second data stream is also used to determine the diagnostic rules used by the pre-learning mechanism of the system. In this phase, the initial cardiac status, rhythm type, statistical and morphological features can be obtained. The rhythm that occurs the most frequently during the 10 second period will be considered as the reference rhythm of the patient, and its feature values are extracted to form the analytical rules. Furthermore, the feature values are also used to describe the normal rhythm and thus to build the rhythm template, i.e. class-complex, for the subsequent rhythm classification task. Additionally, the analytical results will be fed back to the automatic learning machine, which are used to adjust the coefficients of the analytical rules. Unlike resting ECG, long-term ambulatory ECG has continuous tiny changes due to the influences of the exterior environment and the patient's physical status. These tiny changes are generally normal and so the coefficients of the analytical rules are updated regularly to correspond to said changes.

The rhythm classifier recognizes two classes of rhythms: sinus rhythm that has a narrow and regular QRS complex and ventricular rhythm that has a broad and normally irregular QRS complex. The rhythm classifier adopts a "template matching" method to classify a QRS complex based on the feature values obtained from the feature extraction module. The signal features, such as the RR interval, the QRS length (LQRS), the left- and right-sides slopes of R wave (SP & SN), the amplitude of R wave (VR), the complex type and the two peaks (Tpeakl & Tpeak2), describes the details of a heart rhythm. They can thus be used to recognize a rhythm by comparing with the features of the rhythm template. The template matching method of the rhythm classifier is based on the feature comparison. Since the features were obtained from a normal rhythm, i.e. the rhythm template in the pre-learning machine, the classification process compares the features of the current rhythm to the rhythm template. The classification equation can be expressed as:

$$F_{estimate} = \sum_{i \in N} \beta * (1 - |\frac{W_i - T_i}{T_i}|)$$

Where, the $F_{estimate}$ is the classification factor which is used to classify the heart rhythm, N indicates the number of the features. The $\beta_i$ is the weighting coefficient of the feature i, which satisfies the equation $\sum_{i \in N} \beta_i = 1$. This coefficient indicates the contributions of the feature i to the rhythm classification. $W_i$ is the value of the feature i of current rhythm, and $T_i$ is the value of the feature i of rhythm template. The expression $1 - |(W_i - T_i)/T_i|$ indicates the deviation ratio of the $W_i$ comparing to $T_i$.

Current rhythm can be recognized and interpreted by the arrhythmia interpreter of the invention on the basis of the analytical rules. Firstly, the arrhythmia interpreter classifies the rhythms into two categories - bradycardia and tachycardia - by comparing current heart rhythm (HR) with the mean HR of the rhythm template. Then, two interpretation procedures are called respectively to process the two classes of cardiac arrhythmias which are shown below :

## Bradycardia interpretation

```
if (RRcur>5s) then
VA; return;
else if (LABEL(last) ==tachy && RRnxt ~=RRavg) then
NORMAL; return;
else if (ventricular rhythm) then
VE; return; %ventricular Escape complex
else if (sinus rhythm) then %delay
if (RRcur==2*RRavg) then
DB; return;
else if (RRcur>3s) then
SA; return;
else if (RRcur>1.7s && RRcur<3s) then
SP; return;
end if
SB;
end if
```

## Tachycardia interpretation

```
if (sinus rhythm) then
if (HR>120bmp) then
PAC;
```

```
if (continuous PAC>=3) then
if (HR >150bmp &&HR <250bmp) then
STV; return;
 else
ST; return;
 end if
end if
end if
if (RRcur+RRnxt ~=2*RRavg) then
C-PAC; return; %compensated
 end if
if (RRcur+RRnxt ~=Rravg && RRcur~=RRnxt) then
D-PAC; return; %double
 end if
if (PATTERN(N,V,N,V,N,V,...)) then
B-PAC; return; %Bigeminy
 end if
else if (ventricular rhythm) then
if (HR>120bmp) then
PVC;
 if (continuous PVC>=3) then
VT; return;
 end if
if (PATTERN(N,V,N,V,N,V,...) ) then
B-PVC; return; %Bigeminy
 end if
if (PATTERN(N,N,V,N,N,V,...)) then
T-PVC; return; %Trigeminy
 end if
end if
else if (no QRS complex) then
if (EXIST(VT) && HR > 250bmp) then
VT;
 end if
end if
```

In accordance with the different cardiac arrhythmias event detected, a multi-level alarm system is defined. An emergency message will be sent to the user to report the occurrence of serious cardiac arrhythmias events. The STAR system according to the present invention provides a multi-level alarm system, which will be described in further detail hereafter. In the arrhythmia interpreter, heart rhythms are identified and classified into two basic categories: normal cardiac rhythms and cardiac arrhythmias, whereby the cardiac arrhythmias can be further divided into two classes: known cardiac arrhythmias and the unknown cardiac arrhythmias. The known cardiac arrhythmias are normally the cardiac tachycardias events which are caused by serious heart diseases, including PVC, VT, VF, SVT and PAC, etc. Known cardiac arrhythmias have distinctive QRS complexes and rapid heart rates which are interpreted accurately. Unknown cardiac arrhythmias are usually related to cardiac bradycardia events, which are caused by less serious or benign heart diseases. Known cardiac arrhythmias have regular heart rhythms, but slow heart rates, rendering identification unreliable when one uses only the heart rate to analyse any given event.

The signal analysis system of the present invention comprises two independent modules, i.e. a feature extraction unit and a cardiac arrhythmia classification unit. Performance evaluation of such a system thus contains two aspects: one is the evaluation of the QRS detector; the second is the evaluation of the rhythm classifier. The evaluation of the QRS detector demonstrates the efficiency of locating the QRS complex. The evaluation of the rhythm classifier reveals the accuracy of the cardiac arrhythmia interpretation. The applicants have tested the performance of both units of their analytical system. The diagnostic algorithm was assessed with respect to two ECG databases: the MIT-BIH arrhythmia database and the CSD database, an internal database of data generated from true life clinical patient study by the applicants. The MIT-BIH Arrhythmia Database contains 48 half-hour excerpts of two-channel ambulatory ECG recordings, obtained from 47 subjects studied by the BIH Arrhythmia Laboratory in Boston between 1975 and 1979. Twenty-four distributing records have been freely published for performance comparison purposes. Each signal file is slightly over 30 minutes in length and contains two signals sampled at 360 Hz. The header files include information about the leads used, the patient's age, sex, and medication. The reference annotation files include beat, rhythm, and signal quality annotations. The CSD database records were obtained from 10 subjects at the Gabriel Montpied University Hospital in Clermont-Ferrand, France, using the systems, methods and devices of the present invention. The CSD records have lengths of from 7 to 18 minutes, and each signal file contains four-channel ambulatory signals sampled at 500 Hz. Each beat was manually annotated by the cardiologist to generate the reference annotation files. The CSD ECG records are recorded in the same format as the MIT-BIH Arrhythmia Database.

The locations or marks of the annotated beats in the reference annotation files have differing time positions. For example, the marks of the MIT-BIH database are mostly near to the onset of the Q wave, and the marks of the CSD database are close to the peak of the R wave. Suppappola and Sun determined a valid detection interval for evaluation of the performance of feature extraction. The interval can be modified by the user, whose borders include the onset and end of the QRS complex. The marks of the tested QRS complexes within the valid interval are considered true positive (TP) with respect to the annotations. Any additional marks in the valid interval are treated as false positive (FP). Detection marks outside of the intervals are classified as false negative (FN). The sensitivity (Se) and specificity (Sp) of the feature extraction procedure are thus normally computed by :

$$Se = 1 - \frac{FN}{TP + FN} = \frac{TP}{TP + FN}$$

Dotsinsky and Stoyanov defined four performance parameters to assess the efficiency of the QRS detector: TP (true positive), FP (false positive), FN (false negative) and shifted SH beats. The SH beat, representing a single fault, is assigned to a couple of adjacent erroneously detected FP and FN, if they are preceded and followed by TP beats. SH beats are in fact non-existing, like the FN beats, both are summed for the computation of Se. The formula below, as an extension of the original formula above, is used to calculate Se :

$$Se = \frac{TP}{TP + FN + SH}$$

Sp is calculated by formula :

$$Se = 1 - \frac{FP}{TP + FP} = \frac{FP}{TP + FP}$$

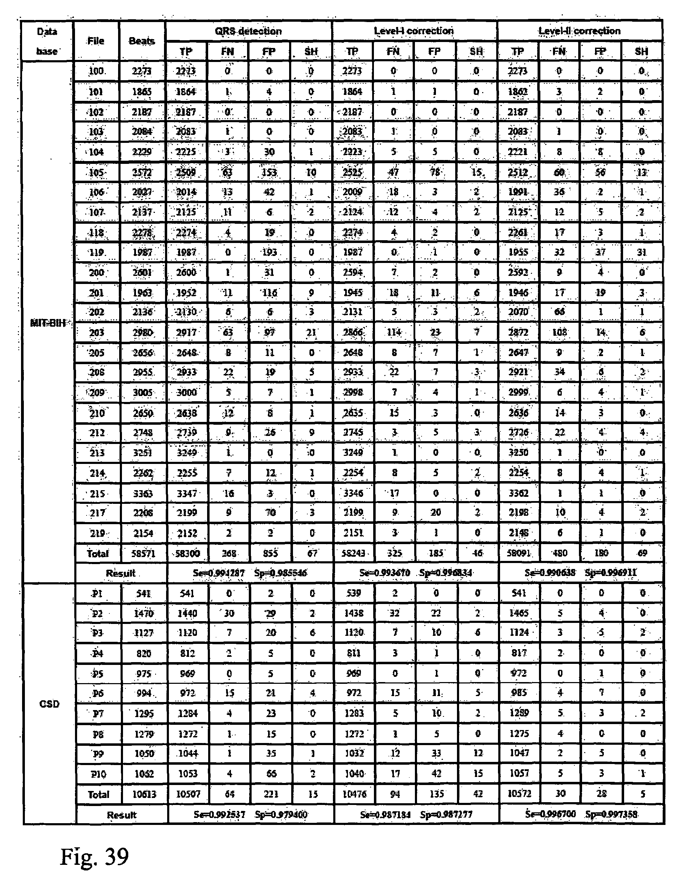

The QRS detector of the invention has two basic functions: QRS detection and Error correction. In order to compare the efficiency of feature extraction, the performance evaluation of the QRS detector is performed in three phases: QRS detection, level-1 correction and level-2 correction. The results of Se and Sp are obtained by evaluating the algorithm on the records of the MIT-BIH and CSD databases, and are shown in Figure 39. In the method according to the invention, the QRS detection procedure provides the ability to locate the QRS complex by analyzing the features of time-domain ECG signals. A sensitivity of 99.43% and a specificity of 98.55% were obtained by the QRS detection procedure of the invention in the detection of the 24 shared records of MIT-BIH database. A sensitivity of 99.25% and a specificity of 97.94% were obtained in the CSD records. The two-level correction procedure is adopted to remove additional artefacts and to add missed QRS complexes. The level-I correction is operated on the single channel ECG signals, the criterion of which is based on the feature comparison between the current QRS complex and the standard QRS complex or template. The results of the evaluation in the level-1 procedure are a sensitivity of 99.37% and a specificity of 99.68% for the MIT database and a sensitivity of 98.72% and a specificity of 98.73% for the CSD database. It is clearl that level-1 correction improves the specificity of algorithm, but slightly lowers the sensitivity. Level-2 correction is performed on multi-lead ECG signals, the criterion of which is based on the contextual relationship of ECG signals and the fusion of multi-lead signals. Under normal conditions, more signals generally means better performance. The multi-lead analysis had the advantages of removing additional artefacts and of supplementing missed beats by comparing the detected beats of different leads' signals. For example, the sensitivity and specificity of the level-2 correction on the CSD database increased respectively to 99.67% and 99.74%. In the diagnostic segment window of the feature extraction module, the detection algorithm selects an ECG lead with the best signal quality, and fusion of two-lead ECG signals does not need to be performed.

The rhythm classifier classifies heart rhythms into two categories : non-alarm and alarm-rhythms. The alarm-rhythms defined in the method of the present invention are cardiac tachycardias, i.e. PAC, PVC, SVT, VT, and VF. They represent normally serious heart diseases which need to be reported immediately. The non-alarm rhythms include the normal rhythms and some benign or less serious cardiac arrhythmias, such as cardiac bradycardia. The four parameters used to assess the method's overall performance are :

a true positive (TP) is a serious cardiac arrhythmia that has been correctly classified as an alarm- rhythm;
a false positive (FP) is an organized normal rhythm that has been incorrectly classified as an alarm- rhythm;
a true negative (TN) is any normal or less serious rhythm that has been correctly classified as a non-alarm rhythm;
a false negative (FN) is a serious cardiac arrhythmia that has been incorrectly classified as a non-alarm rhythm.

The sensitivity (Se) of the device is the number of true positive abnormal rhythms, expressed as a percentage of the total number of abnormal rhythms. Se is calculated using the formula given above. The specificity (Sp, also named positive predictive accuracy) is the number of organized rhythms that have been correctly classified as normal rhythms by the method, and is expressed as a percentage of the total number of normal rhythms which is calculated by the following formula:

$$Sp = \frac{TN}{FP+TN}$$

The values of Se and Sp were obtained by applying the rhythm classification method on recordings of the MIT-BIH and CSD database, and are given in Figure 39. The algorithm performance of the classification of non-alarm and alarm rhythms was tested. A sensitivity of 90.94% and a specificity of 95.50% were obtained by the classification procedure on MIT-BIH database. A sensitivity of 95.60% and a specificity of 99.54% were obtained with the CSD clinical diagnosis data. The results of the performance evaluation for the QRS detector and rhythm classifier of the present invention show that they satisfy the demands of a AED application. The QRS detection algorithm adopts fractional and geometric methods to locate the QRS complex from AECG signals. The signal preprocessing and conditioning procedure, adopting adaptive filter and bandpass filter, remove or reduce various interferences caused by physical and technical factors. Additionally, the most serious noises, i.e. motion artefacts, have effectively been eradicated by the adaptive filter. Accuracy of detection has been improved using a two-level correction procedure to remove additional artefacts and supplement missing beats. Compared to other known algorithms, the overall results of the detection method of the invention show high sensitivity and specificity. The method of the invention has very low computational consumption and fast detection ability, thus meeting the requirements for real-time diagnostics. Compared to other known methods of classification, the overall results of the classification method of the present invention show excellent performance. Furthermore, the classification method of the invention can directly use the experience gained clinically by cardiologists, thereby reducing the complexity of rules for training the systel and thus improving accuracy of classification. The method of the invention is also capable a very broad spectrum cardiaci arrhythmias, in fact, far more than any other system to date.

In accordance with still yet another object of the present invention, the inventions provides a continuous remote real-time detection, monitoring and control system for cardiac arrhythmia, known as STAR. The main aim of the STAR system is to provide a rapid detection and analytical method for the high-risk population of cardiac arrhythmia sufferers to prevent sudden death. It can also be used for long-term heart surveillance for a population who has a history of heart disease, or to do periodic heart examinations for a healthy population of individuals.

The function of STAR system according to the invention is as follows :

A wireless ECG sensor acquires and analyses ECG signals in real-time from a person under surveillance. When a cardiac abnormal event is detected, an alarm message is sent to the local access station via local wireless technologies, such as WiFi and Bluetooth. If the patient stays indoors and the fixed wire network infrastructures are available, the local access station then transmits the alarm message, for example a ECG signals sequence, to a remote system via indoor network mediums, such as a cable modem, ADSL, ISDN, Ethernet, etc; if the patient is outdoors, the local access station transmit an alarm message, for example, a SMS or an email, to a remote system via wide wireless technologies, such as GSM, GPRS, UMTS and satellite, etc. Using such a system, a cardiologists can examine the abnormal events and respond within a very short period. In an alternative embodiment, the wireless sensor is capable of detecting, analyzing and classifying the signal and informing the person under surveillance, for example via a screen located on the sensor, of the actions to be taken when an abnormal event occurs, thus avoiding a diagnosis by a trained clinician. In such a situation, the wireless sensor is a self-contained unit that undertakes all of the roles that previously required a clinician's input.

The system according to the invention thus demonstrates an effective combination of WSN technologies, network communication technologies and AED technologies. The system of the invention is very flexibile and extensible. It can be made to work in various application environments, not just in arrhythmia detection, and can be configured to work in various operating modes. The real-time and reliable network communication that is located within the device is one of the foundations of the system. This was achieved by focusing on two particular aspects : signal compression techniques, that are used to reduce data transmission; an application layer protocol which provides a guaranteed real-time ECG signals transmission service.

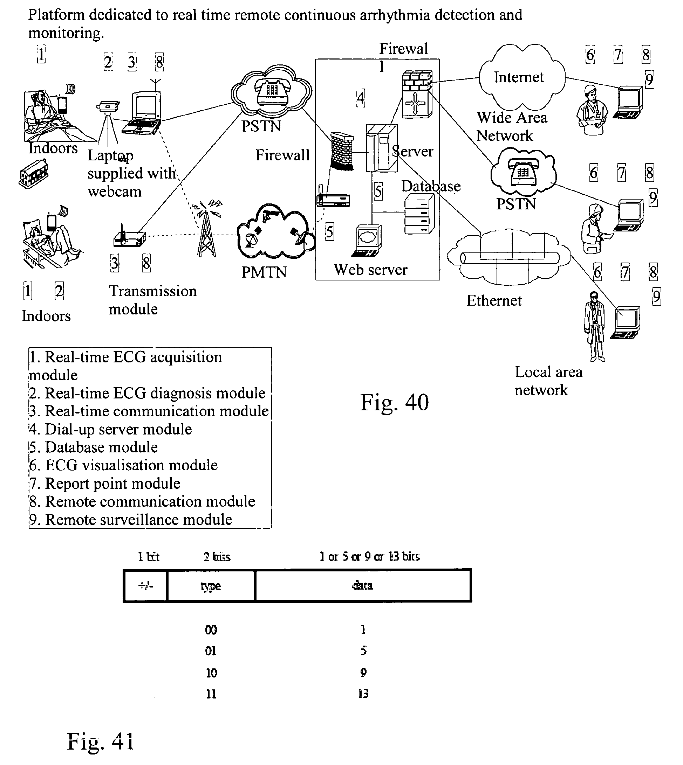

The system of the invention integrates advanced wireless technology and distributed embedded real-time intelligent sensors that communicate over the Internet. In one preferred embodiment, it consists of two subsystems: a local subsystem and a remote subsystem, which contains four main configurable components. The local subsystem has a wireless ECG sensor (WES) and a local access server, and the remote system includes a remote access server and a remote surveillance system capable of diagnosis and visualization. Figure 40 shows the function modules of the system according to the present invention. According to desired application of the system, functional modules can be mounted on one of four system components.

The system according to the invention uses an energy-efficient compact wireless ECG sensor hereafter abbreviated to WES. The WES conforms to the basic characteristics of a wireless sensor device: tiny resources, tiny power-consumption and associated tiny cost. In addition, the WES of the invention comprises an embedded distributed real-time fault tolerant microkernel, dedicated hardware, and a specific TCP/IP protocol stack. An example of such a wireless ECG sensor typically has dimensions of about 70 mm by 100 mm, and incorporates in a preferred embodiment a Texas Instruments ultra-low-power MSP430 microcontroller. The sensor, when not equipped with a wireless adapter, consumes only 10mA of power. The key features of one preferred WES are:

Gain: 1000 CMMR(min): 120dB
Bandwidth: 0.05Hz to 125Hz
Programmable sample frequency more than 500Hz
Analogue to digital converter (ADC): 12 bits
Leakage current: 10$\mu$A.

The WES according to the invention makes it possible to capture 4 leads of ECG signals sampled at 500Hz in real-time, the sample frequency being reprogrammable. The sample signals are sent to a local server over a wireless medium such as WiFi or Bluetooth. In offline mode, ECG signals can be stored in an exterior or on-board flash memory card or chip. The store duration of the ECG signals depends on the capacity of the flash memory card, the sample frequency and the number of ECG leads. The sample frequency and the lead number can be configurable by the user. For example, a 128Mb flash memory card can store 24 hours continuous data from 4-lead ECG signals sampled at 500Hz. In this way, the WES works as a Holter or an RTEST.

The local access server may be implemented on a standard PC, a PDA, a mobile phone or a dedicated network access medium. It provides two classes of network connection services: connection with the WES via a local wireless medium e.g. WiFi or Bluetooth, and connection with the remote system via existing network media, such as cable modem, ADSL, ISDN, GPRS, satellite, etc... In view of the difference of network media, local servers, and network speeds caused by network traffic, the system of the invention provides adaptable communication techniques to guarantee a reliable data transmission service between the local server and the remote server. Furthermore, in order to provide real-time data transmission, it is important to minimize the amount of data transmission to reduce network traffics over the low-bandwidth network links. To this end, a lossless ECG signal compression algorithm is implemented in the system. If the local access server is implemented on a high-performance machine, such as a PC, the ECG diagnostic module can be migrated from the WES onto the local server and the cost of the WES will be reduced even further. In addition, it can be advantageous to obtain video information from the person under surveillance, as an auxiliary means for online diagnosis. Consequently, a webcam can be installed in, or connected, to a local server to provide images as desired.

The remote access server provides the capability of network connection between the local access server and the remote surveillance server. Depending on the type of network, it can support two types of access servers: a PPP server and a WAP server. The PPP server establishes connections between persons under surveillance and the clinician via a Public Switched Telephone Network (PSTN). It supports several network bandwidths: 56Kbps (cable modem), 512Kbps or more (ADSL). The WAP server establishes connections via a wide wireless network. It can tune automatically to different wireless bandwidths: 9,6Kbps (GSM) or 115Kbps (GPRS). In addition, if the persons under surveillance and the clinicians are in the local area, e.g. in a hospital or clinic, and share local network infrastructures, e.g. high-speed LAN, or they utilize the global network connections provided by commercial ISPs (Internet Service Providers), this component is optional and can be removed from the system architecture. Several function modules can be loaded onto the remote access server: a database service and a web service. The medical history records of a person under surveillance are important for analysis of the condition, for example, in this case, arrhythmia. Medical records in multimedia formats are stored in the patient database system on the remote access server, which include ECG signal sequences, cardiologists' diagnostic reports, patients' video, voice and individual profile information, etc. The Web service provides the capacity of Internet access to the patients' database system via a simple Web browser.

The remote surveillance server has an interactive visualization Graphical User Interface, with which the cardiologist can diagnose cardiac arrhythmia events in real-time and respond to alarm messages by monitoring the ECG signal sequence and the patients' images. This server supports multi-patient surveillances, and one patient on-line diagnosis by a cardiologist at a time. The four-lead ECG signals and related diagnostic results obtained from the the AED method of signal processing are displayed on the screen and stored into data files in the WFDB format. Diagnostic reports can be built automatically and printed with the ECG signal sequence and related statistical results. When the number of persons under surveillance is relatively low, e.g. when in use in a small clinic, the cardiologist at the remote surveillance server can connect directly with the patient at the local access server via the Internet. In this way, the database service and Web service can be implemented directly in the remote surveillance server.

The system according to the invention is very flexible and extensible. It can be applied to a number of configurations, and therefore can operate in various modes. The scenarios of the application modes are:

ECG Surveillance Automaton: the WES is configured to work as an absolute ECG surveillance automaton to carry out long-term monitoring of a patients' heart status. It displays the ECG signals on a LCD screen, and records the ECG signals and related diagnostic results into the external or internal flash memory card. The WES can integrate an optional ECG diagnostic chip and thus offer the possibility of real-time ECG diagnosis.

Clinical Surveillance: In this mode, the patients and cardiologists are in the local area and share the local network infrastructure. Where a limited number of users is envisaged, the ECG signals acquired by the WES are sent directly to the remote surveillance server via local wireless media. Where large-scale application is envisaged, the WES first sends the ECG signals to a local access server in which the ECG signals are rapidly processed and diagnosed, and then the compressed ECG signals and diagnostic results are sent to a remote surveillance server via a local high speed network. As a high-speed LAN infrastructure is used in this mode, the remote surveillance server supports the multi-user multi-lead continuous real-time ECG signal monitoring. In this case, the diagnostic method can be implemented on a remote surveillance server or a local access server. This is similar to the telemetry cardiac arrhythmia detection system commercialized by Agilent Technologies.

Remote Surveillance: In this mode, the patient is at a location remote from the hospital or clinic and may even be at home, at the office or travelling. Via the existent network infrastructures, In such an example, the platform establishes connections between patients and cardiologists via existing network infrastructures. For example, if the patient is at home or at the office, the local access server can be implemented on a PC, and thus connected to a remote PPP server via a dial-up connection; if the patient is travelling, the local access server can be implemented on a mobile phone, and connected to a remote WAP server via wide wireless technologies. This mode contains all of the system components and supports four operation modes.

The operation mode of the system according to the invention is configurable and modifiable by the doctor in view of the patient's heart status, hardware devices and network bandwidths. Each operation mode is defined as an alarm level with a unique message format. The system offers four operation modes. It should be noted that the cardiologist can change the operation mode in order to tune to the evolution of the patient's heart status:

Level-I: Real-time continuous ECG signals. Level-I is the highest alarm level. It is applied when the cardiologist needs to diagnose online a patient or the patient needs to be monitored continuously in real-time. In this mode, the continuous ECG signals acquired from the WES and the diagnostic results processed by the AED methods of the invention are displayed in real-time. By observing the ECG signals, the pre-diagnostic information and the patient's optional video or digital image information, the cardiologist can build a final diagnostic report. This operation mode is normally not suitable for simultaneously processing a large number of patients due to the limitations of network bandwidth, system resources and human resources.

Level-II: ECG signal sequence. The WES is configured to send a sequence of ECG signals, for example, the pre and the post five-second ECG signals of a cardiac arrhythmia event, to the remote system when a cardiac arrhythmia event defined by the cardiologist is detected. As the system stores and sends only the limited data of a cardiac arrhythmia event, it avoids generating huge data files and massive network traffic caused by long-term ECG monitoring. This operation mode is suitable for long-term multi-patient cardiac arrhythmia event surveillance for the lower-risk heart disease population.

Level-III: textual or SMS emergency message. In this mode, only a short textual emergency message is sent to the cardiologist when a cardiac arrhythmia event is detected. Depending on the seriousness of the symptom, the cardiologist decides to intervene immediately or to respond later. This mode may be operated on any access medium, i.e. fixed wire or wireless.

Level-IV: diagnostic report e-mail. this is the lowest-level operation mode. The local server will periodically send an e-mail to the cardiologist to report the patient's status. The time interval of dispatching e-mail is defined by the cardiologist. This operation mode is suitable for the heart care of a healthy population.

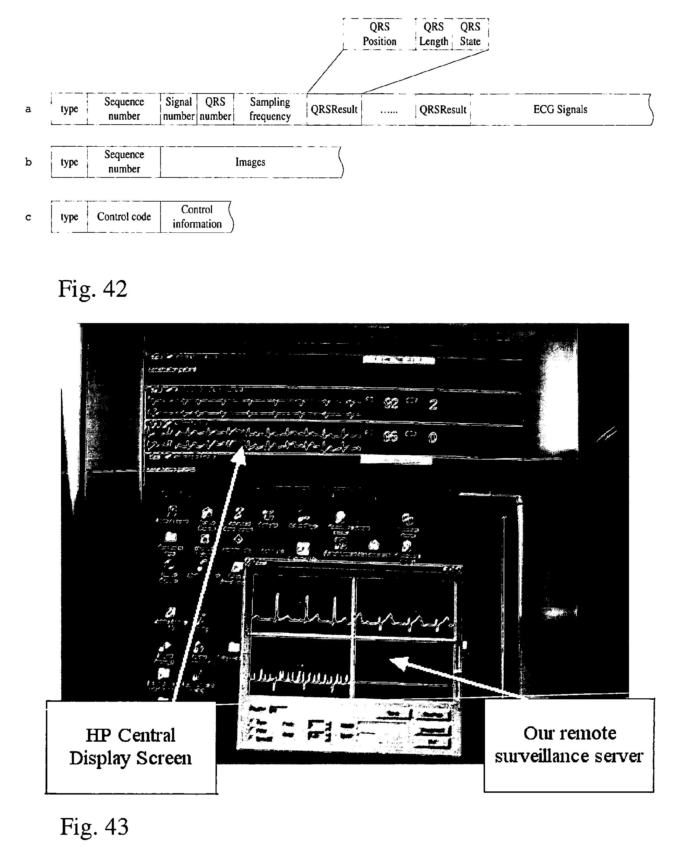

The most effective methods for reducing network traffic lie in decreasing the amount of signal transmission and in compression of the ECG signals. The 500 Hz sampling frequency of the WES offers high quality ECG signals for ECG diagnosis, but also generates huge network traffic for data transmission. For example, a 4-lead ECG signals at 500Hz sample frequency and 12 bits resolution ADC, will occupy 20,000 bytes over a period of 5 seconds (4 leads * 500Hz * 5s * 2 bytes). In order to support real-time transmission, the network speed must be more than 32Kbps (20,000 * 8bits / 5s) when the system works in the Level-I operation mode. This speed is obviously unfit for low bandwidth networks. Although the ECG signals with high sampling frequency are considered important to guarantee the accuracy of the ECG diagnostic algorithm, ECG signals with a relatively low sampling rate, often 128 Hz, are sufficient for the purposes of ECG observation in the remote visualization system. The sampling frequency of 128 Hz is typically used for an ECG signal. Hence, the sampling frequency of ECG signals acquired by a local server can be de-multiplied to reduce the network traffic. Furthermore, waveform features show that ECG signals have huge data redundancies. Hence, a good data compression algorithm can also reduce network traffic. The applicants have devised a lossless compression algorithm that deals with such problems : only the difference value between two consecutive samples is sent to the remote server. Since the electrical amplitudes of ECG signals in cardiac expansion are almost equal to the zero isoelectric value, the difference between the samples during cardiac expansion is almost equal to zero. The sample compression format is illustrated in Figure 41, where :

the "+/-" field indicates the positive/negative property of the difference data: 1 - positive and 0 - negative;
the "type" field indicates the type of data length and the "data" field stores the difference data.

Statistical results show that this compression algorithm has a 50% to 60% compression ratio. For example, with 4-lead ECG signals at 500Hz sample frequency and 12 bits resolution ADC, when applying the demultiply frequency technique to 125 Hz and the signal compression algorithm according to the invention, it is possible to reduce 87.5% to 90% of all network traffic.

In order to support real-time data transmission, the UDP protocol is used to deliver ECG signals. However, the UDP protocol does not offer a guaranteed datagram delivery service. Hence, a reliable data transmission mechanism must be implemented in the application layer. As the speed of the network communication system fluctuates and is affected by the network traffic and other interference factors, an application layer communication protocol dedicated to the system of the present invention was implemented, and is responsible for a real-time and reliable data transmission service over various hardware and network environments.

The data unit of transfer between two machines in the system according to the invention is called a STAR frame. STAR frames are used to establish/terminate STAR connections, to deliver data, e.g. ECG signals or images, and to configure operation modes or other system parameters. Each frame is divided into two parts, a STAR frame header followed by data. Figure 42 shows the STAR frame format. The type field is used to identify the type of STAR frame. Three types of STAR frames are defined in a STAR system according to the invention : a value of 0x01 indicates a system control frame; a value of 0x02 indicates an ECG signal frame; and a value of 0x03 indicates an image frame. Each type of STAR frame has a unique system priority identified by the type value (1 to 3, from high to low). Figure 42 (a) shows the format of the ECG signal frame. Each ECG frame has a unique identifier specified by the sequence number. Every time a local server sends an ECG frame, the sequence number will automatically increment by one for the next frame. It is therefore possible to calculate the surveillance time of heart monitoring from this value. The value of the signal number field indicates the channel number of ECG signals. In the present invention, the preferred number of channels is four, but of course, it is possible to have more or less than four channels. The value of the QRS number fields represents the number of QRS complexes detected by the AED method in this ECG frame. The default sampling frequency of the WES is 500 Hz, which is identified in the sampling frequency field. This value is alterable using a frequency demultiplier operation. As the signal compression algorithm changes the size of the original ECG frame, the frequency value is useful for determining the length of the uncompressed original frame on the remote server. The following area of the ECG frame stores the diagnostic results of the AED method. It is a QRS structure queue, the QRS member number of which is indicated in the QRS number field. Each QRS member, named QRSResult, consists of three elements: QRS position, QRS length and QRS state. The QRS position element indicates the onset position of a QRS in the uncompressed ECG frame. The heart rate of each beat can be calculated based on the positions of two consecutive QRSs. The QRS length represents the time interval of a QRS. The QRS state shows the type of heart rhythm and its related heart status classified by the AED method. The ECG signals field stores the compressed ECG signals. In the preferred STAR system of the invention, each ECG frame contains a five-second ECG signal. Figure 42 (b) shows the format of the image frame. The Sequence number field is unused for an image frame. The image field contains an image in the jpeg format, although other well known image formats could be used, for example, the portable network graphic format also known as the PNG format. Figure 42(c) shows the format of the system control information frame. The value of the control code field indicates the type of control code. The related control information is stored in this field.

Three kinds of UDP connections are established in the STAR communication system of the present invention. They are responsibile for the system control (udp_CMD), ECG signal transmission (udp_SIG) and images transmission (udp_IMG).

The STAR control frames of the present invention are responsible for system remote configuration, patient online / offline notification, and ECG frames retransmission management. The control frame is transferred via the udp_ CMD connection. The system of the invention defines nine types of control frames defined as follows:

REO_Connect
ACK_Connect
REO_Terminate
ACK_Terminate
REQ_Configure
ACK_Configure
REQ_Restra
ACK_5Frames
ACK_Image

The control frames with the code of REQ_Connect and ACK_Connect are responsible to for establishing connection between patients and cardiologists. While the control frames with the code of REQ_Terminate and ACK_ Terminate are responsible for terminating this same connection. Both the patients and the cardiologists have a right to open and close the udp_CMD connection voluntarily. The control frames with the code of REQ_Configure and ACK_ Configure are responsible for the system configuration. The other control frames with codes REQ_Restra, ACK_5Frames and ACK_Image will be discussed hereafter.

The system according to the invention guarantees a reliable ECG signal delivery service by providing a signal retransmission mechanism. Two ECG frame queues are defined respectively for the local server peer, Hold_Queue, and the remote surveillance server peer, Wait_Queue. The standard length of the two queues is five frames. The retransmission mechanism is described below:

## Remote Surveillance Peer Retransmission

```
If (an ECG frame is recieved )
If (Seq_cur == Seq_exp)
Insert current frame to wait_queue; The position of current frame in wait_queue is decided by its Seq_cur;
Seq_exp++;
else if (Seq_cur < Seq_exp)
drop it;
Send Control Frame REQ_Restra to the local server;
else if (Seq_cur > Seq_exp)
Add current frame to the tail of wait_queue ;
end
if (continuous Five frame is received)
```

```
Send Control Frame ACK_5Frames to the local server;
end
end
```

## Local Acess Peer Retransmission

```
If (ACK_5Frames frame is received)
Release the ECG frames whose Seq_fra <= Seq_ack from the
hold_queue;
else if (REQ_Restra frame is received )
Retransmit an ECG frame of Seq_req to the remote surveillance server;
Release the ECG frames whose Seq_fra < Seq_req from the hold_queue;
end
if (an ECG frame is ready)
Send an ECG frame of Seq_cur to remote surveillance server;
Seq_cur++;
end
```

When an ECG frame with the sequence number k is lost during network transmission, the remote surveillance server sends a retransmission request frame with a control code of REQ_Restra, the local server responds to this request by retransmitting the ECG frame k. When five consecutive ECG frames are received by the remote surveillance server, a control frame with the ACK_5Frames code is sent to the local server to inform the local server to release the backup ECG frames in Hold_Queue.

As mentioned above, the system according to the invention has three types of data frame. Each data frame type has a unique transmission priority. The system control frame has the highest priority in the system. Furthermore, because the ECG signals are more important than the images for the purposes of diagnosis, the ECG frame has higher priority than the image frame. In order to guarantee a real-time ECG signal transmission service, a data competition mechanism is implemented in the system, shown below :

## Competition Mechanism : Remote Surveillance Peer

```
If (an Image frame is received)
If (the length of wait_queue >= 5) //25 seconds delays
Send a Control frame ACK_Image to the local server ;
Set the Image_Enable = TRUE;
else
Set the Image_Enable = FALSE;
end
end
if ( an ECG frame is received)
If (the length of wait_queue > 5 && Image_Enable == FALSE )
Send a Control frame ACK_Image to the local server ;
```

72

```
Set the Image_Enable = TRUE;
end
end
```

### Competition Mechanism : local access peer

```
If (Image_Enable == TRUE)
Send an Image frame to the remote surveillance server ;
Set the Image_Enable = FALSE;
end
if ( a Control frame ACK_Image is received)
Set the Image_Enable = TRUE;
end
```

When the queue length of Wait_Queue is equal to or greater than five, there are at least 25 seconds of ECG signals that have been reserved on the remote surveillance server. It is therefore acceptable to allow the local server to transmit images. However, when the queue length of Wait_Queue is smaller than five, it means that the network speed has began to fluctuate and network quality has dropped, so the system will stop image transmission to reduce network traffic.

Currently, the STAR system according to the invention and dedicated to real-time remote continuous cardiac arrhythmia detection has been evaluated on about ten patients at the Gabriel Montpied University Hospital hospital in Clermont-Ferrand, France. Diagnostic algorithms have also been evaluated by using the MIT-BIH cardiac arrhythmias database. Insofar as VT and ESV are concerned, the average detection rate is about 95%. It is to be noted that the quality of the ECG signals on our system is significantly better than corresponding HP telemetry systems, as illustrated in Figure 43.

The test platform that corresponds to Figure 43 is configured to operate as a Clinical Surveillance application, and runs in level-1 mode, i.e real-time continuous ECG signals mode. The local access server is a laptop on which the real-time cardiac arrhythmia program is implemented. The remote access server and the remote surveillance server are installed in the same laptop or a standard PC, which connect with the local server via the local high-speed network. The system of the invention supports continuous remote cardiac arrhythmia monitoring and allows the patient to continue with his daily activities, both indoors and outdoors. The results of the tests and evaluations show the system to be fully functional, and is an efficient system for diagnosing cardiac arrhythmia, opening up a new clinical approach to more accurately evaluating a larger number of high-risk patients. Furthermore, it may be used by cardiologists to remotely heart monitor and evaluate the efficacy of drugs or to discuss difficult cardiac pathology cases with other colleagues.

As a variant to the general system described above, the applicants have also designed and implemented an Intelligent Wireless ECG Sensor (IWES) by integrating the cardiac arrhythmia detection algorithm onto a ICAC chip. The ICAC has been evaluated and tested on an FPGA. Thus, the alternatively preferred embodiment of the system platform contains both an IWES and a remote server. With the IWES, when the patient is outdoors, the sensor will automatically disconnect from the remote server. When cardiac arrhythmia events are detected, an ECG signal sequence will be recorded locally on a flash memory card. Only an emergency short message will be sent to a remote server, for example a WAP server, via a mobile phone connection as a SMS text. The emergency message may be defined by the cardiologist according to the physical state of the patient. The IWES will periodically attempt to connect to the remote server, and if the network connection is established, a cardiac arrhythmia report along with all the recorded events is sent periodically.

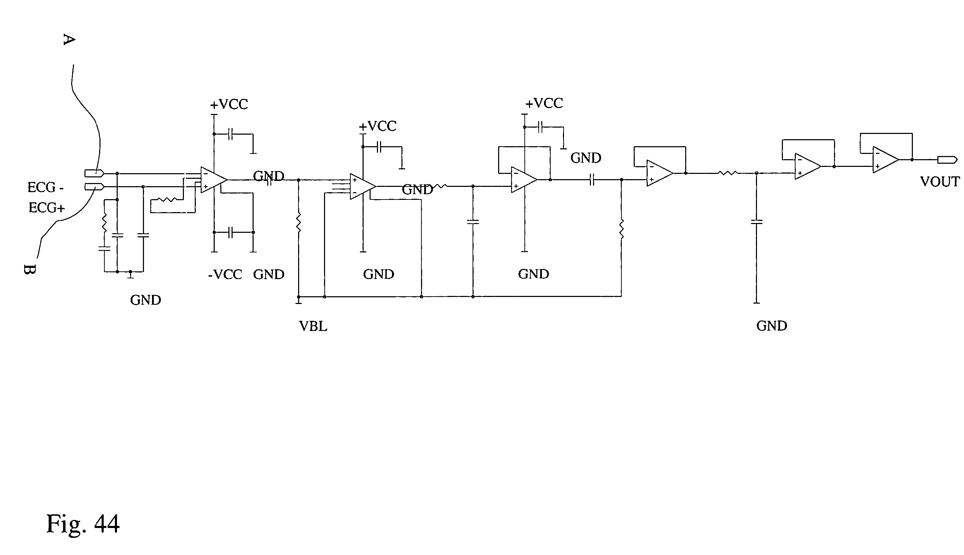

It was mentioned above that the ECG signals that are classified in the system of the present invention are far more stable and regular than those of a corresponding commercial telemetry sensor. An exemplary circuit diagram of the filter and signal conditioning system is shown for the purposes of illustration in Figure 44. One of the reasons for the stability of the ECG signals acquired in the present invention is due to the fact that the ECG signal entry, indicated as A and B on Figure 44, into the filtering circuit is asynchronous. This goes against all current train of thought in this field, because it has always been felt that a synchronized ECG signal entry was required in order to obtain a sufficiently stable signal. Indeed, the present applicants compared the system with synchronous ECG signal entry, but all of the experiments carried out demonstrated a loss in signal stability, with more baseline drift over time, compared to the asynchronous ECG signal entry adopted and used in the device of the present invention.

Although some of the present objects have been identified in relation to software programs and languages, it is perfectly possible for most of the functions to be implemented as hardware, i.e. using VLSI or VHDL techniques that are well known to the skilled person per se. Consequently, any of the components of the system that are currently implemented via software can be readily integrated directly into a chip via the usual lithographic techniques known in the integrated circuit manufacturing industry, such that the chip actually provides for a physical structure engraved in, for example, silicon, representing the various sequences of instructions, tests, comparisons, and operations that the software implementation carries out.

**Claims**

1. Real-time true multi-tasking operating system loaded or engraved on a data support media comprising an executable microkernel capable of addressing a microprocessor for execution of instructions, and comprising a task scheduler, wherein the scheduler deals with two classes of tasks, a periodic task class and a priority task class and wherein :

   - a periodic task has a very short execution time and deterministic response time, and has a highest priority level, preferably a priority level of 10, said periodic task being interruptible but non-pre-emptible;
   - a priority task is interruptible at variant-time intervals, and has a predictable response time, and is also pre-emptible, with a priority level comprised from low to high, preferably from 1 to 9.

2. Real-time operating system according to claim 1, wherein the priority levels range from 0 (lowest) to 10 (highest).

3. Real-time operating system according to claim 1, wherein a priority level of 0 is attributed to a daemon, or an idle task.

4. Real-time operating system according to claim 1, wherein the task scheduler has a two-tier scheduling scheme wherein :

   - periodic tasks are handled on a FIFO (first in, first out) basis;
   - and priority tasks are handled on a "priority-based pre-emptive" basis, wherein any first task is selected preferentially on the basis of a comparison of its priority level with respect to the priority level of any other priority task, a higher priority level of the first task being selected in preference to a lower priority level of an other task.

5. Real-time operating system according to claim 4, wherein priority tasks are always executed before periodic tasks.

6. Real-time operating system according to claim 1, wherein the microkernel also comprises a tuple based messaging system.

7. Real-time operating system according to claim 1, wherein the microkernel also comprises a tuple space dedicated to task communication and synchronization.

8. Real-time operating system according to claim 1, wherein each message associates with a tuple in a shared system and application tuple space.

9. Real-time operating system according to claim 1, comprising a set of primitives that are defined by a Meta language.

10. Real-time operating system according to claim 9, wherein the system primitives consist of the SND() and RCV() primitives.

11. Real-time operating system according to claim 1, wherein "task switching" or "context switching" occurs as follows :

    - determine whether a currently running task should continue to run ; if said currently running task should not continue to run, suspend said current task and goto next step;
    - determine which task should run next;
    - save an environment of the task that was suspended;
    - set up a running environment of an other task that will run next;
    - allow this other task to run.

12. Real-time operating system according to claim 1, wherein a task is initialized to a "sleep" state when it is created.

13. Real-time operating system according to claim 1, wherein the data support media is a semi-conducting material, and the operating system instructions are integrated directly into said semi-conducting material.

14. Real-time operating system according to claim 1, wherein the data support media is volatile or non-volatile, and is preferably chosen from the group consisting of rom, eeprom, ram, optical recording media, cd-rom, dvd-rom, rewritable dvd, and magnetic tape, and magneto-optical storage media.

15. Method of analysis of an ECG signal, comprising :

   - acquiring one or more ECG signals over a period of time;
   - filtering said one or more ECG signals with at least one filter selected from the group consisting of a low pass filter, a high pass filter, and an adaptive filter; and
   - extracting one or more features from said filtered signal.

16. Method according to claim 15, wherein the low pass filter has a cut off of 40 Hz.

17. Method according to claim 15, wherein the high pass filter has a cut off of 0.1 Hz.

18. Method according to claim 15, wherein the acquired signal is subjected to differentiation before filtering.

19. Method according to claim 15, wherein the acquired signal is subjected to reconstruction after filtering.

20. Method according to claim 15, wherein the adaptive filter (AT) is used to reduce motion artefacts, and the filtered signal series, named A(t) or Aecg(t), are generated by performing the AT filter operation on the original ECG series R(t) according to the following expression :

$$\begin{cases} Aecg(0) = R(0) \\ Aecg(t) = \alpha * Aecg(t-1) + (1-\alpha)R(t) \end{cases} \quad 0 < \alpha < 1, \; t = 1 \cdots N \; ,$$

   where $\alpha$ is the balance coefficient of AT and has a value comprised between about 0.5 and 0.98, preferably between about 0.75 and 0.95, and most preferably is equal to 0.95.

21. Method according to claim 15, wherein in step (a), the period of time does not exceed 5 seconds, thereby forming a diagnostic segment window (DSW).

22. Method according to claim 15, further comprising the step of identifying QRS complexes from the filtered signals.

23. Method according to claim 22, wherein the QRS complexes are identified via application of the self-adaptive threshold (SAT) method and the state transition recognition (STR) method.

24. Method according to claim 23, wherein the SAT method comprises determining the pair-threshold by :

   - dividing up a given DSW into 5 sub-segments of 1 second each;
   - detecting and recording five pair-peaks from the relative sub-segments in a current DSW;
   - calculating the relative mean of positive and negative peaks, and then excluding the peaks that are too far from the mean;
   - repeating step (b) for the remaining pair-peaks;
   - verifying the current pair-threshold with the previous estimated pair-threshold.

25. Method according to claim 22, wherein the STR comprises applying the transition rules identified in Figure 26.

26. Method according to claim 15, wherein the feature extraction is effected by applying a geometrical analysis method (GAM), in which the following steps are carried out to evaluate the baseline and peaks of a QRS complex, and where Tmax and Tmin indicate the positions of maximum and minimum peaks, and Vmax and Vmin are the amplitudes of these two peaks :

   - trace the changes of sub-segment data and record peaks information;
   - estimate the Tmax and Tmin peaks and calculate the slope of each peak;

- remove the peaks which have too small / large slopes and too dull / steep changes of amplitudes between two consecutive peaks;
- repeat steps (b) and (c) in the remaining peaks set and then obtain more accurate Tmax and Tmin.

**27.** Method according to claim 26, further comprising classification of the peaks as follows :

- verifying the Tmax and Tmin peaks;
- pre-estimating the type of complex by comparing the absolute amplitudes of Tmax and Tmin;
- if Vmax >> Vmin, set class 1;
- else if Vmax << Vmin, set class 2;
- else, continue to next step;
- estimating the type of complex : for class 1 or 2, search for the second peak that has similar peak characteristics to the Tmax (1) or Tmin (2);
- if one is found, adjust class to class 3 or class 4, the second peak is then marked as Tmax2 or Tmin2;
- for other situations, if Tmax >= Tmin, set class to class 5;
- else set class to class 6.

**28.** Method according to claim 27, wherein the measurement and the detection phases of the Qt and $S_i$ points are determined as follows:

- defining two-level thresholds for left and right sides of the R wave in a QRS complex respectively, where the the left two-level thresholds are defined as LH=1/4*Vpeak1, LL=3/4*Vpeak1, and the right-level thresholds are RH=1/4*Vpeak2, RL=3/4*Vpeak2;
- calculating the intersections between the threshold values and the complex signal;
- obtaining the length of the QRS (LQRS), which is the distance between two intersections on the baseline and two approach lines.

**29.** Method according to claim 15, wherein after the feature extraction step, error correction is carried out.

**30.** Method according to claim 29, wherein the error correction is a level 1 error correction according to the following schema :

```
State = 0; % Artifact Flag

if (RRcur< 0.8* RRnormal ) then

Slopecur = Max(SP, SN); % max slope of current complex

Dev = Slopecur / Slopenormal ; % Slope ratio

if (Dev<0.3 || Dev >3 ) then

State = 2;

end if

Peakcur = Max(|Vpeak1|, |Vpeak2|); % max peak of current complex

Dev = Peakcur / Peaknormal ; % Peak ratio

if (Dev<0.3 || Dev >3 ) then

State = 2;

end if

if (LQRScur>100) then % the duration of QRS

State = 2;
```

```
end if

if (State==2 && Numpeak>10) then % noise situation

State = 1;

end if

if (Numpeak > 12) then % strong noise situation

State = 1;

end if

if (RRcur< 0.3* RRnormal) then % impossible RR

State =1;

end if

end if

if (Numpeak > 8 && (Tpeak1 > 50 || Tpeak2 > 100)) then %inaccurate baseline

State = 1;

end if.
```

31. Method according to claim 29, wherein the error correction is a level-2. correction according to the following schema :

```
if (Additional Interval)  then

Estimate Pnxt = Ppre+ RRavg;

Call CHECK(Pnxt) to verify complex at Pnxt position(multi-leads);

if (Find a complex)  then

Call DETECT(Pcur), analysis methods contain

comparing with pattern Template, calculating signal

pollution ratio of current window, analyzing contextual complexes.

else

Call CHECK(Pcur);

if (Find a complex)  then

Call DETECT(Pcur);
```

```
end if

end if

else if (Missing interval)  then

Estimate Pnxt = Ppre+ RRavg;

 while (Pnxt < Pcur)  do

Call CHECK(Pnxt);

 if (Find a complex)  then

Call ADD(Pcur);

 end if

Pnxt = Pnxt+ RRavg;

 end while

end if
```

**32.** Method according to claim 15, further comprising a classification step after extraction of the features comprising :

- a pre-learning step;
- a rhythm classifier; and
- an arrhythmia interpreter.

**33.** Method according to claim 32 wherein the arrhythmia interpreter distinguishes between bradycardia and tachycardia arrhythmias.

**34.** Method according to claim 33, wherein the test for bradycardia arrhythmias is as follows:

```
if (RRcur>5s)  then

VA;  return;

 else if (LABEL(last) ==tachy && RRnxt ~=RRavg)  then

NORMAL;  return;

 else if (ventricular rhythm)  then

VE;  return; %ventricular Escape complex
```

```
else if (sinus rhythm)  then %delay

if (RRcur==2*RRavg)  then

DB;  return;

else if (RRcur>3s)  then

SA;  return;

else if (RRcur>1.7s && RRcur<3s)  then

SP;  return;

end if

SB;

end if
```

**35.** Method according to claim 33, wherein the test for tachycardia arrhythmias is as follows :

```
  if (sinus rhythm)  then

  if (HR>120bmp)  then

  PAC;

   if (continuous PAC>=3)  then

  if (HR >150bmp &&HR <250bmp)  then

  STV;  return;

   else

  ST;  return;

   end if

  end if

  end if

  if (RRcur+RRnxt ~=2*RRavg)  then

  C-PAC;  return; %compensated

   end if

  if (RRcur+RRnxt ~=Rravg && RRcur~=RRnxt)  then
```

```
D-PAC;  return; %double
 end if
if (PATTERN(N,V,N,V,N,V,...))  then
B-PAC;  return; %Bigeminy
 end if
else if (ventricular rhythm)  then
if (HR>120bmp)  then
PVC;
 if (continuous PVC>=3)  then
VT;  return;
 end if
if (PATTERN(N,V,N,V,N,V,...) )  then
B-PVC;  return; %Bigeminy
 end if
if (PATTERN(N,N,V,N,N,V,...))  then
T-PVC;  return; %Trigeminy
 end if
end if
else if (no QRS complex)  then
if (EXIST(VT) && HR > 250bmp)  then
VT;
 end if
end if
```

**36.** Method according to claim 32, wherein the rhythm classifier compares the features of the current rhythm to a rhythm template, and the classification equation can be expressed as:

$$F_{estimate} = \sum_{i \in N} \beta * (1 - |\frac{W_i - T_i}{T_i}|)$$

where:

the $F_{estimate}$ is the classification factor which is used to classify the heart rhythm; N indicates the number of the features;

$\beta_i$ is the weighting coefficient of the feature i, which satisfies the equation $\sum_{i \in N} \beta_i = 1$ , this coefficient indicating the contributions of the feature i to the rhythm classification;
$W_i$ is the value of the feature i of the current rhythm; and
$T_i$ is the value of the feature i of rhythm template.

37. A wireless sensor network device comprising :

   - a power subsystem;
   - a computation subsystem to communicate and process sensor data;
   - a sensor subsystem including at least one sensor;
   - a wireless communication subsystem,

   wherein the computation subsystem comprises an embedded real-time operating system according to any one of claims 1 to 14.

38. A wireless sensor network device according to claim 37, wherein the sensor subsystem comprises at least one ECG sensor, and preferably four ECG sensors.

39. A wireless sensor network device according to claim 37, wherein the computation subsystem also comprises a dedicated real-time minimal TCP/IP stack.

40. A wireless sensor network device according to claim 37, wherein the wireless communication subsystem communicates with other devices or a network via a Bluetooth or Wifi protocol.

41. A wireless sensor network device according to claim 37, wherein the sensor subsystem also comprises means integrating the method of any one of claims 15 to 36.

42. A wireless sensor network device according to claim 37, wherein the sensor subsystem comprises one or more executable software programs on a data storage media which correspond to the method of any one of claims 15 to 36.

43. A wireless sensor network device according to claim 37, wherein the computation subsystem also comprises means integrating the method of any one of claims 15 to 36.

44. A wireless sensor network device according to claim 37, wherein the computation subsystem comprises one or more executable software programs on a data storage media which correspond to the method of any one of claims 15 to 36.

45. A wireless sensor network device according to claim 37, wherein the device further comprises memory storage means adapted to store captured ECG signals and other relevant data transiently or permanently.

46. A wireless sensor network device according to claim 45, wherein the transient memory storage means is chosen from the group consisting of a smartcard, secure digital (SD) card, a smartmedia card, and a usb memory stick.

47. Method for the diagnosis of cardiac arrhythmia, wherein the method comprises :

   - placing a wireless sensor network device according to any one claims 37 to 47 on a person to be put under cardiac surveillance;
   - placing ECG sensors in appropriate locations on said person;
   - activating the device to capture ECG signals and process them in accordance with any one of claims 15 to 36;
   - upon detection of arrhythmia by the device, issuing warnings to the person under surveillance via the device, a packet switched telephone network, wireless telephone network or via a computer network connection to a nearby computer terminal;
- optionally transmitting the data collected and analysed to a remote clinical facility for diagnosis or confirmation of diagnosis by a trained physician.

**48.** Method according to claim 47, wherein the method further comprises reprogramming or resetting the wireless sensor network device remotely via the network.

**49.** Method according to claim 47, wherein multiple wireless sensor network devices may be placed on multiple persons to be put under surveillance, thereby enabling a population of persons to be studied in real time.

**50.** Method according to claim 47, wherein the trained physician reprograms the operating mode of the device remotely from the patient via the network.

Sensors

14 pin J-Tag connector ⟷ MSP430FI49

RFM

**Fig. 1a**

Screen    Sensors

14 pin J-Tag connector ⟷ MSP430FI49 ⟷ Serial Flash

RFM

**Fig. 1b**

Sensors

Screen    MSP430FI49

14 pin J-Tag connector ⟷ CP3000 ⟷ Serial Flash

Bluetooth/ Wifi    RFM

**Fig. 1c**

Exceptions

RCV()

Task

SND()

Fig. 2a

Exceptions

RCV()

Job

SND()

Fig. 2b

Fig. 3

Fig. 4a      periodic task

Fig. 4b      priority task

Flash

| Constant Codes |
| --- |
| |
| |

SRAM

| Global Variables |
| --- |
| Stacks |
| Tuples |
| |

| System stack |
| --- |
| Priority task 1 stack |
| ... |
| Daemon |

| Tuple Key = 1 |
| --- |
| Tuple Key = 2 |
| ... |
| Tuple Key = 3 |

# Fig. 5

Timer interrrupt (time TICK)
n

Timer interrrupt (time TICK)
n + 1

Case 4    Case 5    Case 1    Case 2    Case 3

Interrupt    Periodic    Periodic    Priority    Priority
Handler      Task 1      Task 2      Task 1      Task 2

Fig. 6

Fig. 7a

Fig. 7b

Fig. 8

Fig. 9

Fig. 10

**Application**

```
┌─────────────────┐        ┌─────────────────┐        ┌─────────────────┐
│ Application read │───────▶│ Socket read from │───────▶│   Application    │
│   from socket    │        │  socket Queue    │        │    get data,     │
│                  │        │                  │        │    continue      │
└─────────────────┘        └─────────────────┘        └─────────────────┘
```

**Transport**

```
                           ┌─────────────────┐        ┌─────────────────┐
                           │ Verify TCP segment│       │    Wait for     │
                           │ or UDP datagram, │        │  socket read    │
                           │ store on socket  │        │                 │
                           │     Queue        │        └─────────────────┘
                           └─────────────────┘
```

**Internet**

```
                           ┌─────────────────┐
                           │ Verify IP packet │
                           └─────────────────┘
```

**Network**

```
                           ┌─────────────────┐
                           │ Verify PPP frame │
                           └─────────────────┘

    ┌──────────────────┐
    │  ▓▓▓▓▓    Data   │   ┌─────────────────┐
    │  ▓▓▓▓▓     IP    │   │ Data arrived on  │
    │  ▓▓▓▓▓ TCP/UDP/ICMP│ │ medium is stored on│
    │  ■         PPP   │   │   input Queue    │
    └──────────────────┘   └─────────────────┘
```

Fig. 11

| flag 0x7e | addr 0xff | control 0x03 | protocol | information | CRC | flag 0x7e |
|:---:|:---:|:---:|:---:|:---:|:---:|:---:|
| 1 | 1 | 1 | 2 | up to MRU (Maximum Receive Unit) | 2 | 1 |

| protocol 0x0021 | IP datagram |
|:---:|:---:|

| protocol 0xc021 | Link control data |
|:---:|:---:|

| protocol 0x8021 | IP network control data |
|:---:|:---:|

# Fig. 12a

| code | identifier | length | data | a |
|:---:|:---:|:---:|:---:|:---:|
| 1 | 1 | 2 | Up to (MRU-4) | |

| type | length | Configuration options | b |
|:---:|:---:|:---:|:---:|
| 1 | 1 | | |

# Fig. 12b

Fig. 13

| | Events | | | Actions | States |
|---|---|---|---|---|---|
| Up | Lower layer is Up | Tlu | This-Layer-Up | | Initial/Closed |
| Down | Lower-layer is down | Tld | This-Layer-Down | | Closing |
| TO+ | Timeout with counter >0 | Irc | Initialize-Restart-Count | | Req-sent |
| TO- | Timeout with counter expired | Zrc | Zero-Restart-Count | | Ack-Rcvd |
| RCR- | Receive-Configure-Request (Good) | Scr | Send-Configure-Request | | Ack-Sent |
| RCR+ | Receive-Configure-Request (Bad) | | | | Opened |
| RCA | Receive-Configure-Ack | Sca | Send-Configure-Ack | | |
| RCN | Receive-Configure-Nak/Rej | Scn | Send-Configure-Nak/Rej | | |
| RTR | Receive-Terminate-Request | Str | Send-Terminate-Request | | |
| RTA | Receive-Terminate-Ack | Sta | Send-Terminate-Ack | | |
| RUC | Receive-Unknown-Code | Scj | Send-Code-Reject | | |

Fig. 14

| State Events | 0 Initial(Closed) | 1 Closing | 2 Req-Sent | 3 Ack-Rcvd | 4 Ack-Sent | 5 Opened |
|---|---|---|---|---|---|---|
| Up | tlu,irc,scr/2 | - | - | - | - | - |
| Down | - | 0 | 0 | 0 | 0 | tld,irc,str/1 |
| TO+ | - | str/1 | scr/2 | scr/2 | scr/4 | - |
| TO- | - | tld/0 | tld/0 | tld/0 | tld/0 | - |
| RCR+ | irc,scr,sca/4 | sta/1 | Sca/4 | sca,tlu/5 | sca/4 | tld,scr,sca/4 |
| RCR- | irc,scr,scn/2 | sta/1 | Scn/2 | scn/3 | scn/2 | tld,scr,scn/2 |
| RCA | - | sta/1 | irc/3 | scr/2 | irc,tlu/5 | tld,scr/2 |
| RCN | - | sta/1 | irc,scr/2 | scr/2 | Irc,scr/4 | tld,scr/2 |
| RTR | - | sta/1 | sta/2 | sta/2 | sta/2 | tld,zrc,sta/1 |
| RTA | - | tld/0 | 2 | 2 | 4 | tld,scr/2 |
| RUX | - | scj/1 | scj/2 | scj/3 | scj/4 | scj/5 |

## Fig. 15

STAR        PPP server

| Event | Phase | LCP stat | IPCP stat | Actions |
|---|---|---|---|---|
|  | Dead | Initial | Initial |  |
| UP | Establish | Req_sent | Initial | tlu, irc,scr |
| RCR+ | Establish | Ack-sent | Initial | sca |
| RCA | Network | Opened | Initial | tlu,irc |
| UP | Network | Opened | Req_sent | tlu,irc, scr |
| RCR+ | Network | Opened | Ack-sent | sca |
| RCA | Network | Opened | Opened | tlu,irc |
| DOWN | Terminate | Closing | Closed | tld,irc,str |
| RTA | Dead | Closed | Closed | tld |

LCP: Conf_Req
LCP: Conf_Req
LCP: Conf_Ack
LCP: Conf_Ack
IPCP: Conf_Req
IPCP: Conf_Req
IPCP: Conf_Ack
IPCP: Conf_Ack
IP datagram
IP datagram
IP datagram
LCP: Term_Req
LCP: Term_Ack

## Fig. 16

| 1 | 1 | 2 |
|---|---|---|
| type (0or8) | code (0) | checksum |
| identifier | | sequence number |
| Optional data | | |

Fig. 17

| 0 | 15 16 | 31 |
|---|---|---|

| Source port number | Destination port number |
|---|---|
| Sequence number | |
| Acknowledge number | |
| Header Length | Reserved (0) | URG ACK PSH RST SYN FIN | Window size |
| TCP checksum | | Urgent pointer |
| TCP Options (if any) | | |
| Data (if any) | | |

20 bytes

Fig. 18

| Flag bit | Meaning if Bit is set to 1 |
|----------|----------------------------|
| URG | The Urgent pointer field is valid |
| ACK | The Acknowledgement field is valid |
| PSH | This segment requests a push, the data should be sent to the application as soon as possible |
| RST | Reset the connection |
| SYN | Synchronize sequence numbers to initiate a connection |
| FIN | The sender is finished sending data |

## Fig. 19

## Fig. 20

Fig. 21

| MIB | Name | Data type | R/W | Description |
|---|---|---|---|---|
| System | SysDesr(1) | BYTE | R | Textual description of entity. |
| (1.3.6.1.2.1.1) | SysObjectID(2) | OID | R | Vendor's ID within the subtree 1.3.6.1.4.1 |
| | SysUpTime(3) | TIMETICKS | R | Time in hundredths of a second since network management |
| | SysContact(4) | BYTE | R & W | Name of contact person and how to contact them |
| | SysName(5) | BYTE | R & W | Node's fully qualified domain name |
| | SysLocation(6) | BYTE | R & W | Physical location of node. |
| STAR | PriTskNum(1) | INT | R | Number of priority processes |
| (1.3.6.1.4.1.?.1) | PrdTskNum(2) | INT | R | Number of period processes |
| | ECGONOFF(3) | INT | R & W | One-off of ECG telemedicine process |
| | TimePeriod(4) | INT | R | Switch time(ms) of STAR system scheduler. |
| | MTU(5) | INT | R | Maximum Transmit unit of network packets |
| | SrvAddress(6) | IPADDR | R | IP address of SNMP agent |

Fig. 22

Fig. 23

102

| IP header (20 bytes) | UDP header | Version | Community | PDU type(0~3) | Request ID | Error Status(0~5) | Error index | Name | value | Name | value | ... |
|---|---|---|---|---|---|---|---|---|---|---|---|---|

20 bytes    8 bytes

| PDU type(4) | Enterprise | Agent address | Trap type(0~6) | Specific code | Time stamp | Name | value | ... |
|---|---|---|---|---|---|---|---|---|

## Fig. 24

## Fig. 25

Fig. 26

Fig. 27

| | Statistical | Structural |
|---|---|---|
| Foundation | Statistical decision theory | Human perception and cognition |
| Description | Quantitative features | Morphological primitives |
| | Fixed number of features | Variable number of primitives |
| | Ignores feature relationships | Captures primitive relationships |
| | Semantics from feature position | Semantics from primitive encoding |
| Classification | Statistical classifiers | Parsing with syntactic grammars |

Fig. 28

Fig. 29a

Fig. 29b

| Filter | | Differentiation | | Smooth | | Reconstruction |
|---|---|---|---|---|---|---|

R(t) → **Filter** [1. Notch / 2. Low-Pass / 3. High-Pass / or / 1. Adaptive Filter] → Rf(t) → **Differentiation** [$D(t) = Rf(t) - Rf(t-1)$] → D(t) → **Smooth** [1. Amplifier / 2. Smoother] → Df(t) → **Reconstruction** [$RC(t) = RC(t-1) + Df(t)$] → RC(t)

## Fig. 30

**Data source: MIT-BIH 203**

## Fig. 31

Fig. 32

Data source: MIT-BIH 104

Fig. 33

**Data source: MIT-BIH 101**

Fig. 34

Fig. 35

Fig. 36

| Label | Rhythm | Criteria/ definition | Alarm Level |
|---|---|---|---|
| VA | Ventricular Asystole | • Cardiac arrest, no QRS complexes for 5 s | Red |
| VF | Ventricular Fibrillation | • Absence of QRS complexes<br>• Consecutive irregular waveforms with high enough amplitude and rate | Red |
| VT | Ventricular Tachycardia | • HR(cur) > 120bmp<br>• >= 3 ESVs | Red |
| PVCs/ ESV | Premature Ventricular Contraction | • rate > 120bmp<br>• LQRS >120ms (Wide QRS Complexes)<br>• Ventricular rhythm | Yellow |
| PACs | Premature atrial Contraction | • Regular waveforms (Narrow QRS complexes)<br>• Irregular sinus rhythm | Yellow |
| SB | Sinus Bradycardia | • Consecutive HR(cur)<50bpm<br>• Sinus rhythm | Yellow |
| ST | Sinus Tachycardia | • Consecutive HR>120bpm && HR>1.5*HR(avg)<br>• Sinus rhythm | White |
| SVT | Supraventricular Tachycardia | • A sequence of Continuous HR(cur) >150bmp && HR(cur) <250bmp<br>• Sinus rhythm | Yellow |
| DB | Dropped beats | • HR(cur) = N* HR(avg), N=2,3,...<br>• Sinus rhythm | White |
| SP | Sinus pause | • RR > 1.7s && RR < 3s<br>• Sinus rhythm | Yellow |
| SA | Sinus arrest | • RR >3s<br>• Sinus rhythm | Yellow |

Fig. 37

Fig. 38

| Data base | File | Beats | QRS detection | | | | Level-I correction | | | | Level-II correction | | | |
|---|---|---|---|---|---|---|---|---|---|---|---|---|---|---|
| | | | TP | FN | FP | SH | TP | FN | FP | SH | TP | FN | FP | SH |
| MIT-BIH | 100 | 2273 | 2273 | 0 | 0 | 0 | 2273 | 0 | 0 | 0 | 2273 | 0 | 0 | 0 |
| | 101 | 1865 | 1864 | 1 | 4 | 0 | 1864 | 1 | 1 | 0 | 1862 | 3 | 2 | 0 |
| | 102 | 2187 | 2187 | 0 | 0 | 0 | 2187 | 0 | 0 | 0 | 2187 | 0 | 0 | 0 |
| | 103 | 2084 | 2083 | 1 | 0 | 0 | 2083 | 1 | 0 | 0 | 2083 | 1 | 0 | 0 |
| | 104 | 2229 | 2225 | 3 | 30 | 1 | 2223 | 5 | 5 | 0 | 2221 | 8 | 8 | 0 |
| | 105 | 2572 | 2509 | 63 | 153 | 10 | 2525 | 47 | 78 | 15 | 2512 | 60 | 56 | 13 |
| | 106 | 2027 | 2014 | 13 | 42 | 1 | 2009 | 18 | 3 | 2 | 1991 | 36 | 2 | 1 |
| | 107 | 2137 | 2125 | 11 | 6 | 2 | 2124 | 12 | 4 | 2 | 2125 | 12 | 5 | 2 |
| | 118 | 2278 | 2274 | 4 | 19 | 0 | 2274 | 4 | 2 | 0 | 2261 | 17 | 3 | 1 |
| | 119 | 1987 | 1987 | 0 | 193 | 0 | 1987 | 0 | 1 | 0 | 1955 | 32 | 37 | 31 |
| | 200 | 2601 | 2600 | 1 | 31 | 0 | 2594 | 7 | 2 | 0 | 2592 | 9 | 4 | 0 |
| | 201 | 1963 | 1952 | 11 | 116 | 9 | 1945 | 18 | 11 | 6 | 1946 | 17 | 19 | 3 |
| | 202 | 2136 | 2130 | 6 | 6 | 3 | 2131 | 5 | 3 | 2 | 2070 | 66 | 1 | 1 |
| | 203 | 2980 | 2917 | 63 | 97 | 21 | 2866 | 114 | 23 | 7 | 2872 | 108 | 14 | 6 |
| | 205 | 2656 | 2648 | 8 | 11 | 0 | 2648 | 8 | 7 | 1 | 2647 | 9 | 2 | 1 |
| | 208 | 2955 | 2933 | 22 | 19 | 5 | 2933 | 22 | 7 | 3 | 2921 | 34 | 6 | 2 |
| | 209 | 3005 | 3000 | 5 | 7 | 1 | 2998 | 7 | 4 | 1 | 2999 | 6 | 4 | 1 |
| | 210 | 2650 | 2638 | 12 | 8 | 1 | 2635 | 15 | 3 | 0 | 2636 | 14 | 3 | 0 |
| | 212 | 2748 | 2739 | 9 | 26 | 9 | 2745 | 3 | 5 | 3 | 2726 | 22 | 4 | 4 |
| | 213 | 3251 | 3249 | 1 | 0 | 0 | 3249 | 1 | 0 | 0 | 3250 | 1 | 0 | 0 |
| | 214 | 2262 | 2255 | 7 | 12 | 1 | 2254 | 8 | 5 | 2 | 2254 | 8 | 4 | 1 |
| | 215 | 3363 | 3347 | 16 | 3 | 0 | 3346 | 17 | 0 | 0 | 3362 | 1 | 1 | 0 |
| | 217 | 2208 | 2199 | 9 | 70 | 3 | 2199 | 9 | 20 | 2 | 2198 | 10 | 4 | 2 |
| | 219 | 2154 | 2152 | 2 | 2 | 0 | 2151 | 3 | 1 | 0 | 2148 | 6 | 1 | 0 |
| | Total | 58571 | 58300 | 268 | 855 | 67 | 58243 | 325 | 185 | 46 | 58091 | 480 | 180 | 69 |
| | Result | | Se=0.994287 Sp=0.985546 | | | | Se=0.993670 Sp=0.996834 | | | | Se=0.990638 Sp=0.996911 | | | |
| CSD | P1 | 541 | 541 | 0 | 2 | 0 | 539 | 2 | 0 | 0 | 541 | 0 | 0 | 0 |
| | P2 | 1470 | 1440 | 30 | 29 | 2 | 1438 | 32 | 22 | 2 | 1465 | 5 | 4 | 0 |
| | P3 | 1127 | 1120 | 7 | 20 | 6 | 1120 | 7 | 10 | 6 | 1124 | 3 | 5 | 2 |
| | P4 | 820 | 812 | 2 | 5 | 0 | 811 | 3 | 1 | 0 | 817 | 2 | 0 | 0 |
| | P5 | 975 | 969 | 0 | 5 | 0 | 969 | 0 | 1 | 0 | 972 | 0 | 1 | 0 |
| | P6 | 994 | 972 | 15 | 21 | 4 | 972 | 15 | 11 | 5 | 985 | 4 | 7 | 0 |
| | P7 | 1295 | 1284 | 4 | 23 | 0 | 1283 | 5 | 10 | 2 | 1289 | 5 | 3 | 2 |
| | P8 | 1279 | 1272 | 1 | 15 | 0 | 1272 | 1 | 5 | 0 | 1275 | 4 | 0 | 0 |
| | P9 | 1050 | 1044 | 1 | 35 | 1 | 1032 | 12 | 33 | 12 | 1047 | 2 | 5 | 0 |
| | P10 | 1062 | 1053 | 4 | 66 | 2 | 1040 | 17 | 42 | 15 | 1057 | 5 | 3 | 1 |
| | Total | 10613 | 10507 | 64 | 221 | 15 | 10476 | 94 | 135 | 42 | 10572 | 30 | 28 | 5 |
| | Result | | Se=0.992537 Sp=0.979400 | | | | Se=0.987184 Sp=0.987277 | | | | Se=0.996700 Sp=0.997358 | | | |

Fig. 39

Platform dedicated to real time remote continuous arrhythmia detection and monitoring.

Fig. 40

1. Real-time ECG acquisition module
2. Real-time ECG diagnosis module
3. Real-time communication module
4. Dial-up server module
5. Database module
6. ECG visualisation module
7. Report point module
8. Remote communication module
9. Remote surveillance module

Fig. 41

| | | | | | QRS Position | QRS Length | QRS State | | | | |
|---|---|---|---|---|---|---|---|---|---|---|---|

a | type | Sequence number | Signal number | QRS number | Sampling frequency | QRSResult | ...... | QRSResult | ECG Signals |

b | type | Sequence number | Images |

c | type | Control code | Control information |

Fig. 42

HP Central Display Screen

Our remote surveillance server

Fig. 43

Fig. 44

**European Patent Office**

**EUROPEAN SEARCH REPORT**

Application Number

EP 05 29 1064

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/054238 A (HONEYWELL INTERNATIONAL INC) 11 July 2002 (2002-07-11)<br><br>* abstract *<br>* page 8, line 9 - line 26 *<br>* page 10, line 11 - page 11, line 4 *<br>----- | 1-5, 11-14, 37-46 | G06F9/46 |
| X | RIPOLL I ET AL: "AN OPTIMAL ALGORITHM FOR SCHEDULING SOFT APERIODIC TASKS IN DYNAMIC-PRIORITY PREEMPTIVE SYSTEMS" IEEE TRANSACTIONS ON SOFTWARE ENGINEERING, IEEE SERVICE CENTER, LOS ALAMITOS, CA, US, vol. 23, no. 6, June 1997 (1997-06), pages 388-399, XP000727454 ISSN: 0098-5589 | 1-3,13, 14,37-46 | |
| A | <br>* abstract *<br>* page 388, left-hand column, line 1 - line 9 *<br>* page 388, right-hand column, line 11 - line 16 *<br>* page 389, left-hand column, line 14 - line 32 *<br>* page 393, right-hand column, line 13 - line 18 *<br>* page 395, right-hand column, line 5 - last line *<br>-----<br>-/-- | 4,5,11, 12 | |

TECHNICAL FIELDS SEARCHED (IPC)

G06F
A61B
A61N

The present search report has been drawn up for all claims

| Place of search | Date of completion of the search | Examiner |
|---|---|---|
| The Hague | 8 February 2006 | Archontopoulos, E |

EPO FORM 1503 03.82 (P04C01)

**EP 1 724 684 A1**

European Patent
Office

# EUROPEAN SEARCH REPORT

Application Number

EP 05 29 1064

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KANEKO H ET AL: "INTEGRATED SCHEDULING OF MULTIMEDIA AND HARD REAL-TIME TASKS" 17TH IEEE REAL-TIME SYSTEMS SYMPOSIUM. WASHINGTON,DC, DEC. 4 - 6, 1996, PROCEEDINGS OF THE IEEE REAL-TIME SYSTEMS SYMPOSIUM, NEW YORK, IEEE, US, vol. SYMP. 17, 4 December 1996 (1996-12-04), pages 206-217, XP000659642 ISBN: 0-7803-3801-4 * abstract * | 1-5, 11-14, 37-46 | |
| A | STANKOVIC J A ET AL: "THE SPRING KERNEL: A NEW PARADIGM FOR REAL-TIME OPERATING SYSTEMS*" OPERATING SYSTEMS REVIEW, ACM, NEW YORK, NY, US, vol. 23, no. 3, 1 July 1989 (1989-07-01), pages 54-71, XP000140317 ISSN: 0163-5980 * abstract * | 1-5, 11-14, 37-46 | TECHNICAL FIELDS SEARCHED (IPC) |
| A | STEVE VESPAL: "MetaH Support for Real-Time Multi-Processor Avionics" WORKSHOP ON OBJECT-ORIENTED REAL-TIME DEPENDABLE SYSTEMS, 1 April 1997 (1997-04-01), pages 11-21, XP002153417 * abstract * * page 14, left-hand column, line 27 - line 34 * | 1-5, 11-14, 37-46 | |

-/--

The present search report has been drawn up for all claims

| Place of search | Date of completion of the search | Examiner |
|---|---|---|
| The Hague | 8 February 2006 | Archontopoulos, E |

EPO FORM 1503 03.82 (P04C01)

117

**EP 1 724 684 A1**

**European Patent Office**

**EUROPEAN SEARCH REPORT**

Application Number

EP 05 29 1064

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIA YU ET AL: "A Novel Architecture for Realizing Grid Workflow using Tuple Spaces" GRID COMPUTING, 2004. PROCEEDINGS. FIFTH IEEE/ACM INTERNATIONAL WORKSHOP ON PITTSBURGH, PA, USA 08-08 NOV. 2004, PISCATAWAY, NJ, USA,IEEE, 8 November 2004 (2004-11-08), pages 119-128, XP010769490 ISBN: 0-7695-2256-4 * abstract * * paragraph [6.2.2] * ----- | 6-8 | |
| A | US 6 502 134 B1 (MAKARIOS SELENE K ET AL) 31 December 2002 (2002-12-31) * abstract * ----- | 6-8 | |
| X | DAMM A ET AL: "THE REAL-TIME OPERATING SYSTEM OF MARS" OPERATING SYSTEMS REVIEW, ACM, NEW YORK, NY, US, vol. 23, no. 3, 1 July 1989 (1989-07-01), pages 141-157, XP000140320 ISSN: 0163-5980 * abstract * * page 143, line 23 - line 29 * * page 146, line 28 - page 147, line 14 * ----- | 9,10 | TECHNICAL FIELDS SEARCHED (IPC) |
| X | US 5 742 825 A (MATHUR ET AL) 21 April 1998 (1998-04-21) * abstract * ----- -/-- | 9,10 | |

The present search report has been drawn up for all claims

| Place of search | Date of completion of the search | Examiner |
|---|---|---|
| The Hague | 8 February 2006 | Archontopoulos, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

118

**European Patent Office**

**EUROPEAN SEARCH REPORT**

Application Number

EP 05 29 1064

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TOMBROFF M ET AL: "OFFEN, VERTEILT UND VERNTZT. ECHTZEITANWENDUNGEN IN UNIX-NETZWERKEN" ELEKTRONIK, WEKA FACHZEITSCHRIFTENVERLAG, POING, DE, vol. 43, no. 21, 18 October 1994 (1994-10-18), pages 110-119, XP000469307 ISSN: 0013-5658 * page 111, left-hand column, line 9 - line 27 * * page 113, left-hand column, line 14 - line 25 * ----- | 9,10 | |
| X | US 2005/004481 A1 (XUE JOEL Q ET AL) 6 January 2005 (2005-01-06) * abstract * ----- | 15-36 | |
| X | US 5 951 483 A (JOO ET AL) 14 September 1999 (1999-09-14) * abstract; figures 2A,2B * ----- | 15-36 | |
| X | WO 2004/008960 A (NEW YORK UNIVERSITY; AXEL, LEON) 29 January 2004 (2004-01-29) * abstract * ----- | 15-36 | TECHNICAL FIELDS SEARCHED (IPC) |
| X | US 3 742 938 A (STERN T,US) 3 July 1973 (1973-07-03) * abstract * ----- | 47-50 | |

The present search report has been drawn up for all claims

| Place of search | Date of completion of the search | Examiner |
|---|---|---|
| The Hague | 8 February 2006 | Archontopoulos, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent

Office

**Application Number**

EP 05 29 1064

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

**European Patent Office**

**LACK OF UNITY OF INVENTION SHEET B**

Application Number

EP 05 29 1064

The Search Division considers that the present European patent application does not comply with the requirements of unity of invention and relates to several inventions or groups of inventions, namely:

```
1. claims: 1-5,11-14,37-46

              Scheduling of two classes of tasks.
              ---

2. claims: 6-8

              Tuple based messaging system.
              ---

3. claims: 9,10

              Primitives defined by a meta language.
              ---

4. claims: 15-36

              Method of analysis of a signal.
              ---

5. claims: 47-50

       Method for the diagnosis of cardiac arrhythmia.
              ---
```

**EP 1 724 684 A1**

## ANNEX TO THE EUROPEAN SEARCH REPORT
## ON EUROPEAN PATENT APPLICATION NO.

EP 05 29 1064

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2006

| Patent document cited in search report | | Publication date | Patent family member(s) | | Publication date |
|---|---|---|---|---|---|
| WO 02054238 | A | 11-07-2002 | NONE | | |
| US 6502134 | B1 | 31-12-2002 | AU | 4986100 A | 17-11-2000 |
| | | | EP | 1093618 A1 | 25-04-2001 |
| | | | WO | 0067142 A1 | 09-11-2000 |
| | | | US | 6553402 B1 | 22-04-2003 |
| US 5742825 | A | 21-04-1998 | JP | 8212086 A | 20-08-1996 |
| US 2005004481 | A1 | 06-01-2005 | NONE | | |
| US 5951483 | A | 14-09-1999 | NONE | | |
| WO 2004008960 | A | 29-01-2004 | AU | 2003261272 A1 | 09-02-2004 |
| US 3742938 | A | 03-07-1973 | NONE | | |

For more details about this annex : see Official Journal of the European Patent Office, No. 12/82